# EUROPEAN PATENT SPECIFICATION

(11) **EP 3 209 684 B1**
(45) Date of publication and mention of the grant of the patent: **23.12.2020**
(21) Application number: 15784344.2
(22) Date of filing: 21.10.2015
(51) Int. Cl.: C07K 16/00

(54) **VH-VL-INTERDOMAIN ANGLE BASED ANTIBODY HUMANIZATION**
ANTIKÖRPERHUMANISIERUNG AUF BASIS VON VH-VL-INTERDOMÄNENWINKEL
HUMANISATION D'ANTICORPS À BASE D'ANGLE VH-VL-INTERDOMAINE

(30) Priority: 24.10.2014 EP 14190307
(43) Date of publication of application: 30.08.2017
(73) Proprietor: F. Hoffmann-La Roche AG, 4070 Basel (CH)
(72) Inventor: BUJOTZEK, Alexander, 81241 München (DE); GEORGES, Guy, 82392 Habach (DE); LIPSMEIER, Florian, 4058 Basel (CH)
(74) Representative: Burger, Alexander
(86) International application number: PCT/EP2015/074294
(87) International publication number: WO 2016/062734

(56) References cited:
- EP-A1- 1 224 224
- WO-A1-2008/003931
- WO-A1-2011/021009
- J. DUNBAR ET AL: "ABangle: characterising the VH-VL orientation in antibodies", PROTEIN ENGINEERING DESIGN AND SELECTION, vol. 26, no. 10, 1 October 2013 (2013-10-01), pages 611-620, XP055179028, ISSN: 1741-0126, DOI: 10.1093/protein/gzt020
- "Chapter 12: Antibody fragments as therapeutics" In: William R Strohl, Lila M Strohl: "Therapeutic Antibody Engineering: Current and Future Advances Driving the Strongest Growth Area in the Pharmaceutical Industry", 2012, Elsevier, XP009183432, ISBN: 9781908818096 pages 265-297, p.273, paragraph 2
- J. DUNBAR ET AL: "SAbDab: the structural antibody database", NUCLEIC ACIDS RESEARCH, vol. 42, no. D1, 8 November 2013 (2013-11-08), pages D1140-D1146, XP055102605, ISSN: 0305-1048, DOI: 10.1093/nar/gkt1043
- ALEXANDER BUJOTZEK ET AL: "Prediction of VH-VL domain orientation for antibody variable domain modeling", PROTEINS: STRUCTURE, FUNCTION, AND BIOINFORMATICS, vol. 83, no. 4, 13 January 2015 (2015-01-13), pages 681-695, XP055179030, ISSN: 0887-3585, DOI: 10.1002/prot.24756

## Description

The current invention is in the field of antibody humanization. Herein is reported a method for antibody humanization comprising the grafting of donor residues onto an acceptor framework wherein the selection of the acceptor framework is done depending on the VH-VL-interdomain angle of the humanized antibody and the donor antibody.

### BACKGROUND

The antigen binding site of antibodies is formed at the interface of the heavy and light chain variable domains, VH and VL, making the VH-VL domain orientation a factor that affects antibody specificity and affinity. Preserving the VH-VL domain orientation in the process of antibody engineering and humanization would be advantageous in order to retain the donor antibody properties. Predicting the correct VH-VL orientation has been recognized as a factor in antibody homology modeling.

In WO 2011/021009 variant immunoglobulins with improved manufacturability related to the finding that modifying the amino acid sequence of immunoglobulin molecules in certain key positions leads to improvements in manufacturability, and in particular to reductions in aggregation propensity and/or increases in production levels.

In WO 2008/003931 a method for framework selection for humanizing antibodies is reported, whereby the most appropriate variable region framework can be selected by taking into account the homology of a human acceptor framework with the donor sequence, but more importantly, selecting those variable region frameworks in which specific residues, being obligatory donor residues, are taken into account, i.e. given weighting. Thus, the more of these weighted (important) donor residues which are already present in a homologous human framework, the more appropriate the human framework is regardless of whether the overall homology is somewhat less than another framework with fewer weighted residues matching.

In WO 2001/027160 (EP 1 224 224) a method of monoclonal antibody production and specifically to the simultaneous in vitro affinity optimization of multiple distinct domains of a variable region of a monoclonal antibody is reported. The grafting is accomplished by generating a diverse library of CDR grafted variable region fragments and then screening the library for binding activity similar or better than the binding activity of the donor. A diverse library is generated by selecting acceptor framework positions that differ at the corresponding position compared to the donor framework and making a library population containing of all possible amino acid residue changes at each of those positions together with all possible amino acid residue changes at each position within the CDRs of the variable region.

Dunbar, J., et al. (Prot. Eng. Des. Sel. 26 (2013) 611-620) report ABangle as characterizing the VH-VL orientation in antibodies. The prediction of VH-VL domain orientation for antibody variable domain modeling was reported by Bujotzek, A., et al. (Proteins: Structure, Function, and Bioinformatics 83 (2015) 681-695). In Chapter 12 of the book "Therapeutic Antibody Engineering: Current and Future Advances Driving the Strongest Growth Area in the Pharmaceutical Industry" (2012, Elsevier, ISBN:9781908818096) on pages 265-297 antibody fragments as therapeutics have been reviewed. Dunbar, J., et al., reported SAbDab, the structural antibody database (Nucleic. Acids Res. 42 (2013) D1140-D1146).

### SUMMARY OF THE INVENTION

The invention is defined by the claims. Everything falling outside the scope of the claims is presented for reference only.

Herein is reported a fast sequence-based method for humanizing an antibody based on the determination of the heavy and light chain variable domain orientation, VH-VL-interdomain orientation (angle). With the methods as reported herein an improved, i.e. faster, more economic, less resource demanding and more efficient, selection of the best suitable humanized variant of a non-human antibody is provided.

In more detail, the method as reported herein uses a fast sequence-based predictor that predicts VH-VL-interdomain orientation. The VH-VL-orientation is described in terms of the six absolute ABangle parameters to precisely separate the different degrees of freedom of VH-VL-orientation. It has been found that with the method as reported herein an improvement in the selection of humanized antibodies regarding the deviation of VH-VL-orientation of variant (humanized) antibodies with regard to the parent (non-human) antibody can be achieved. This shows an improvement regarding the similarity of the VH-VL-interdomain angle between parent (non-human) and variant (humanized) antibody. The method as reported herein (comprising a grafting procedure) is delivering better binding properties of the variant (humanized) antibodies compared to humanized antibodies obtained with different methods. Other engineering methods such as framework shuffling can be combined with the method as reported herein resulting in improved binding of the variant antibodies obtained when exchanging a human framework by another one in order to change the bio-physical properties of the antibody.

One aspect of the invention is a method for selecting one or more variant antibody Fv fragments derived from a parent antibody Fv fragment comprising the following steps:
- generating a multitude of variant antibody Fv fragments by grafting/transferring the antigen binding specificity determining residues from the non-human parent antibody Fv fragment on an human antibody Fv fragment germline amino acid sequence, whereby each variant antibody Fv fragment of the multitude of variant antibody Fv fragments differs from the other variant antibody Fv fragments by at least one amino acid residue,
- determining the VH-VL-orientation for the non-human Fv fragment and for each of the variant antibody Fv fragments of the multitude of variant antibody Fv fragments based on a set of VH-VL-interface residues comprising residues L44, L46, L87, H45, H62 (numbering according to Chothia index) of the antibody Fv fragment,
- selecting those variant antibody Fv fragments that have the smallest difference in the VH-VL-angle compared to the non-human antibody's VH-VL-angle and thereby selecting one or more variant antibody Fv fragments derived from a non-human antibody Fv fragment,
   whereby the one or more variant antibody Fv fragments bind to the same antigen as the non-human antibody Fv fragment,
   wherein the VH-VL-angle is determined by calculating the six parameters
   - the length of C, dc,
   - the torsion angle, HL, from H1 to L1 measured about C,
   - the bend angle, HC1, between H1 and C,
   - the bend angle, HC2, between H2 and C,
   - the bend angle, LC1 between L1 and C, and
   - the bend angle, LC2, between L2 and C,
wherein reference frame planes are registered by i) aligning the Cα coordinates corresponding to the eight positions H36, H37, H38, H39, H89, H90, H91 and H92 of VH and fitting a plane through them and ii) aligning the Cα coordinates corresponding to the eight positions L35, L36, L37, L38, L85, L86, L87 and L88 of VL and fitting a plane through them, iii) placing a placed on each plane, whereby each structure has equivalent mesh points and equivalent VH-VL mesh point pairs, and iv) measuring the Euclidean distance for each pair of mesh points in each structure, whereby the vector C joins the pair of points with the minimum variance in their separation distance,
wherein H1 is the vector running parallel to the first principal component of the VH plane, H2 is the vector running parallel to the second principal component of the VH plane, L1 is the vector running parallel to the first principal component of the VL plane, L2 is the vector running parallel to the second principal component of the VL plane, the HL angle is the torsion angle between the two domains, the HC1 and LC1 are the bend angles equivalent to tilting-like variations of one domain with respect to the other, and the HC2 and LC2 bend angles are equivalent to the twisting-like variations of one domain to the other.

In one embodiment of the invention the method comprising the following step:
- selecting those variant antibody Fv fragments that have the highest (structural) similarity in the VH-VL-interdomain angle compared to the parent antibody's VH-VL-interdomain angle and thereby selecting one or more variant antibody Fv fragments derived from a parent antibody Fv fragment.

Disclosed herein is a method for humanizing a non-human antibody comprising the following steps:
- providing a non-human antibody specifically binding to an antigen,
- generating a multitude of variant antibodies by grafting/transferring one or more specificity determining residues from the non-human antibody on a human or humanized acceptor antibody or germline antibody sequence, whereby each variant antibody of the multitude of variant antibodies differs from the other variant antibodies by at least one amino acid residue,
- determining the VH-VL-orientation for the non-human antibody Fv fragment and for each of the variant antibody's Fv fragments of the multitude of variant antibodies based on a sequence fingerprint of the antibody Fv fragment,
- selecting those variant antibody Fv fragments that have the smallest difference in the VH-VL-orientation compared to the parent antibody's VH-VL-orientation and thereby selecting one or more humanized antibodies derived from a non-human,
whereby the one or more humanized antibodies bind to the same antigen as the non-human antibody.

Disclosed herein is a method for humanizing a non-human antibody comprising the following steps:
- providing a non-human antibody specifically binding to an antigen,
- generating a multitude of variant antibodies by grafting/transferring one or more specificity determining residues from the non-human antibody on a human or humanized acceptor antibody or germline antibody sequence, whereby each variant antibody of the multitude of variant antibodies differs from the other variant antibodies by at least one amino acid residue,
- determining the VH-VL-orientation for the non-human antibody Fv fragment and for each of the variant antibody's Fv fragments of the multitude of variant antibodies based on a sequence fingerprint of the antibody Fv fragment,
- selecting those variant antibody Fv fragments that have the highest (structural) similarity in the VH-VL-interdomain angle compared to the parent antibody's VH-VL-interdomain angle and thereby selecting one or more humanized antibodies derived from a non-human antibody,
whereby the one or more humanized antibodies bind to the same antigen as the non-human antibody.

In one embodiment of the invention the set of VH-VL-interface residues comprises residues H35, H37, H39, H45, H47, H50, H58, H60, H61, H91, H95, H96, H98, H100x-2, H100x-1, H100x, H101, H102, H103, H105, L32, L34, L36, L38, L43, L44, L46, L49, L50, L55, L87, L89, L91, L95x-1, L95x, L96 (numbering according to Chothia index).

In one embodiment of the invention the set of VH-VL-interface residues comprises residues H33, H35, H43, H44, H46, H50, H55, H56, H58, H61, H62, H89, H99, L34, L36, L38, L41, L42, L43, L44, L45, L46, L49, L50, L53, L55, L56, L85, L87, L89, L91, L93, L94/L95x-1, L95x, L96, L97, L100 (numbering according to Chothia index).

In one preferred embodiment of the invention the set of VH-VL-interface residues comprises residues H33, H35, H37, H39, H43, H44, H45, H46, H47, H50, H55, H56, H58, H60, H61, H62, H89, H91, H95, H96, H98, H99, H100x-2, H100x-1, H100x, H101, H102, H103, H105, L32, L34, L36, L38, L41, L42, L43, L44, L45, L46, L49, L50, L53, L55, L56, L85, L87, L89, L91, L93, L94/L95x-1, L95x, L96, L97, L100 (numbering according to Chothia index).

In one embodiment of the invention the set of VH-VL-interface residues comprises residues H35, H37, H39, H45, H47, H50, H58, H60, H61, H91, H95, H96, H98, H100x-2, H100x-1, H100x, H101, H102, H103, H105, L32, L34, L36, L38, L43, L44, L46, L49, L50, L55, L87, L89, L91, L95x-1, L95x, L96, L98 (numbering according to Chothia index).

In one embodiment of the invention the set of VH-VL-interface residues comprises residues H33, H35, H37, H39, H43, H44, H45, H46, H47, H50, H58, H60, H61, H62, H89, H91, H95, H96, H98, H99, H100x-2, H100x-1, H100x, H101, H102, H103, H105, L32, L34, L36, L38, L41, L42, L43, L44, L45, L46, L49, L50, L53, L55, L56, L85, L87, L89, L91, L93, L94, L95x-1, L95x, L96, L97, L98, L100 (numbering according to Chothia index).

In one embodiment of the invention the set of VH-VL-interface residues comprises residues 210, 296, 610, 612, 733 (numbering according to Wolfguy index).

In one embodiment of the invention the set of VH-VL-interface residues comprises residues 199, 202, 204, 210, 212, 251, 292, 294, 295, 329, 351, 352, 354, 395, 396, 397, 398, 399, 401, 403, 597, 599, 602, 604, 609, 610, 612, 615, 651, 698, 733, 751, 753, 796, 797, 798 (numbering according to Wolfguy index).

In one embodiment of the invention the set of VH-VL-interface residues comprises residues 197, 199, 208, 209, 211, 251, 289, 290, 292, 295, 296, 327, 355, 599, 602, 604, 607, 608, 609, 610, 611, 612, 615, 651, 696, 698, 699, 731, 733, 751, 753, 755, 796, 797, 798, 799, 803 (numbering according to Wolfguy index).

In one embodiment of the invention the set of VH-VL-interface residues comprises residues 197, 199, 202, 204, 208, 209, 210, 211, 212, 251, 292, 294, 295, 296, 327, 329, 351, 352, 354, 355, 395, 396, 397, 398, 399, 401, 403, 597, 599, 602, 604, 607, 608, 609, 610, 611, 612, 615, 651, 696, 698, 699, 731, 733, 751, 753, 755, 796, 796, 797, 798, 799, 801, 803 (numbering according to Wolfguy index).

In one embodiment of the invention the set of VH-VL-interface residues comprises residues 199, 202, 204, 210, 212, 251, 292, 294, 295, 329, 351, 352, 354, 395, 396, 397, 398, 399, 401, 403, 597, 599, 602, 604, 609, 610, 612, 615, 651, 698, 733, 751, 753, 796, 797, 798, 801 (numbering according to Wolfguy index).

In one embodiment of the invention the set of VH-VL-interface residues comprises residues 197, 199, 202, 204, 208, 209, 210, 211, 212, 251, 292, 294, 295, 296, 327, 329, 351, 352, 354, 355, 395, 396, 397, 398, 399, 401, 403, 597, 599, 602, 604, 607, 608, 609, 610, 611, 612, 615, 651, 696, 698, 699, 731, 733, 751, 753, 755, 796, 797, 798, 799, 801, 803 (numbering according to Wolfguy index).

In one embodiment of the invention the selecting/selection is based on the top 80 % variant antibody Fv fragments regarding VH-VL-orientation.

In one embodiment of the invention the selecting/selection is of the top 20 % variant antibody Fv fragments regarding VH-VL-orientation.

In one embodiment of the invention the selecting is a deselecting of the worst 20 % variant antibody Fv fragments regarding VH-VL-orientation.

In one embodiment of the invention the VH-VL-orientation is determined by calculating the ABangle VH-VL-orientation parameters using a random forest method.

In one embodiment of the invention the VH-VL-orientation is determined by calculating the ABangle VH-VL-orientation parameters using one random forest method for each ABangle.

In one embodiment of the invention the VH-VL-orientation is determined by calculating the habitual torsion angle, the four bend angles (two per variable domain), and the length of the pivot axis of VH and VL (HL, HC1, LC1, HC2, LC2, dc) using a random forest model.

In one embodiment of the invention the random forest model is trained only with complex antibody structure data.

In one embodiment of the invention the smallest difference is the smallest difference between real and predicted angle parameter value relating to the highest Q² value.

In one embodiment of the invention the smallest difference is the smallest difference between the parent antibody angle parameter and the humanized variant antibody angle parameter value relating to the highest Q² value.

In one embodiment of the invention the highest structural similarity is the lowest average root-mean-square deviation (RMSD). In one embodiment the RMSD is the RMSD determined for all Calpha atoms (or carbonyl atoms) of the amino acid residues of the non-human or parent antibody to the corresponding Calpha atoms of the variant antibody.

In general, the d̅i̅s̅t̅_{ABangle} was improved with regard to the reference of structures by using the VH-VL predictor. The reduction of d̅i̅s̅t̅_{ABangle} by VH-VL reorientation translated generally into better RMSD values, especially with regard to the framework regions. On average, notable improvements of d̅i̅s̅t̅_{ABangle} and improvements of the carbonyl RMSD for the whole Fv was found.

In one embodiment of the invention a model assembled from template structures aligned on either consensus VH or VL framework, followed by VH-VL reorientation on a consensus Fv framework is used for determining the VH-VL-orientation.

In one embodiment of the invention a model aligned on the β-sheet core of the complete Fv (VH and VL simultaneously) is used for determining the VH-VL-orientation.

In one embodiment of the invention a model in which the antibody Fv fragment is reoriented on a consensus Fv framework is used for determining the VH-VL-orientation.

In one embodiment of the invention a model using template structures aligned onto a common consensus Fv framework and VH-VL orientation not being adjusted in any form is used for determining the VH-VL-orientation.

In one embodiment of the invention a model assembled from template structures aligned on either consensus VH or VL framework, followed by VH-VL reorientation on a VH-VL orientation template structure chosen based on similarity is used to determine the VH-VL-orientation.

Disclosed herein is a method for producing an antibody comprising the following steps:
- selecting one or more antibodies or antibody Fv fragments according to a method as reported herein,
- selecting from the one or more antibodies or antibody Fv fragments a single antibody or antibody Fv fragment based on its binding properties,
- cloning the VH and VL encoding nucleic acids into one or more expression vectors,
- transfecting a cell with the expression vectors obtained in the previous step,
- cultivating the transfected cell and thereby producing the antibody.

Disclosed herein is a method for producing an antibody comprising the following steps:
- selecting one or more antibodies or antibody Fv fragments comprising the following steps:
   - generating a multitude of variant antibodies by grafting/transferring one or more specificity determining residues from a non-human antibody on a human or humanized acceptor antibody or germline antibody sequence, whereby each variant antibody of the multitude of variant antibodies differs from the other variant antibodies by at least one amino acid residue,
   - determining the VH-VL-orientation for the non-human antibody Fv fragment and for each of the variant antibody's Fv fragments of the multitude of variant antibodies by calculating the habitual torsion angle, the four bend angles (two per variable domain), and the length of the pivot axis of VH and VL (HL, HC1, LC1, HC2, LC2, dc) using a random forest model based on a set of VH-VL-interface residues consisting of residues H33, H35, H37, H39, H43, H44, H45, H46, H47, H50, H55, H56, H58, H60, H61, H62, H89, H91, H95, H96, H98, H99, H100x-2, H100x-1, H100x, H101, H102, H103, H105, L32, L34, L36, L38, L41, L42, L43, L44, L45, L46, L49, L50, L53, L55, L56, L85, L87, L89, L91, L93, L94/L95x-1, L95x, L96, L97, L100 (numbering according to Chothia index) of the antibody Fv fragment,

   - selecting those variant antibody Fv fragments that have the smallest average root-mean-square deviation (RMSD) determined for all pairs of corresponding Calpha atoms of the non-human antibody Fv fragment and variant antibody Fv fragment,
- selecting from the one or more antibodies a single antibody based on its binding properties,
- cloning the VH and VL encoding nucleic acids into one or more expression vectors,
- transfecting a cell with the expression vectors obtained in the previous step,
- cultivating the transfected cell and thereby producing the antibody.

Disclosed herein is a humanized antibody that comprises amino acid residues from a donor non-human antibody at amino acid positions L26-L32, L44, L46, L50-L52, L87, L91-L96, H26-H32, H45, H53-H55, H62 and H96-H101 (numbering according to Chothia index) and at the remaining positions in the light and heavy chain variable domain residues from an acceptor human or humanized antibody or an acceptor human germline amino acid sequence.

Disclosed herein is a humanized antibody that comprises amino acid residues from a donor non-human antibody at amino acid positions H26-H32, H35, H37, H39, H45, H47, H50, H53-H55, H58, H60, H61, H91, H95, H96-H101, H102, H103, H105, L26-L32, L34, L36, L38, L43, L44, L46, L49, L50-L52, L55, L87, L89, L91-L96 (numbering according to Chothia index) and at the remaining positions in the light and heavy chain variable domain residues from an acceptor human or humanized antibody or an acceptor human germline amino acid sequence.

Disclosed herein is a humanized antibody that comprises amino acid residues from a donor non-human antibody at amino acid positions H26-H32, H33, H35, H43, H44, H46, H50, H53-H55, H56, H58, H61, H62, H89, H96-H101, L26-L32, L34, L36, L38, L41, L42, L43, L44, L45, L46, L49, L50-L52, L53, L55, L56, L85, L87, L89, L91-L96, L97, L100 (numbering according to Chothia index) and at the remaining positions in the light and heavy chain variable domain residues from an acceptor human or humanized antibody or an acceptor human germline amino acid sequence.

Disclosed herein is a humanized antibody that comprises amino acid residues from a donor non-human antibody at amino acid positions H26-H32, H33, H35, H37, H39, H43, H44, H45, H46, H47, H50, H53-H55, H56, H58, H60, H61, H62, H89, H91, H95, H96-H101, H102, H103 H105, L26-L32, L34, L36, L38, L41, L42, L43, L44, L45, L46, L49, L50-L52, L53, L55, L56, L85, L87, L89, L91-L96, L97, L100 (numbering according to Chothia index) and at the remaining positions in the light and heavy chain variable domain residues from an acceptor human or humanized antibody or an acceptor human germline amino acid sequence.

Disclosed herein is a humanized antibody that comprises amino acid residues from a donor non-human antibody at amino acid positions H26-H32, H35, H37, H39, H45, H47, H50, H53-H55, H58, H60, H61, H91, H95, H96-H101, H102, H103, H105, L26-L32, L34, L36, L38, L43, L44, L46, L49, L50-L52, L55, L87, L89, L91-L96, L98 (numbering according to Chothia index) and at the remaining positions in the light and heavy chain variable domain residues from an acceptor human or humanized antibody or an acceptor human germline amino acid sequence.

Disclosed herein is a humanized antibody that comprises amino acid residues from a donor non-human antibody at amino acid positions H26-H32, H33, H35, H37, H39, H43, H44, H45, H46, H47, H50, H53-H55, H58, H60, H61, H62, H89, H91, H95, H96-H101, H102, H103, H105, L26-L32, L34, L36, L38, L41, L42, L43, L44, L45, L46, L49, L50-L52, L53, L55, L56, L85, L87, L89, L91-L96, L97, L98, L100 (numbering according to Chothia index) and at the remaining positions in the light and heavy chain variable domain residues from an acceptor human or humanized antibody or an acceptor human germline amino acid sequence.

### DESCRIPTION OF THE FIGURES

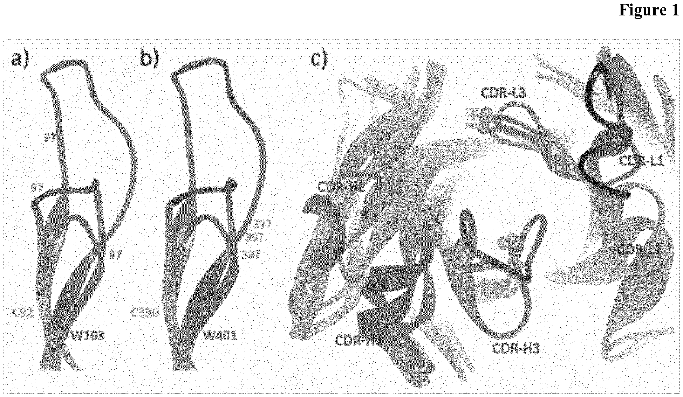

- **Figure 1**: Overlay of three exemplary CDR-H3 loops with 5, 10 and 15 amino acids length, taken from crystal structures with PDB ID 1N7M, 1DLF and 3HZM, respectively: a) Chothia/Kabat numbering shows the wide spatial distribution of residue 97 in the three representative CDR-H3 loops; b) Wolfguy numbering shows a compact spatial localization of residue 97, as it is always the third to last residue before the end of CDR-H3, denominated 397 according to Wolfguy index; c) several amino acids from the CDRs have inter-chain contacts, especially those located at the end of CDR-H3 and CDR-L3 (residue 797 according to Wolfguy index clearly co-localizes and performs contacts with the VH).
- **Figure 2**: Predicted (vertical axis) versus actual ABangle orientation parameters (horizontal axis) for an exemplary run on the complex structures only test dataset (2/3 of the complex structures are used as training set whereas 1/3 is used as the test set). Perfect predictions would lie on the diagonal line.
- **Figure 3**: Top 25 important Fingerprint 3 positions for the six ABangle parameters in terms of Percent Selection Frequency during predictor training. The values are averaged over ten runs with varying, randomly chosen training set (complex structures only). Error bars correspond to one standard deviation. Framework and CDR classification follows Wolfguy nomenclature.
- **Figure 4**: Average change in carbonyl RMSD for framework (FW), CDRs (CDR) and all Fv residues (All) and average change in dist_{ABangle} when using unrestrained instead of restrained minimization (shown for the three variants 1, II, III vs 1, 2, 3).
- **Figure 5**: Average change in carbonyl RMSD for framework (FW), CDRs (CDR) and all Fv residues (All) and average change in dist_{ABangle} per AMAII antibody between original and reoriented models.
- **Figure 6**: Average change in carbonyl RMSD for framework (FW), CDRs (CDR) and all Fv residues (All) and average change in dist_{ABangle} per AMAII participant between original and reoriented models.
- **Figure 7**: The HCs (rows of the matrix, left) and LCs (columns of the matrix, right) are sorted according to their mean angle-distance. These visualizations are used to pick "bad" HCs/LCs.
- **Figure 8**: Matrix with ELISA measurements for the different HC/LC combinations. Antibodies which are deselected by the different methods are shaded; 1: bad HC/LC combinations; 2: whole HCs/LCs rejected; 3: worst 20 %.
- **Figure 9**: Stacked histograms of the ELISA measurements for all three selection methods "bad HC/LC combinations" (left), "whole HCs and LCs" (middle) and "worst 20%" (right). The light-grey regions of the histogram bars indicate the antibodies that are rejected.
- **Figure 10**: The HCs (rows of the matrix, left) and LCs (columns of the matrix, right) are sorted according to their mean angle-distance. These visualizations are used to pick "bad" HCs/LCs.
- **Figure 11**: Matrix with ELISA measurements for the different HC/LC combinations. Antibodies which are deselected by the different methods are shaded; 1: bad HC/LC combinations; 2: whole HCs/LCs rejected; 3: worst 20 %.
- **Figure 12**: Stacked histograms of the ELISA measurements for all three selection methods "bad HC/LC combinations" (left), "whole HCs and LCs" (middle) and "worst 20%" (right). The light-grey regions of the histogram bars indicate the antibodies that are rejected.
- **Figure 13**: The HCs (rows of the matrix, left) and LCs (columns of the matrix, right) are sorted according to their mean angle-distance. These visualizations are used to pick "bad" HCs/LCs.
- **Figure 14**: The three pictures each show the matrix with the BL measurements for the different HC/LC combinations; the antibodies which are deselected by the different methods are shaded; 1: bad HC/LC combinations; 2: whole HCs/LCs rejected; 3: worst 20 %.
- **Figure 15**: Stacked histograms of the ELISA measurements for all three methods "bad HC/LC combinations" (left), "whole HCs and LCs" (middle) and "worst 20%" (right). The light-grey regions of the histogram bars indicate the antibodies that are deselected.
- **Figure 16**: The three pictures each show the matrix with the t1/2 measurements for the different HC/LC combinations; the antibodies which are selected by the different methods are shaded; 1: bad HC/LC combinations; 2: whole HCs/LCs rejected; 3: worst 20 %.
- **Figure 17**: Stacked histograms of the t1/2 measurements for all three methods "bad HC/LC combinations" (left), "whole HCs and LCs" (middle) and "worst 20%" (right). The light-grey regions of the histogram bars indicate the antibodies that are deselected.

### DEFINITIONS

### Wolfguy Numbering Scheme

The Wolfguy numbering defines CDR regions as the set union of the Kabat and Chothia definition. Furthermore, the numbering scheme annotates CDR loop tips based on CDR length (and partly based on sequence) so that the index of a CDR position indicates if a CDR residue is part of the ascending or the descending loop. A comparison with established numbering schemes is shown in Table 1.

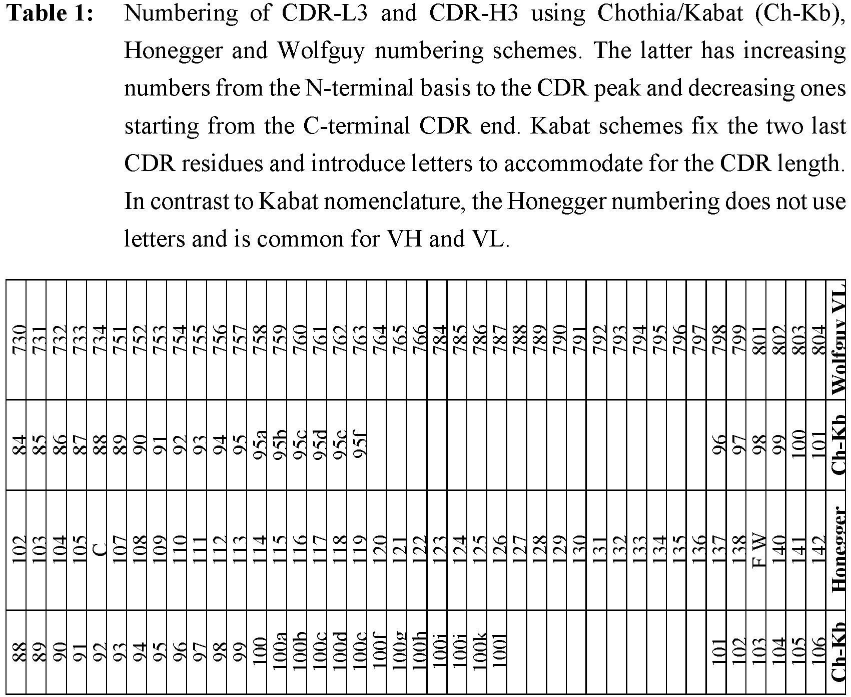

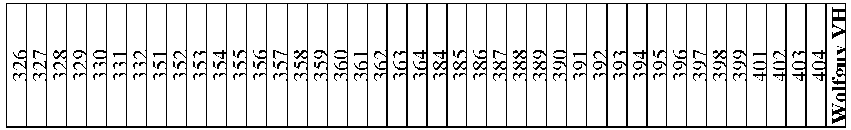

Wolfguy is designed such that structurally equivalent residues (i.e. residues that are very similar in terms of conserved spatial localization in the Fv structure) are numbered with equivalent indices as far as possible. This is illustrated in Figure 1.

An example for a Wolfguy-numbered full-length VH and VL sequence can be found in Table 2.

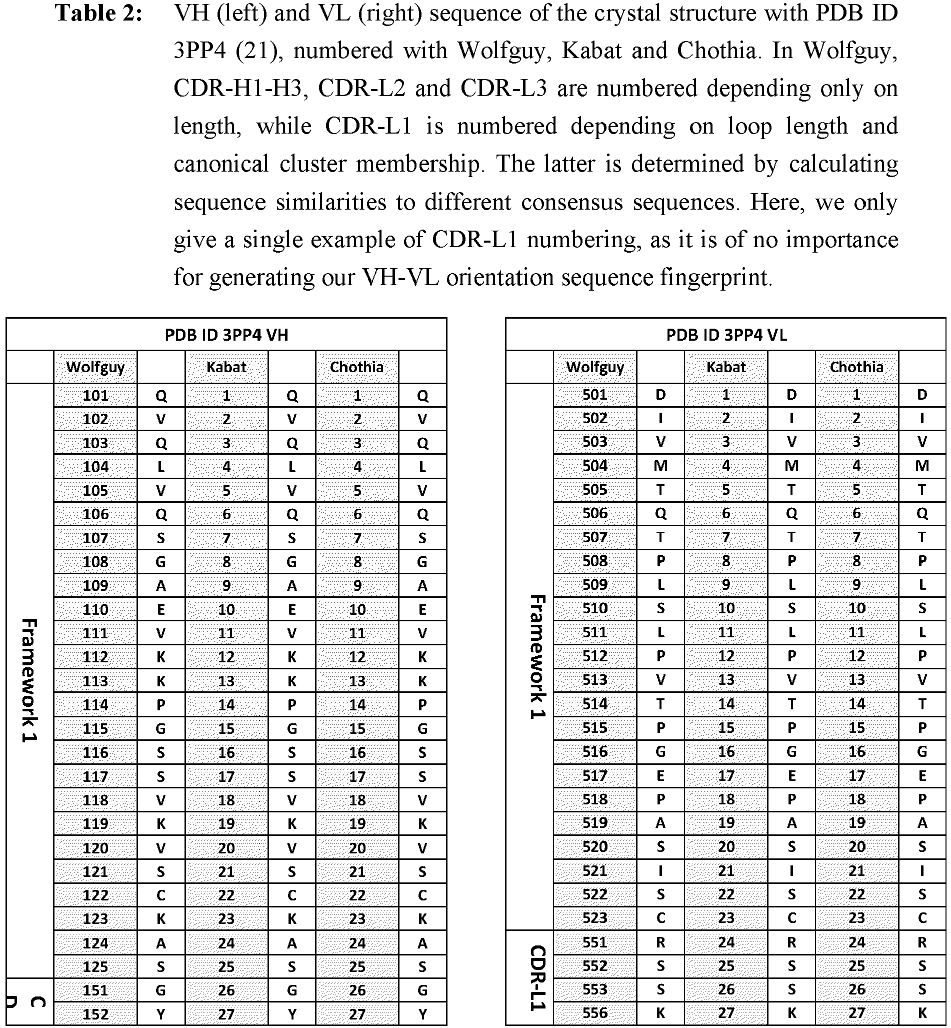

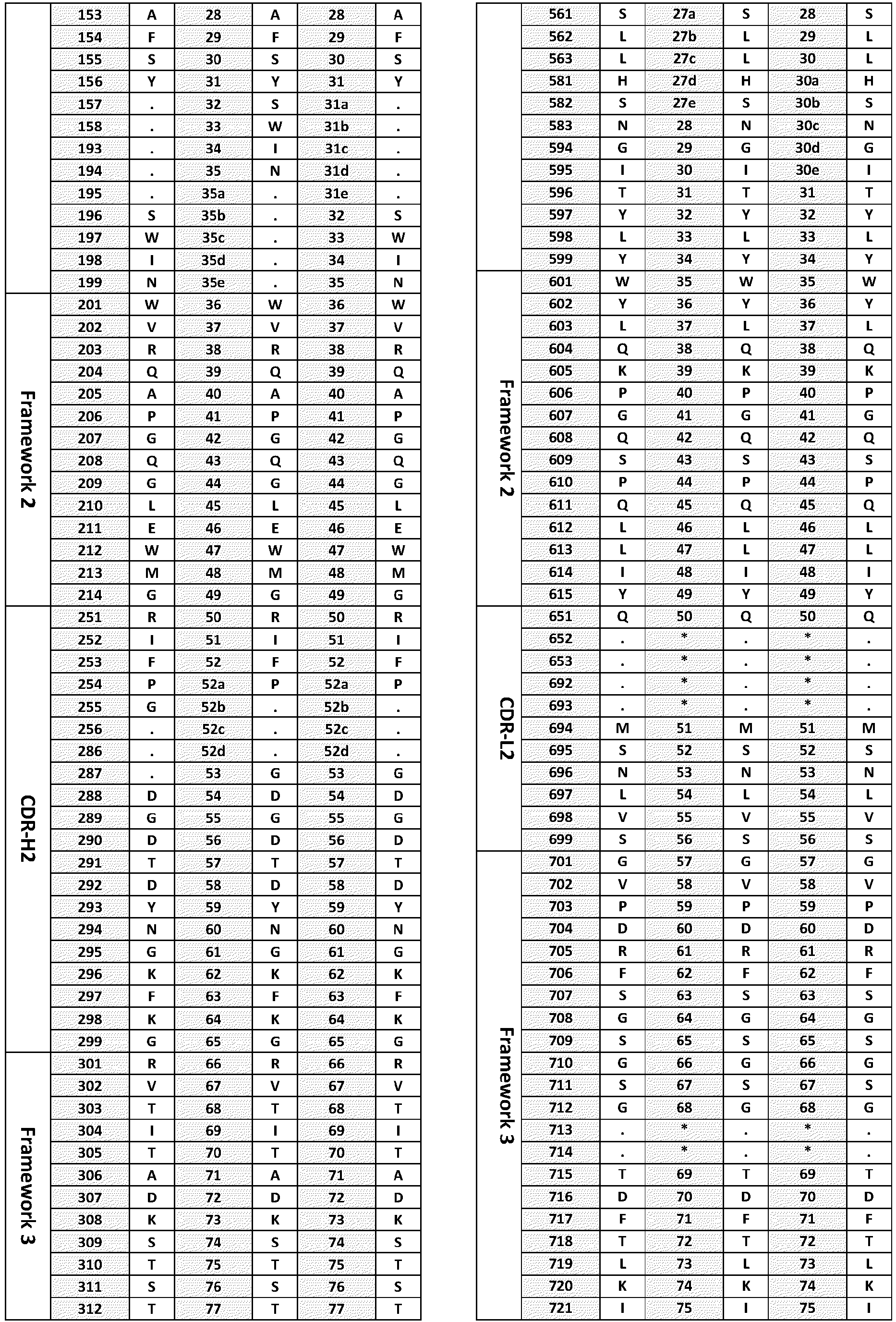

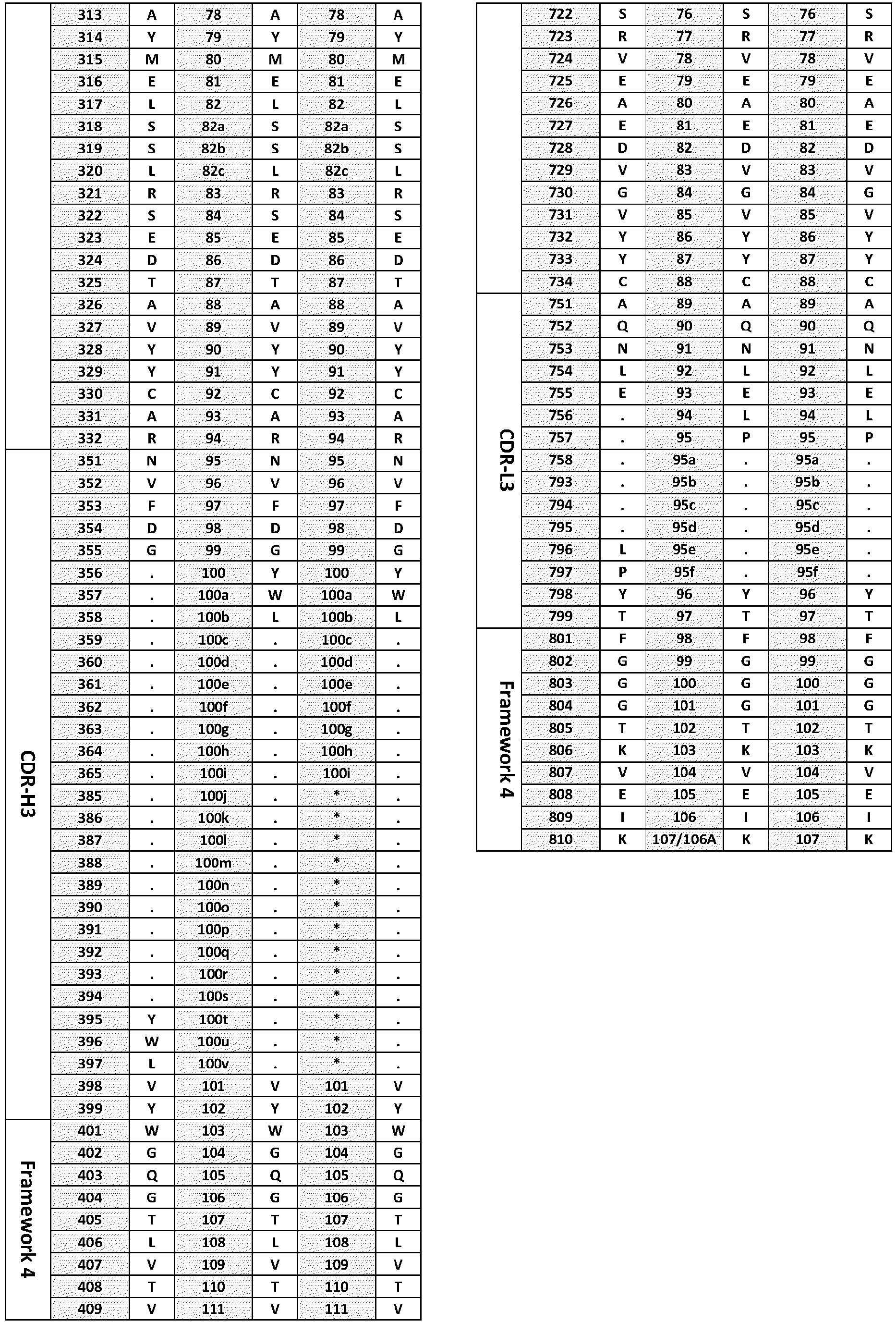

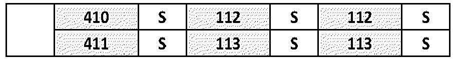

### The ABangle concept (7)

When making a comparison between any two amino acid based structures, generally distance-based metrics such as the root-mean-square deviation (RMSD) of equivalent atoms are used.

To characterize the orientation between any two three-dimensional objects, it is necessary to define:
- a frame of reference on each object.
- axes to measure orientation parameters about.
- terminology to describe and quantify these parameters.

The ABangle concept is a method which fully characterizes VH-VL orientation in a consistent and absolute sense using five angles (HL, HC1, LC1, HC2 and LC2) and a distance (dc). The pair of variable domains of an antibody, VH and VL, is denoted collectively as an antibody Fv fragment.

In a first step antibody structures were extracted from a data bank (e.g. the protein data bank, PDB). Chothia antibody numbering (Chothia and Lesk, 1987) was applied to each of the antibody chains. Chains that were successfully numbered were paired to form Fv regions. This was done by applying the constraint that the H37 position Cα coordinate of the heavy chain (alpha carbon atom of the amino acid residue at heavy chain variable domain position 37) must be within 20 Å of the L87 position Cα coordinate of the light chain. A non-redundant set of antibodies was created using CDHIT (Li, W. and Godzik, A. Bioinformatics, 22 (2006) 1658-1659), applying a sequence identity cut-off over the framework of the Fv region of 99 %.

The most structurally conserved residue positions in the heavy and light domains were used to define domain location. These positions are denoted as the VH and VL coresets. These positions are predominantly located on the β-strands of the framework and form the core of each domain. The coreset positions are given in the following Table 3:

| **light chain** | **heavy chain** |
|---|---|
| L44 | H35 |
| L19 | H12 |
| L69 | H38 |
| L14 | H36 |
| L75 | H83 |
| L82 | H19 |
| L15 | H94 |
| L21 | H37 |
| L47 | H11 |
| L20 | H47 |
| L48 | H39 |
| L49 | H93 |
| L22 | H46 |
| L81 | H45 |
| L79 | H68 |
| L80 | H69 |
| L23 | H71 |
| L36 | H70 |
| L35 | H17 |
| L37 | H72 |
| L74 | H92 |
| L88 | H84 |
| L38 | H91 |
| L18 | H90 |
| L87 | H20 |
| L17 | H21 |
| L86 | H85 |
| L85 | H25 |
| L46 | H24 |
| L70 | H86 |
| L45 | H89 |
| L16 | H88 |
| L71 | H87 |
| L72 | H22 |
| L73 | H23 |

The coreset positions were used to register frames of reference onto the antibody Fv region domains.

The VH domains in the non-redundant dataset were clustered using CDHIT, applying a sequence identity cut-off of 80 % over framework positions in the domain. One structure was randomly chosen from each of the 30 largest clusters. This set of domains was aligned over the VH coreset positions using Mammoth-mult (Lupyan, D., et al., Bioinf. 21 (2005) 3255-3263). From this alignment the Cα coordinates corresponding to the eight structurally conserved positions H36, H37, H38, H39, H89, H90, H91 and H92 in the β-sheet interface were extracted. Through the resulting 240 coordinates a plane was fitted. For the VL domain positions L35, L36, L37, L38, L85, L86, L87 and L88 were used to fit the plane.

The procedure described above allows mapping the two reference frame planes onto any Fv structure. Therefore the measuring of the VH-VL orientation can be made equivalent to measuring the orientation between the two planes. To do this fully and in an absolute sense requires at least six parameters: a distance, a torsion angle and four bend angles. These parameters must be measured about a consistently defined vector that connects the planes. This vector is denoted C in the following. To identify C, the reference frame planes were registered onto each of the structures in the non-redundant set as described above and a mesh placed on each plane. Each structure therefore had equivalent mesh points and thus equivalent VH-VL mesh point pairs. The Euclidean distance was measured for each pair of mesh points in each structure. The pair of points with the minimum variance in their separation distance was identified. The vector which joins these points is defined as C.

The coordinate system is fully defined using vectors, which lie in each plane and are centered on the points corresponding to C. H1 is the vector running parallel to the first principal component of the VH plane, while H2 runs parallel to the second principal component. L1 and L2 are similarly defined on the VL domain. The HL angle is a torsion angle between the two domains. The HC1 and LC1 bend angles are equivalent to tilting-like variations of one domain with respect to the other. The HC2 and LC2 bend angles describe twisting-like variations of one domain to the other.

To describe the VH-VL orientation six measures are used, a distance and five angles. These are defined in the coordinate system as follows:
- the length of C, dc,
- the torsion angle, HL, from H1 to L1 measured about C,
- the bend angle, HC1, between H1 and C,
- the bend angle, HC2, between H2 and C,
- the bend angle, LC1 between L1 and C, and
- the bend angle, LC2, between L2 and C.

The term "VH-VL orientation" is used in accordance with its common meaning in the art as it would be understood by a person skilled in the art (see, e.g., Dunbar et al., Prot. Eng. Des. Sel. 26 (2013) 611-620; and Bujotzek, A., et al., Proteins, Struct. Funct. Bioinf., 83 (2015) 681-695). It denotes how the VH and VL domains orientate with respect to one another.

Thus the VH-VL orientation is defined by
- the length of C, dc,
- the torsion angle, HL, from H1 to L1 measured about C,
- the bend angle, HC1, between H1 and C,
- the bend angle, HC2, between H2 and C,
- the bend angle, LC1 between L1 and C, and
- the bend angle, LC2, between L2 and C,
wherein reference frame planes are registered by i) aligning the Cα coordinates corresponding to the eight positions H36, H37, H38, H39, H89, H90, H91 and H92 of VH and fitting a plane through them and ii) aligning the Cα coordinates corresponding to the eight positions L35, L36, L37, L38, L85, L86, L87 and L88 of VL and fitting a plane through them, iii) placing a placed on each plane, whereby each structure has equivalent mesh points and equivalent VH-VL mesh point pairs, and iv) measuring the Euclidean distance for each pair of mesh points in each structure, whereby the vector C joins the pair of points with the minimum variance in their separation distance,
wherein H1 is the vector running parallel to the first principal component of the VH plane, H2 is the vector running parallel to the second principal component of the VH plane, L1 is the vector running parallel to the first principal component of the VL plane, L2 is the vector running parallel to the second principal component of the VL plane, the HL angle is the torsion angle between the two domains, the HC1 and LC1 are the bend angles equivalent to tilting-like variations of one domain with respect to the other, and the HC2 and LC2 bend angles are equivalent to the twisting-like variations of one domain to the other.

The positions are determined according to the Chothia index.

The vector C was chosen to have the most conserved length over the non-redundant set of structures. The distance, dc, is this length. It has a mean value of 16.2 Å and a standard deviation of only 0.3 Å.

Table 4 lists the top 10 positions and residues identified by the random forest algorithm as being important in determining each of the angular measures of VH-VL orientation.

**Table 4: X represents the variable L36Va/L38Eb/L42Ha/L43La/L44Fa,b/L45T/ L46Gb/L49G/L95H**

| Angle | top 10 important input variables |
|---|---|
| HL | L87Fb L42Ga/L43Ta L44Va,b H61D L89L H43Q H43N/H44K H62Kb/H89V L55H L53R |
| HC1 | Xa,b L56P L41Da,b L89A L97V L94N L34H L34N L96W L100A |
| HC2 | H62Sb H62Kb/H89V H43K H50W H46K/H62Db H35S H61Q |
| | H43Q |
| | H33W H58T |
| LC1 | L91W L89A Xa,b L97V L94N L50G H43Q L56P H62Sb L55A |
| LC2 | L50Y L42Ga/L43Ta L44Va,b L42Qa L55H H99Y L93T L94L |
| | L53R |
| | L85T |
| a: denotes those positions also found to be influential by Chailyan et al. | |
| b: denotes positions also found to be influential by Abhinandan and Martin. | |

(for more detailed information see reference 7 and Bujotzek, A., et al., Prot. Struct. Funct. Bioinf. 83 (2015) 681-695).

### Further definitions:

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of non-covalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat, E.A. et al., Sequences of Proteins of Immunological Interest, 5th ed., Public Health Service, National Institutes of Health, Bethesda, MD (1991), NIH Publication 91-3242.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody", "intact antibody," and "whole antibody" are used herein interchangeably to refer to an antibody having a structure substantially similar to a native antibody structure or having heavy chains that contain an Fc region as defined herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat, E.A. et al., Sequences of Proteins of Immunological Interest, 5th ed., Bethesda MD (1991), NIH Publication 91-3242, Vols. 1-3. In one embodiment, for the VL, the subgroup is subgroup kappa I as in Kabat et al., supra. In one embodiment, for the VH, the subgroup is subgroup III as in Kabat et al., supra.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR", as used herein, refers to each of the regions of an antibody variable domain which are hypervariable in sequence ("complementarity determining regions" or "CDRs") and/or form structurally defined loops ("hypervariable loops"), and/or contain the antigen-contacting residues ("antigen contacts"). Generally, antibodies comprise six HVRs; three in the VH (H1, H2, H3), and three in the VL (L1, L2, L3).

HVRs herein include
(a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);
(b) CDRs occurring at amino acid residues 24-34 (L1), 50-56 (L2), 89-97 (L3), 31-35b (H1), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., supra.

The term "specificity determining residue" is used according to its meaning in the art. It defines the residues of an antibody that are directly involved in the interaction with antigen (see e.g. Padlan, E.A., et al., FASEB J. 9 (1995) 133-139).

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman, S. et al., J. Chromatogr. B 848 (2007) 79-87.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt, T.J. et al. Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano, S. et al., J. Immunol. 150 (1993) 880-887; Clackson, T. et al., Nature 352 (1991) 624-628).

### DETAILED DESCRIPTION OF THE INVENTION

Herein is reported a fast sequence-based predictor that predicts VH-VL-interdomain orientation. Q² values ranging from 0.67 to 0.80 are achieved. The VH-VL-orientation is described in terms of the six absolute ABangle parameters to precisely separate the different degrees of freedom of VH-VL-orientation. The impact of VH-VL-orientation was evaluated on different antibody structures. It has been found that with the method as reported herein an improvement regarding the deviation of VH-VL-orientation of variant (humanized) antibodies with regard to the parent (non-human) antibody can be achieved. This is shown by the average root-mean-square deviation (RMSD) of the carbonyl atoms of the amino acid backbone. This shows an improvement regarding the similarity of the VH-VL-interdomain angle between parent (non-human) and variant (humanized) antibody. The method as reported herein (comprising a grafting procedure) is delivering better binding properties of the variant (humanized) antibodies. Other engineering methods such as framework shuffling can be combined with the method as reported herein resulting in improved binding of the variant antibodies obtained when exchanging a human framework by another one in order to change the bio-physical properties of the antibody. This results in the provision of a method for selecting better humanized antibodies from a multitude of variant antibodies derived from a parent antibody.

The use of antibodies in therapeutics and clinical diagnostics created a demand for precise homology models of antibody structures that enable rational antibody engineering whenever a crystal structure is not available. Therefore a multitude of computational methods for computer-aided antibody design (1), among them a number of homology modeling approaches that are regularly being assessed by blind modeling studies (8,2), has been developed.

Due to the number of experimentally derived antibody structures (the structural antibody database SAbDab3 counts 1841 entries as of May 2014) the quality of antibody homology models is excellent in comparison to homology models of other biomolecules. The six antigen-binding loops of the two antibody variable fragments (Fvs) are hypervariable in sequence (hypervariable regions, HVRs). Five of them are prone to adapt canonical conformations that can be predicted from sequence based on existing template structures. This does not hold for the third loop on the variable region of the heavy chain, HVR-H3. The HVR-H3 is the most variable loop with regard to sequence and length, and typically the main antigen interaction specificity determining site.

The antigen binding site of an antibody forms at the interface of the two Fvs (heavy chain variable domain (VH) and light chain variable domain (VL). Each variable domain comprises three HVRs. The relative orientation of VH and VL domain adds to the topology of the antigen binding site.

In their recent Antibody Modeling Assessment study 2 (AMAII), Teplyakov et al. (2) used a single angular measure to describe VH-VL orientation. The difference in VH-VL tilt angle with respect to a reference structure is calculated as the κ angle in spherical angular system (ω, φ, κ) of the coordinate transformation achieved by sequential superposition of the VL and VH domains using a set of structurally conserved β-sheet core positions. Narayanan et al. (6) used an RMSD (root mean square deviation) based metric to train and evaluate an energy-based predictor of VH-VL orientation. Chailyan and coworkers (5) identified clusters of Fv structures of similar VH-VL orientation and determined influential sequence positions by measuring the Cα superposition RMSD of certain conserved residues. Other studies augment the RMSD values by providing the amount of rotation necessary to reorient one crystal structure's VH or VL onto another (10-12).

Abhinandan and Martin (4) defined the VH-VL packing angle, an absolute metric for comparing VH-VL orientation. The VH-VL packing angle is the torsion angle spanned by a vector fitted through the principal axes of a highly conserved set of Cα positions in each of the two domains. In contrast to relative RMSD values, the VH-VL packing angle allows to describe each individual Fv structure in terms of its VH-VL orientation in structural space. Along with the definition of the VH-VL packing angle, the authors identified a set of influential positions and provided a sequence-based predictor of VH-VL packing learned with a neural network.

Based on the past observations, which are at least in parts inconsistent with regard to Fv sequence positions deemed to have an impact VH-VL orientation (4, 5), Dunbar and coworkers (7) suggested that VH-VL orientation is subject to multiple degrees of freedom, and that each degree of freedom is determined by a different set of influential sequence positions. Consequently, the authors, in addition to the habitual torsion angle, defined four bend angles (two per variable domain), as well as the length of the pivot axis of VH and VL, and, using a random forest model, identified the most influential sequence positions for each of the five angle parameters (ABangle), as well as for the length of the pivot axis between VH and VL

Herein is reported an ABangle-based method for the characterization and exploitation of the VH-VL-orientation during the humanization of an antibody. Herein is reported a sequence-based predictor of VH-VL-orientation for each of the six ABangle measures. Also a method of adjusting VH-VL orientation in actual antibody homology models is reported.

Herein is reported an ABangle-based method for the characterization and exploitation of the VH-VL-orientation during the transfer of binding determining residues from a donor antibody to an acceptor antibody framework.

Herein is reported an ABangle-based method for the characterization and exploitation of the VH-VL-orientation during the exchange of parts or entire framework regions of an antibody (framework shuffling).

### VH-VL Orientation Predictor

**Table 5: Q² and RMSE values for the prediction of the six ABangle parameters averaged over 50 runs. The number of trees per random forest model was tuned manually so as to maximize Q². The values in brackets specify the standard deviation.**

| | | **Apo and complex structures (n = 2249)** | | **Complex structures only (n_complex = 1468)** | |
|---|---|---|---|---|---|
| **Parameter** | **N trees** | **Q² test set** | **RMSE test set** | **Q² test set** | **RMSE test set** |
| HL | 33 | 0.68 (0.02) | 2.28 (0.08) | 0.67 (0.02) | 2.26 (0.10) |
| HC1 | 50 | 0.77 (0.02) | 1.04 (0.05) | 0.80 (0.02) | 0.97 (0.04) |
| LC1 | 50 | 0.73 (0.02) | 1.26 (0.05) | 0.75 (0.02) | 1.25 (0.06) |
| HC2 | 50 | 0.78 (0.01) | 1.48 (0.04) | 0.79 (0.02) | 1.40 (0.07) |
| LC2 | 75 | 0.65 (0.02) | 1.40 (0.07) | 0.69 (0.03) | 1.30 (0.06) |
| dc | 100 | 0.56 (0.08) | 0.21 (0.05) | 0.67 (0.02) | 0.18 (0.01) |

The random forest model was trained once on the complete dataset of apo and complex structures (Table 5, central column) and once on the complex structures only (Table 5, right column). Although the training set was reduced by almost 550 structures, the Q² and RMSE values improved when only complex structures were used. For HL, LC2 and dc, the Q² value is about 0.68, while HC1, LC1 and LC2 have Q² values of 0.75 and above (when considering complex structures).

Alternatively to ensure to include the maximum diversity of different orientation fingerprints in the training set CD-HIT can be used to cluster the orientation fingerprints at 100 % identity, and, for each cluster, at least one representative can be added to the training set, until 2/3 of the available structures are assigned to the training set. The remaining ⅓ can be used for testing. Due to the fact that the test set then would consist of orientation fingerprints that are also included in the training set, the resulting Q² values, e.g. ranging from 0.71 to 0.88 for the current datd set depending on the respective ABangle parameter, would overstate the actual capabilities of the predictor when confronted with an unknown orientation fingerprint. In that case, Q² values to range 0.54 to 0.73, approximately, could be found for the current dataset.

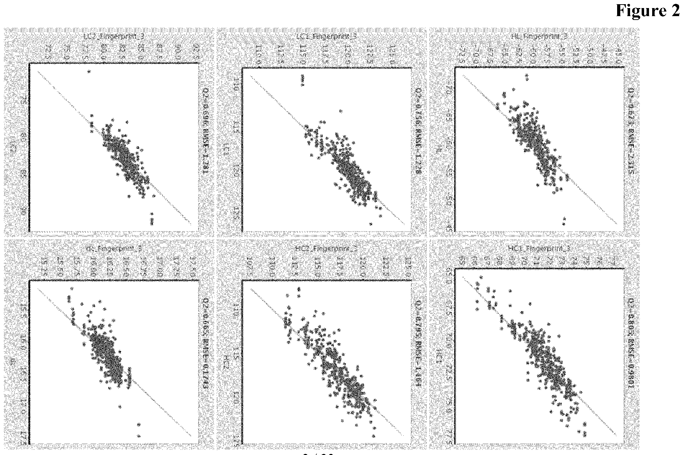

Figure 2 shows exemplary regression plots for predicted versus actual ABangle parameters on the complex structures only dataset.

The correlation is improved compared e.g. to that reported by Abhinandan and Martin (4). Without being bound by this theory the improvement can be attributed to a finer description of the degrees of freedom of VH-VL-orientation in terms of the six ABangle parameters and the use of the Wolfguy numbering scheme reducing or even avoiding ambiguities in HVR residue numbering.

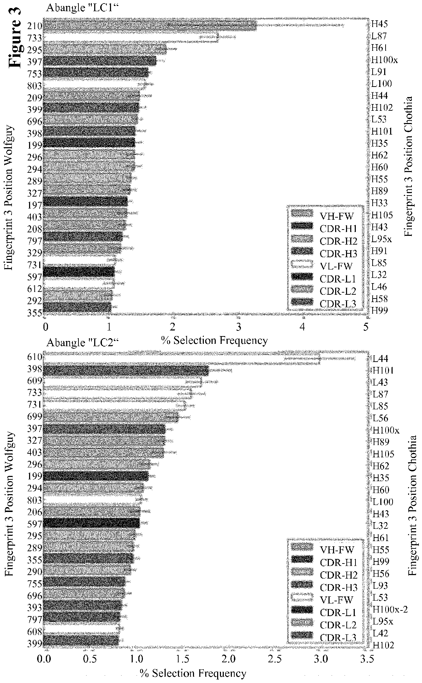

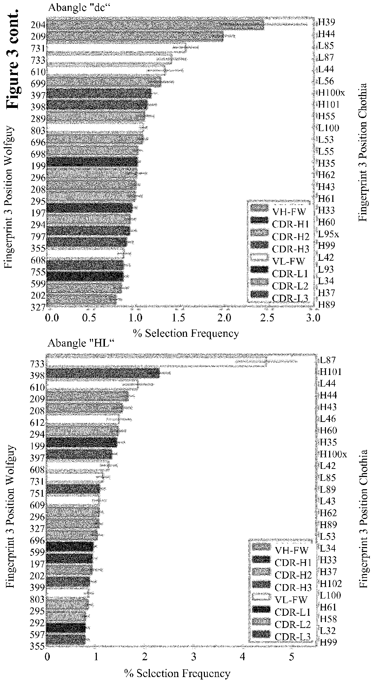

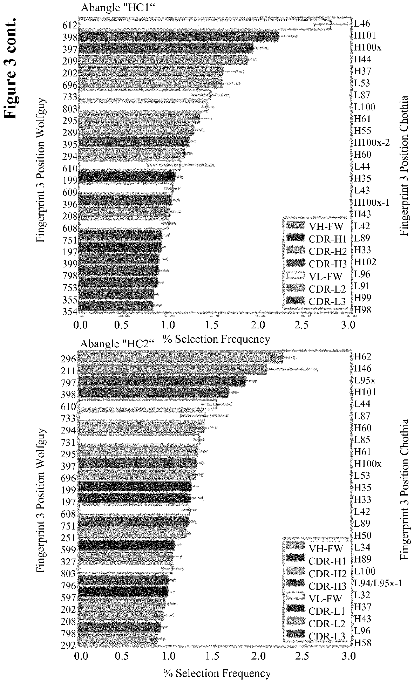

The importance ranking of the fingerprint positions as descriptors for the different ABangle parameters is depicted in Figure 3.

Based on the finding of the fingerprint position importance ranking it has been found that each ABangle parameter is influenced by a largely different set of interface positions on both VH and VL. For all parameters except HC2, a framework position was the most important descriptor. Nonetheless, in each case at least two HVR-H3 residues were among the most important descriptors. Positions that have been ranked among the top ten important input variables in the original ABangle publication (7) were tracked in the ranking presented herein, too. But, whereas Dunbar et al. (7) find HC1 to be exclusively determined by residues on the heavy chain, and LC1 exclusively determined by residues on the light chain, the top ten descriptors as determined with a method as reported herein for HC1 and LC1 involve fingerprint positions on both chains. Herein the fingerprint positions are ranked irrespective of amino acid specificity.

The top 25 ranked fingerprint positions also contain a number of members of the sets of VH-VL-orientation determining positions identified by Chailyan et al. (5) (L41, L42, L43, L44) and by Abhinandan and Martin (4) (L41, L44, L46, L87, H33, H45, H60, H62, H91, H105). It has been found that L87 is the top descriptor for HL, L46 for HC1, H45 for LC1, H62 for HC2, and L44 for LC2.

### Antibody Homology Modeling With VH-VL Reorientation

### MoFvAb Models

A detailed description of the MoFvAb (Modeling of the Fv for Antibody) procedure has been published by Bujotzek, A., et al. (mAbs 7 (2015) 838-852). The results obtained for model building Variant 1 (models assembled from template structures aligned on either consensus VH or VL framework, followed by VH-VL reorientation on an consensus Fv framework), are shown in Table 6.

**Table 6: AMAII models built with MoFvAb Variant 1. Values state the carbonyl RMSD for the fragments as defined by Teplyakov et al. (7) after chain-wise alignment on the β-sheet core.**

| **Model** | **Reference** | **VL** | **VH** | **L1** | **L2** | **L3** | **H1** | **H2** | **H3** | **H4** |
|---|---|---|---|---|---|---|---|---|---|---|
| Ab01 | 4MA3_B_A | 0.37 | 0.42 | 0.48 | 0.28 | 1.08 | 1.21 | 1.16 | 6.08 | 0.67 |
| Ab01 | 4MA3_H_L | 0.35 | 0.43 | 0.36 | 0.45 | 1.50 | 0.98 | 1.41 | 6.07 | 0.68 |
| Ab02 | 4KUZ_ H_L | 0.40 | 0.66 | 0.48 | 0.36 | 0.69 | 0.85 | 1.15 | 3.19 | 1.01 |
| Ab03 | 4KQ3_H_L | 0.34 | 0.44 | 0.38 | 0.35 | 0.51 | 2.41 | 0.35 | 2.09 | 0.88 |
| Ab04 | 4KQ4_H_L | 0.40 | 0.47 | 1.10 | 0.38 | 0.85 | 0.73 | 0.76 | 2.13 | 0.95 |
| Ab05 | 4M6M_ H_L | 0.53 | 0.40 | 1.28 | 0.38 | 1.96 | 0.28 | 0.29 | 2.93 | 0.37 |
| Ab06 | 4M6O_H_L | 0.37 | 0.59 | 0.42 | 0.40 | 0.86 | 0.88 | 0.62 | 3.57 | 0.89 |
| Ab07 | 4MAU_H_L | 0.38 | 0.40 | 0.54 | 0.52 | 0.81 | 0.57 | 0.44 | 2.10 | 0.59 |
| Ab08 | 4M7K_H_L | 0.35 | 0.46 | 0.75 | 0.21 | 0.77 | 0.61 | 0.79 | 2.69 | 0.43 |
| Ab09 | 4KMT_H_L | 0.32 | 0.37 | 0.29 | 0.39 | 0.29 | 0.47 | 0.97 | 2.89 | 0.38 |
| Ab10 | 4M61_B_A | 0.34 | 0.40 | 0.90 | 0.15 | 1.43 | 0.52 | 0.61 | 2.41 | 0.99 |
| Ab10 | 4M61_D_C | 0.31 | 0.41 | 1.16 | 0.22 | 1.47 | 0.78 | 0.58 | 2.42 | 0.42 |
| Ab11 | 4M43_ H_L | 0.34 | 0.61 | 0.37 | 0.25 | 1.01 | 1.07 | 0.54 | 2.95 | 0.35 |
| | | 0.37 | 0.47 | 0.65 | 0.33 | 1.02 | 0.87 | 0.74 | 3.20 | 0.66 |

In order to factor in carbonyl displacement caused by deviations in VH-VL-orientation, the same models were aligned on the β-sheet core of the complete Fv (VH and VL simultaneously) and recalculated the values. The results are shown in Table 7.

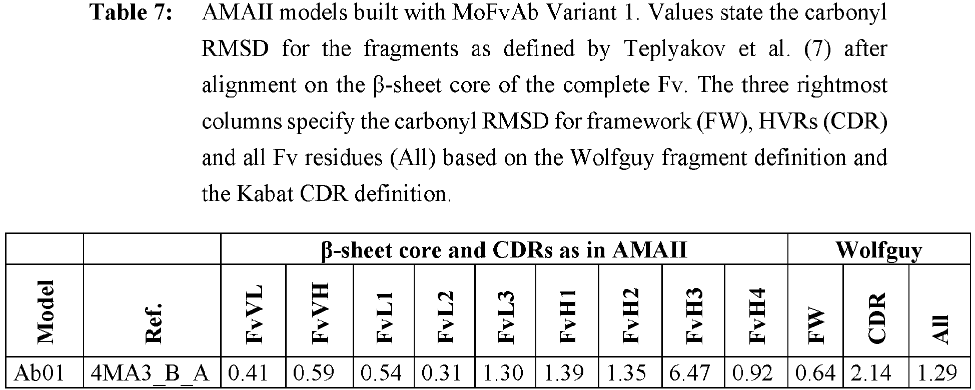

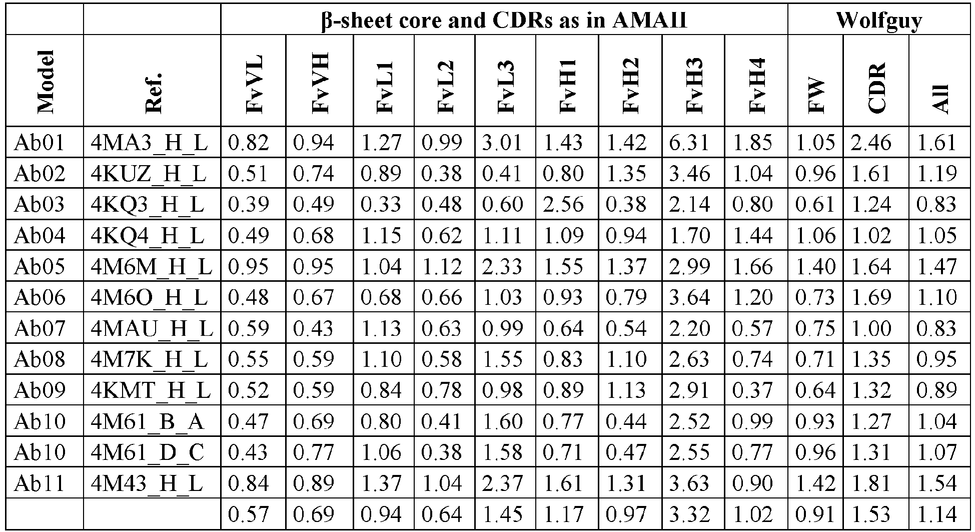

The comparison between Table 6 and Table 7 reveals how RMSD values deteriorate as soon as one considers the complete Fv structure. The mean carbonyl RMSD for the β-sheet core increased from 0.37 Å to 0.57 Å for VL, and from 0.47 Å to 0.69 Å for VH. This trend was not limited to the framework, but extended to the HVRs. The mean carbonyl RMSD for HVR-L3, for instance, increased from 1.02 Å to 1.45 Å, and from 3.20 Å to 3.32 Å for HVR-H3. The deviation in VH-VL-orientation by looking directly at the six ABangle parameters and the differences with regard to the reference structures is shown in Table 8.

**Table 8: Deviation in VH-VL-orientation with regard to the reference structure in terms of the six ABangle parameters for the AMAII models built with MoFvAb Variant 1.**

| **Model** | **Reference** | **ΔHL** | **AHC1** | **ΔLC1** | **ΔHC2** | **ΔLC2** | **Δdc** | **dist_{ABangle}** |
|---|---|---|---|---|---|---|---|---|
| Ab01 | 4ma3_B_A | 1.85 | 0.04 | 0.33 | 2.88 | 0.39 | 0.27 | 3.47 |
| Ab01 | 4ma3_ H_L | 7.72 | 2.46 | 0.07 | 4.51 | 0.78 | 0.03 | 9.31 |
| Ab02 | 4kuz_ H_L | 0.71 | 1.57 | 1.97 | 1.61 | 1.10 | 0.40 | 3.29 |
| Ab03 | 4kq3_H_L | 1.71 | 0.19 | 0.95 | 2.45 | 0.27 | 0.10 | 3.15 |
| Ab04 | 4kq4_H_L | 0.59 | 1.94 | 1.42 | 4.95 | 0.45 | 0.00 | 5.55 |
| Ab05 | 4m6m_H_L | 8.84 | 3.90 | 3.30 | 4.17 | 0.32 | 0.35 | 11.04 |
| Ab06 | 4m6o_ H_L | 3.73 | 1.61 | 0.00 | 0.54 | 0.51 | 0.28 | 4.14 |
| Ab07 | 4mau_ H_L | 2.60 | 2.33 | 3.87 | 0.38 | 2.28 | 0.23 | 5.71 |
| Ab08 | 4m7k_ H_L | 2.32 | 1.61 | 3.08 | 0.28 | 0.09 | 0.34 | 4.20 |
| Ab09 | 4kmt_ H_L | 2.47 | 0.98 | 2.08 | 3.82 | 1.36 | 0.05 | 5.28 |
| Ab10 | 4m61_B_A | 0.63 | 0.25 | 1.15 | 4.56 | 1.60 | 0.20 | 5.02 |
| Ab10 | 4m61_ D_ C | 0.98 | 0.70 | 0.31 | 5.52 | 0.94 | 0.29 | 5.74 |
| Ab11 | 4m43_H_L | 2.39 | 2.15 | 4.38 | 2.25 | 1.33 | 0.35 | 6.04 |
| | | 2.81 | 1.52 | 1.76 | 2.92 | 0.88 | 0.22 | 5.53 |

The models listed in Table 8 have been reoriented on the same consensus Fv framework and, thus, share essentially the same ABangle orientation of approximately *θ_{cons}* := (-59.45, 71.65, 120.49, 117.46, 82.77, 16.11). A large diversity in VH-VL-orientation that was inherent to the AMAII structures is shown. The largest deviations occurred for the parameters HL and HC2, not only between different structures, but also for sequence-identical structures from the same asymmetric unit: The parameter HL for 4MA3_B_A and 4MA3_H_L deviate by 5.87 degrees. This confirms that VH-VL-orientation, while being guided by certain sequence features (see Figure 3), is also subject to an intrinsic, undirected variability. This is especially pronounced for protein-binding antibodies in the unbound form (7).

All models were rebuild with model building Variant 2 (models assembled from template structures aligned on either consensus VH or VL framework, followed by VH-VL-reorientation on a VH-VL-orientation template structure chosen based on similarity to predicted ABangle parameters) using the same choice of template structures. The results are shown in Table 9 (values refer to model-reference pairs aligned on the β-sheet core of the complete Fv).

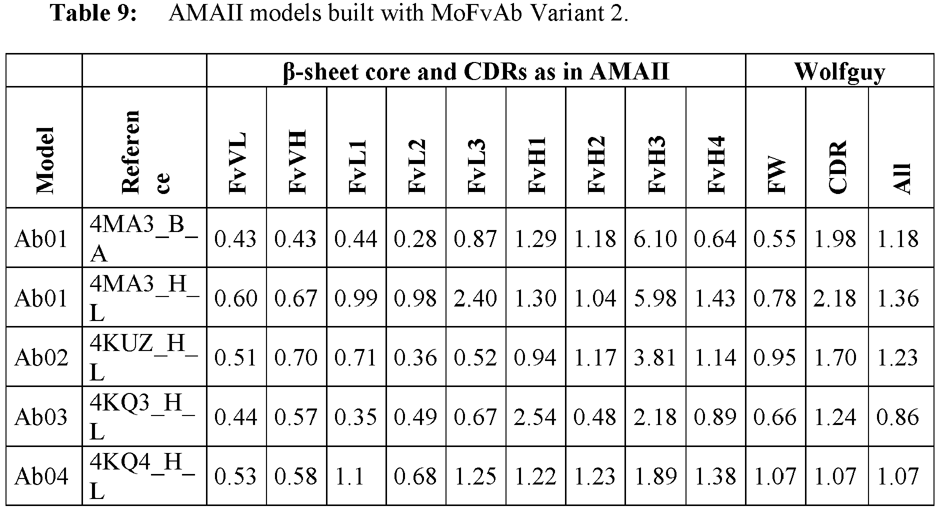

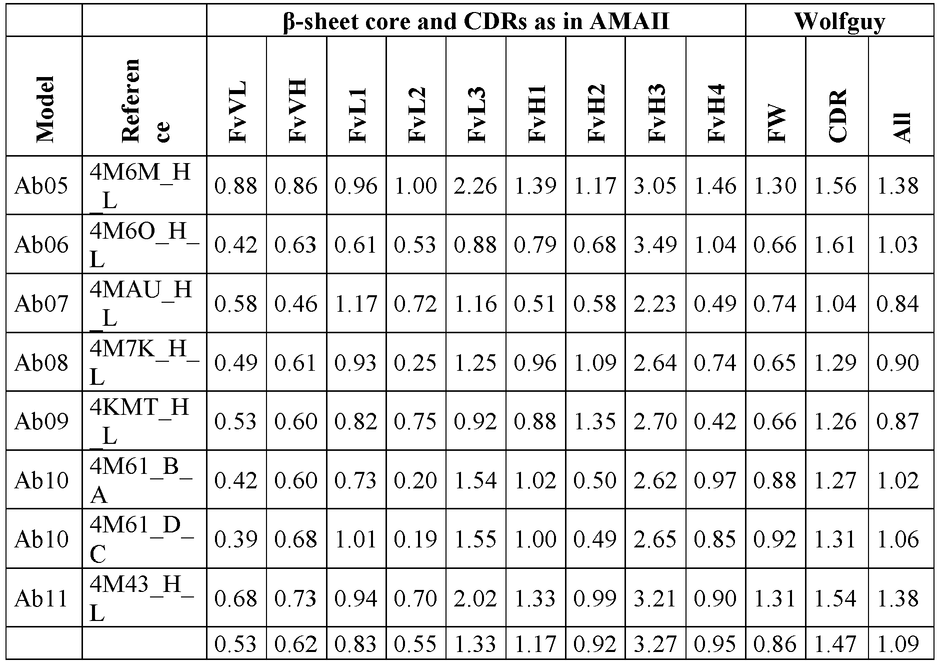

The mean carbonyl RMSD values per fragment calculated for the VH-VL-orientation-optimized Variant 2 models showed an improvement of approximately 0.05 Å in comparison to the models using a generic VH-VL-orientation (see Table 7). The ABangle deviations revealed that the Variant 2 models have moved closer to the actual VH-VL orientation of the reference structures (see Table 10).

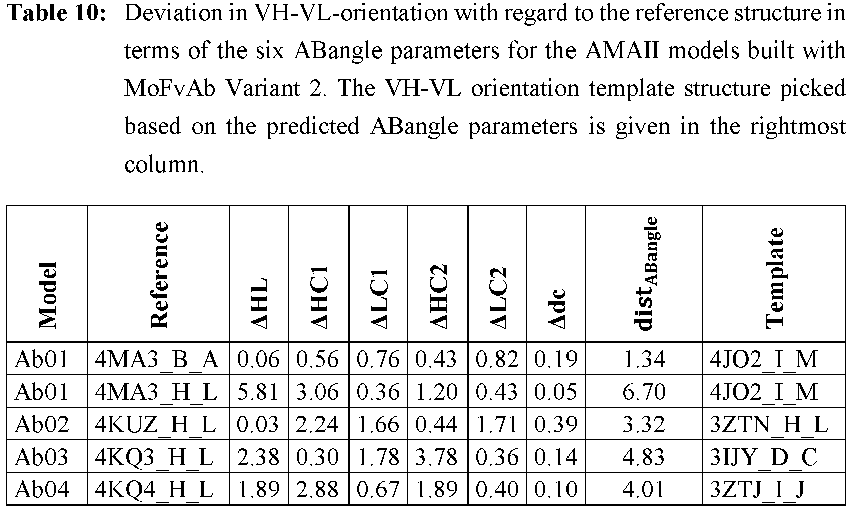

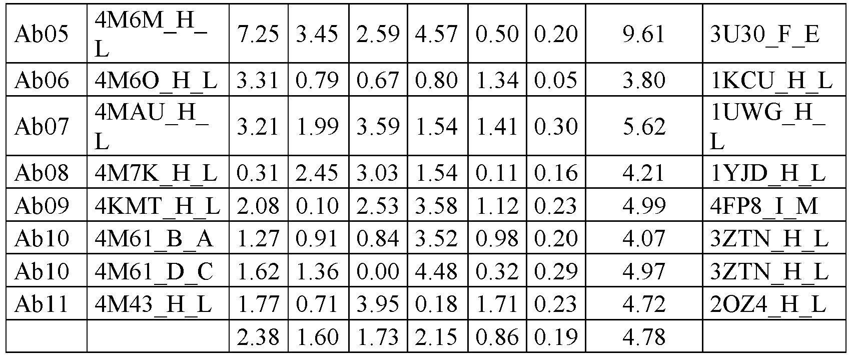

The mean dist_{ABangle} improved from 5.53 for the generic orientation models to 4.78 for the orientation-optimized versions. Shown in the rightmost column of Table are the VH-VL-orientation templates chosen based on the weighted distance d̅i̅s̅t̅_{ABangle} to the predicted ABangle parameters. The VH-VL-orientation templates were not picked based on fingerprint similarity but by similarity in ABangle orientation space.

All models were rebuild with model building Variant 3, using template structures aligned onto a common consensus Fv framework instead of a per-chain consensus structure, and VH-VL-orientation not being adjusted in any form. Due to the fact that all template structures were aligned per Fv, the chain-wise carbonyl RMSD (see Table 6) increased from 0.37 Å to 0.43 Å for VL and from 0.47 Å to 0.55 Å for VH (data not shown). The carbonyl RMSD values for the model-reference pairs aligned on the complete Fv are listed in Table 11.

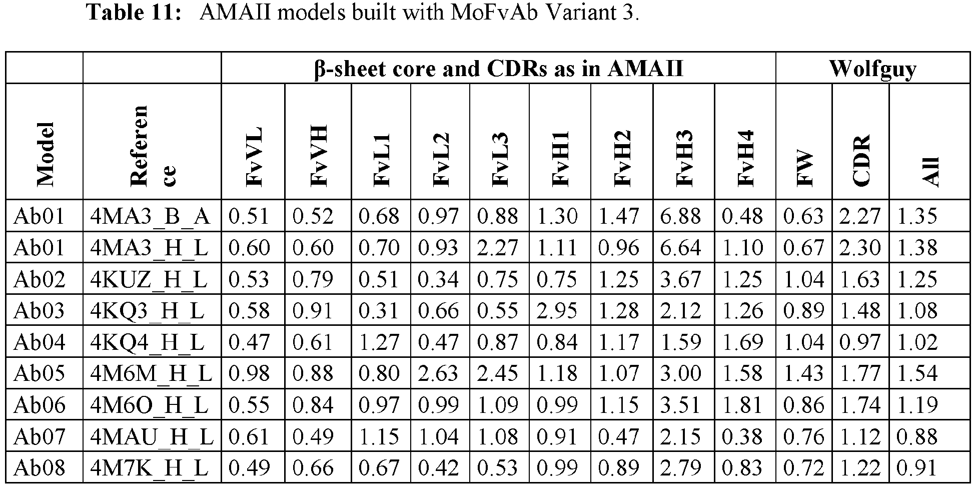

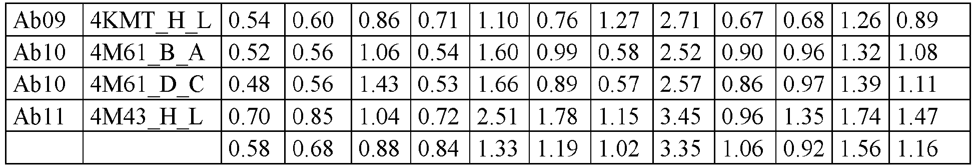

The results for Variant 3 were not as good as for the other two variants. Despite the fact that in Variant 3 template fragments from Fv structures with completely unrelated VH-VL-orientation were mixed, there seemed to be no particularly harmful effect on model quality. The corresponding ABangle deviations are shown in Table 12.

**Table 12: Deviation in VH-VL-orientation with regard to the reference structure in terms of the six ABangle parameters for the AMAII models built with MoFvAb Variant 3.**

| **Model** | **Reference** | **ΔHL** | **ΔHC1** | **ΔLC1** | **ΔHC2** | **ΔLC2** | **Δdc** | **dist_{ABangle}** |
|---|---|---|---|---|---|---|---|---|
| Ab01 | 4MA3_B_A | 2.99 | 0.21 | 0.59 | 0.89 | 0.20 | 0.52 | 3.23 |
| Ab01 | 4MA3_H_L | 2.88 | 2.29 | 0.99 | 0.74 | 0.59 | 0.28 | 3.94 |
| Ab02 | 4KUZ_ H_L | 3.53 | 1.10 | 3.38 | 2.46 | 2.69 | 0.33 | 6.20 |
| Ab03 | 4KQ3_H_L | 3.63 | 0.54 | 2.22 | 4.81 | 1.03 | 0.24 | 6.53 |
| Ab04 | 4KQ4_H_L | 0.91 | 2.09 | 0.71 | 1.25 | 0.73 | 0.10 | 2.79 |
| Ab05 | 4M6M_ H_L | 9.76 | 2.80 | 3.22 | 0.93 | 2.76 | 0.03 | 11.04 |
| Ab06 | 4M6O_H_L | 5.95 | 1.51 | 1.50 | 0.38 | 0.92 | 0.17 | 6.40 |
| Ab07 | 4MAU_H_L | 2.39 | 2.45 | 2.97 | 0.76 | 3.11 | 0.54 | 5.57 |
| Ab08 | 4M7K_H_L | 2.63 | 2.20 | 0.18 | 2.45 | 0.95 | 0.41 | 4.34 |
| Ab09 | 4KMT_ H_L | 3.25 | 0.11 | 2.32 | 0.55 | 0.18 | 0.11 | 4.04 |
| Ab10 | 4M61_ B_A | 0.92 | 0.39 | 3.26 | 0.41 | 0.08 | 0.29 | 3.45 |
| Ab10 | 4M61_ D_ C | 0.57 | 0.84 | 2.42 | 1.37 | 0.74 | 0.38 | 3.08 |
| Ab11 | 4M43_ H_L | 0.94 | 1.91 | 2.77 | 1.42 | 1.58 | 0.23 | 4.10 |
| | | 3.10 | 1.42 | 2.04 | 1.42 | 1.20 | 0.28 | 4.98 |

The mean dist_{ABangle} of the Variant 3 models was not as good as for the VH-VL-orientation optimized models, but better than for the models with the consensus Fv orientation produced by Variant 1. Without being bound by this theory, the template fragments fetched from structures aligned onto a common consensus Fv framework do encode some VH-VL orientation information that would be otherwise lost.

All data shown above refer to models minimized in the presence of position restraints on all residues but those that were remodeled or were situated at fragment edges with adjacent residues originating from different template structures. Hence, a maximum of VH-VL-orientation information conferred by the template structures and/or VH-VL-reorientation was preserved.

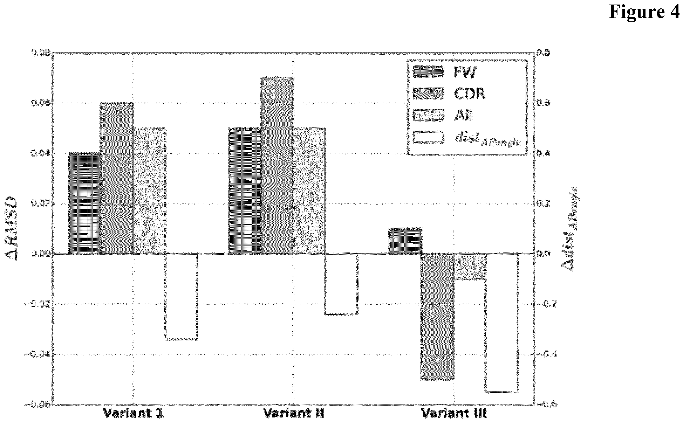

For comparison, the model building process was repeated and all models were minimized using the same force field and implicit water model combination (CHARMm and GBSW) while omitting the position restraints. For simplicity, only the average change in carbonyl RMSD and dist_{ABangle} when switching from restrained to fully flexible minimization is summarized (see Figure 4). For models built with MoFvAb Variant 1 and 2, the mean carbonyl RMSD with regard to the reference structures becomes slightly bigger. The Variant 3 models, due to their template structure setup probably most affected by steric inaccuracies, benefit from unrestrained energy minimization by a small margin. All three model variants improve in terms of dist_{ABangle} to the reference structures. Without being bound by this theory it appears that unrestrained energy minimization induces an equalization of model quality with regard to the different model building variants (force field/implicit water model combination).

### Original AMAII Models

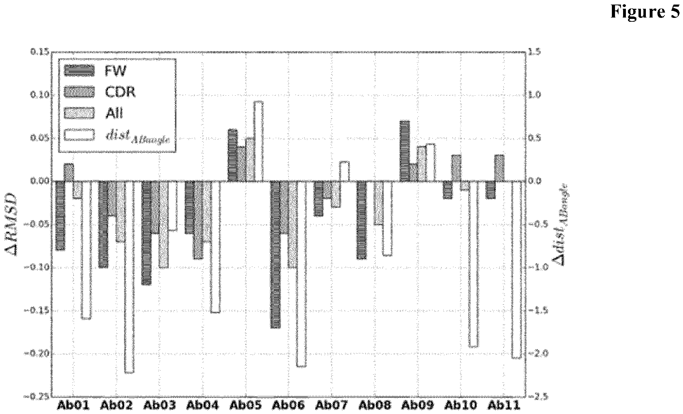

The approach of improving a given Fv homology model by reorienting it onto a VH-VL-orientation template as reported herein was integrated into current state-of-the-art modeling software. The original AMAII models were obtained from http://www.3dabmod.com, the structures were annotated with Wolfguy numbering so as to facilitate integration, and the models were reoriented onto the VH-VL-orientation templates as listed in Table 8. The mean change in carbonyl RMSD and dist_{ABangle} per antibody after reorientation, averaged over the respective models of all AMAII participants, is shown in Figure 5.

After chain-wise reorientation onto a different Fv structure (in particular without any post-processing) for eight of eleven sets of antibody models (constituted of all structures submitted by acc, ccg, jef, joa, mmt, pig and sch for the according Ab02), the carbonyl RMSD for the complete Fv backbone is improved by VH-VL-reorientation. Furthermore, the model sets Ab01, Ab10 and Ab11 improve in VH-VL orientation and framework RMSD.

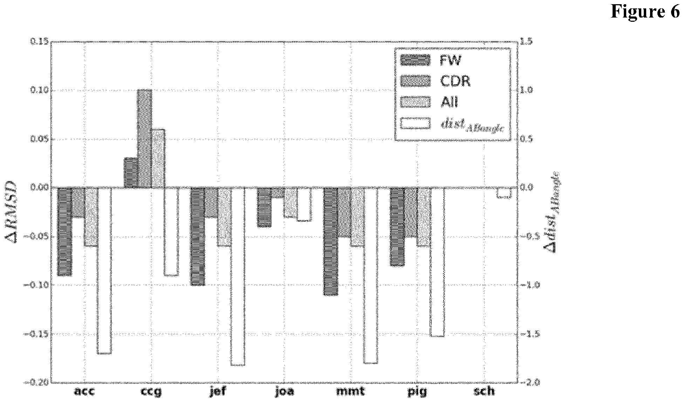

Finally, the model set was split in order to assess in how far the method as reported herein of VH-VL-reorientation agrees with antibody models built with different approaches. The mean change in carbonyl RMSD and dist_{ABangle} after reorientation, averaged over all models of the respective AMAII participant, is shown in Figure 6.

The mean dist_{ABangle} was improved with regard to the reference of structures for the models of all participants. The reduction of dist_{ABangle} by VH-VL-reorientation translated into better RMSD values in five of the seven cases, especially with regard to the framework regions.

On average, notable improvements of dist_{ABangle} and small improvements of the carbonyl RMSD for the whole Fv was found.

Thus, as reported herein the concept of VH-VL-orientation prediction based on sequence features can be extended by moving from a single VH-VL packing angle to a finer description of VH-VL orientation in terms of the six ABangle parameters defined by Dunbar et al. (7).

For each ABangle parameter, a random forest model was trained on an up-to-date set of known Fv structures. The Q² values for the six predictors range from 0.67 to 0.80 when trained on a set consisting only of complex structures.

An analysis of the top descriptors of our random forest models revealed a number of HVR-H3 residues that had not been known as such before. The herein reported antibody numbering scheme "Wolfguy" contributed to identifying of these residues, as it is designed such that structurally equivalent residues are numbered with equivalent indices as far as possible, also (and in particular) in the hypervariable regions.

Two model building variants without VH-VL-orientation prediction and adjustment (Variants 1 and 3) were compared with a model building variant that predicts the most likely VH-VL-orientation in terms of the six ABangle parameters, automatically looks up the most similarly oriented Fv structure in an antibody template database, and reorients the raw model onto this VH-VL orientation template prior to further processing (Variant 2).

Synergy effects with regard to modeling HVR-H3 loops due to improved preorientation of VH and VL are to be expected. Furthermore, the computational cost of optimizing VH-VL-orientation based on a sequence-based predictor and subsequent reorientation on a template structure is negligible (e.g. when compared to synthetic work).

### The current invention

It has been found that the total VH-VL-orientation difference between two (humanized) antibody variants binding to the same epitope of an antigen relative to its parent non-human antibody correlates with the respective difference in the antigen binding ability of the antibodies.

The VH-VL orientation is herein predicted from a (meaningful) subset of Fv sequence positions (a "sequence fingerprint") rather than from complete Fv sequences. Based on the assumption that VH-VL orientation is governed by residues on or near the VH-VL interface, a set of interface residues has been identified wherein a residue is defined to be part of the VH-VL interface if its side chain atoms are neighboring atoms of the opposite chain with a distance of less or equal than 4 Å in at least 90 % of all superimposed Fv structures in the database, e.g. in RAB3D. The results are summarized in Table 29, which also states if a sequence position has previously been connected to being a determinant of VH-VL orientation based on statistical analyses (4, 5, 7).

**Table 29: VH-VL interface residues where a residue is part of the interface if its side chain atoms are neighboring atoms of the opposite chain with a distance of less or equal than 4 Å in at least 90 % of all superpositioned Fv structures in RAB3D.**

| **Wolfguy Index** | **Chothia (14) Index** | **Wolfguy Region** | **Dunbar et al. (7)** | **Abhinandan, Martin (4)** | **Chailyan et al. (5)** |
|---|---|---|---|---|---|
| 199 | H35⁺ | CDR-H1 | X | | |
| 202 | H37⁺ | VH-FW2 | | | |
| 204 | H39⁺ | VH-FW2 | | | |
| 210 | H45⁺ | VH-FW2 | | X | |
| 212 | H47⁺ | VH-FW2 | | | |
| 251 | H50 | CDR-H2 | X | | |
| 292* | H58 | CDR-H2 | X | | |
| 294* | H60 | CDR-H2 | | X | |
| 295* | H61 | CDR-H2 | X | | |
| 329 | H91⁺ | VH-FW3 | | X | |
| 351 | H95 | CDR-H3 | | | |
| 352* | H96 | CDR-H3 | | | |
| 354* | H98 | CDR-H3 | | | |
| 395* | H100x-2* | CDR-H3 | | | |
| 396* | H100x-1* | CDR-H3 | | | |
| 397* | H100x* | CDR-H3 | | | |
| 398* | H101 | CDR-H3 | | | |
| 399 | H102 | CDR-H3 | | | |
| 401 | H103⁺ | VH-FW4 | | | |
| 403 | H105⁺ | VH-FW4 | | X | |
| 597* | L32 | CDR-L1 | | | |
| 599 | L34⁺ | CDR-L1 | X | | |
| 602 | L36⁺ | VL-FW2 | X | | X |
| 604 | L38⁺ | VL-FW2 | X | X | |
| 609 | L43⁺ | VL-FW2 | X | | X |
| 610 | L44⁺ | VL-FW2 | X | X | X |
| 612 | L46⁺ | VL-FW2 | X | X | |
| 615 | L49 | VL-FW2 | X | | |
| 651 | L50 | CDR-L2 | X | | |
| 698* | L55⁺ | CDR-L2 | X | | |
| 733 | L87⁺ | VL-FW3 | X | X | |
| 751 | L89 | CDR-L3 | X | | |
| 753* | L91 | CDR-L3 | X | | |
| 796* | L95x-1* | CDR-L3 | X | | |
| 797* | L95x* | CDR-L3 | X | | |
| 798* | L96 | CDR-L3 | X | | |
| 801 | L98⁺ | VL-FW4 | | | |

| | | | | | |
|---|---|---|---|---|---|
| *Numbering depending on loop length +Part of the VH-VL interface as defined by Chothia et al. (13) | | | | | |

The above set of interface residues is missing some of the sequence positions that had been listed among the "top 10 important input variables" for VH-VL orientation by Dunbar et al. (7). Those sequence positions are listed in the following Table 30.

**Table 30: Additional sequence positions listed among the "top 10 important input variables" for VH-VL orientation by Dunbar et al. (7).**

| **Wolfguy Index** | **Chothia (14) Index** | **Wolfguy Region** | **Abhinandan, Martin (4)** | **Chailyan et al. (5)** |
|---|---|---|---|---|
| 197* | H33 | CDR-H1 | X | |
| 208 | H43 | VH-FW2 | | |
| 209 | H44⁺ | VH-FW2 | | |
| 211 | H46 | VH-FW2 | | |
| 296* | H62 | CDR-H2 | X | |
| 327 | H89 | VH-FW3 | | |
| 355* | H99 | CDR-H3 | | |
| 607 | L41 | VL-FW2 | X | X |
| 608 | L42 | VL-FW2 | | X |
| 611 | L45 | VL-FW2 | | |
| 696* | L53 | CDR-L2 | | |
| 699 | L56 | CDR-L2 | | |
| 731 | L85 | VL-FW3 | | |
| 755* | L93 | CDR-L3 | | |
| 796* | L94 | CDR-L3 | | |
| 799 | L97 | CDR-L3 | | |
| 803 | L100⁺ | VL-FW4 | | |

| | | | | |
|---|---|---|---|---|
| *Numbering depending on loop length +Part of the VH-VL interface as defined by Chothia et al. (13) | | | | |

In order to select an appropriate (humanized) variant antibody of a parent antibody the VH-VL-orientation is described in terms of the six "ABangle" orientation parameters, consisting of one torsion angle, four bend angles (two per variable domain), as well as the length of the pivot axis of VH and VL.

It has been found that the relative orientation between the VH- and VL-domains (VH-VL orientation) can be used to (pre)select the variant antibody(ies) with the best binding affinity. This is applicable within a group of humanized antibodies as well as to between a group of humanized antibodies and the parent non-human antibody.

Furthermore, it has been found that each time a framework residue or an entire framework has to be (ex)changed, the binding of the new variant to its antigen can be evaluated based on the method as reported herein.

The invention is in the following exemplified with specific antibodies which are intended to serve as an example and should not be construed to limit the scope of the invention thereto. The method as reported herein is a generally applicable method.

The sequence fingerprint consists of 54 amino acids, 29 in the VH region, and 25 in the VL region. See following Table 13.

**Table 13: Sequence fingerprint.**

| **Wolfguy Index** | **Chothia (14) Index** | **Wolfguy Region** |
|---|---|---|
| 199 | H35⁺ | CDR-H1 |
| 202 | H37⁺ | VH-FW2 |
| 204 | H39⁺ | VH-FW2 |
| 210 | H45⁺ | VH-FW2 |
| 212 | H47⁺ | VH-FW2 |
| 251 | H50 | CDR-H2 |
| 292* | H58 | CDR-H2 |
| 294* | H60 | CDR-H2 |
| 295* | H61 | CDR-H2 |
| 329 | H91⁺ | VH-FW3 |
| 351 | H95 | CDR-H3 |
| 352* | H96 | CDR-H3 |
| 354* | H98 | CDR-H3 |
| 395* | H100x-2* | CDR-H3 |
| 396* | H100x-1* | CDR-H3 |
| 397* | H100x* | CDR-H3 |
| 398* | H101 | CDR-H3 |
| 399 | H102 | CDR-H3 |
| 401 | H103⁺ | VH-FW4 |
| 403 | H105⁺ | VH-FW4 |
| 597* | L32 | CDR-L1 |
| 599 | L34⁺ | CDR-L1 |
| 602 | L36⁺ | VL-FW2 |
| 604 | L38⁺ | VL-FW2 |
| 609 | L43⁺ | VL-FW2 |
| 610 | L44⁺ | VL-FW2 |
| 612 | L46⁺ | VL-FW2 |
| 615 | L49 | VL-FW2 |
| 651 | L50 | CDR-L2 |
| 698* | L55⁺ | CDR-L2 |
| 733 | L87⁺ | VL-FW3 |
| 751 | L89 | CDR-L3 |
| 753* | L91 | CDR-L3 |
| 796* | L95x-1* | CDR-L3 |
| 797* | L95x* | CDR-L3 |
| 798* | L96 | CDR-L3 |
| 801 | L98⁺ | VL-FW 4 |
| 197* | H33 | CDR-H1 |
| 208 | H43 | VH-FW2 |
| 209 | H44⁺ | VH-FW2 |
| 211 | H46 | VH-FW2 |
| 296* | H62 | CDR-H2 |
| 327 | H89 | VH-FW3 |
| 355* | H99 | CDR-H3 |
| 607 | L41 | VL-FW2 |
| 608 | L42 | VL-FW2 |
| 611 | L45 | VL-FW2 |
| 696* | L53 | CDR-L2 |
| 699 | L56 | CDR-L2 |
| 731 | L85 | VL-FW3 |
| 755* | L93 | CDR-L3 |
| 796* | L94 | CDR-L3 |
| 799 | L97 | CDR-L3 |
| 803 | L100⁺ | VL-FW 4 |

| | | |
|---|---|---|
| *Numbering depending on loop length +Part of the VH-VL interface as defined by Chothia et al. (13) | | |

In the first example two murine antibodies, CD81K04 and CD81K13 binding to the large extra-cellular loop (LEL) of the CD81 receptor extracellular domain (ECD) and humanized variants thereof are evaluated according to the methods as reported herein.

In the second example, a rabbit antibody recognizing a peptide segment from the pTau protein (including the S422 phosphorylation) and its humanized variants are evaluated according to the methods as reported herein.

In the third example, an anti-Hepsin antibody and its humanized variants are evaluated according to the methods as reported herein.

In the sequence alignments the HVRs are marked with a gray background. The HVR definition used corresponds to the set union of the Kabat and Chothia CDR definition. Sequence positions that are part of the sequence fingerprint used for predicting VH-VL-orientation are marked with a black background. Fingerprint positions that are unpopulated in a given antibody are marked with an 'X'.

### Original VH sequences of CD81K04, CD81K13 (murine) and Rb86:

FR1 HVR-H1
CD81K04 QVQLQQSGPELVKPGASVKISCKAS GYTFSSS[?]M[?]
CD81K13 EVRLHQSAAQLVQPGASVRLSCTTS GFNFKDSYLH
Rb86 -QSVEESGGRLVTPGTPLTLTCTVS GFSLSSNAIN

FR2
W K RPG     IG
WVKQRPAQGLEWIG
WVRQAPGKGLEWIG

HVR-H2 FR3
[?]IYSGD[?][?]A[?]Y[?][?][?]FKG KATLTADKSSSTAYMQLSSLTSEDSA[?]Y[?]CAR
RIDTGNGNVKFDPKFQD KATITTDIPSMTAYLHLSNLTSEDTAVYYCVP
YIAV-SGNTYYASWAKG RFTISKAS--TTVDLKMTSPTAEDTGTYFCGK

HVR-H3 FR4
  K  D       G GSALTVSS (SEQ ID NO: 01)
YGYXX-XGFHS WGDGTTLTVSS (SEQ ID NO: 02)
SX-XX-XXXNI WGPGTLVTVSL (SEQ ID NO: 03)

### Original VL sequences of CD81K04, CD81K13 (murine) and Rb86:

FR1 HVR-L1
CD81K04 DIVLTQSPASLSVSLGQRATISC RASKSVSTSIYSYMH
CD81K13 DIQMTQSPASLSVSVGETVTITC RASENIY----RTLA
Rb86 AQVLTQTTSPVSAAVGSTVTISC QSSQSVRT---NKLA

FR2 HVR-L2
WYQQKPGQPPKLLIK YASYLES
WYLQKQGKSPQLLVY GATTLAD
WFQQKPGQPPKRLIY SASTLDF

FR3
GVPARFSGSGSGTDFTLNIHPVEEEDAATYYC
GVPSRFSGSGSGTQYYLKINSLQSEDFGTYHC
GVPSRFSASGSGTQFTLTISDVQCDDAATYYC

HVR-L3 FR4
EHSRE----FPFT FGTGTKLEIK (SEQ ID NO: 04)
QHFWG----TPWT FGGGTKVEIK (SEQ ID NO: 05)
LGYFDCSIADCVA FGGGTEVVVK (SEQ ID NO: 06)

### CD81K04 VH humanization variants (the original murine sequence is shown on top):

FR1
CD81K04 QVQLQQSGPELVKPGASVKISCKAS
JA_GG-14-hVH_1_69 
JA_GG-14-hVH_1_69-GA 
GG-04-hVH_1_69 
GG-02-hVH_1_69 
GG-03-hVH_1_69 
GG-06-hVH_1_69 
JA_GG-13-hVH_1_69 
GG-01-hVH_5_51 EVQLVQSGAEVKKPGESLKISCKGS
GG-07-hVH_5_51 EVQLVQSGAEVKKPGESLKISCKGS
GG-05-hVH_1_18 
JA_GG-14-hVH_1_3 
JA_GG-16-hVH_1_3 
JA_GG-15-hVH_1_3 
JA-13-hVH_1_3 
JA_GG-17-hVH_1_3 

HVR-H1 FR2 HVR-H2
GYTFSSS[?]M[?] WVRQAPGQRLEWMG RIYSGDGDTIYSQKFQG
GYTFSSSWMN WVRQAPGQRLEWMG RIYSGDGDAIYNGKFKG
GYTFSSSWMN WVRQAPGQRLEWMG RIYSGDGDAIYNGKFKG
GYTFSSSWMN WVRQAPGQGLEWMG RIYSGDGDAIYNGKFKG
GYTFSSSWMN WVRQAPGQGLEWMG RIYSGDGDAIYNGKFKG
GYTFSSSWMN WVRQAPGQGLEWMG RIYSGDGDAIYAQKLQG
GYTFSSSWMC WVRQMPGKGLEWMG CIYSGDGDAIYSPSFQG
GGTFSSSWMN WVRQMPGKGLEWMG RIYSGDGDAIYSPSFQG
GYSFSSSWMN WVRQAPGQGLEWMG RIYSGDGDAIYAQKFQG
GYSFSSSWMC WVRQAPGQGLEWMG CIYSGDGDAIYNQKFQG
GYTFSSSWMN WVRQAPGQGLEWMG RIYSGDGDAIYAQKFQG
GYTFSSSWMN WVRQAPGQGLEWMG RIYSGDGDAIYNQKFQG

GYTFSSSWMN WVRQAPGQGLEWMG RIYSGDGDAIYNQKFQG
GYTFSSSWMN WVRQAPGQGLEWMG RIYSGDGDAIYNGKFKG
GYTFSSSWMN WVRQAPGQGLEWMG RIYSGDGDAIYNGKFKG
GYTFSSSWMN W[?]K[?]RPG[?][?][?][?][?]IG [?]IYSGD[?][?]A[?]Y[?][?][?]FKG

FR3
KATLTADKSSSTAYMQLSSLTSEDSA Y CAR
KATITADESTSTAYMELSSLRSEDTAVYYCAR
KATITADESTSTAYMELSSLRSEDTAVYYCAR
RATITADESTSTAYMELSSLRSEDTAVYYCAR
RVTITADESTSTAYMELSSLRSEDTAVYYCAR
RVTITADESTSTAYMELSSLRSEDTAVYYCAR
RVTITADESTSTAYMELSSLRSEDTAVYYCAR
RVTITADESTSTAYMELSSLRSEDTAVYYCAR
QVTISADKSISTAYLQLSSLKASDTAMYYCAR
QVTISADKSISTAYLQLSSLKASDTAMYYCAR
RVTMTTDTSTSTAYMELRSLRSDDTAVYYCAR
RATITADTSASTAYMELSSLRSEDTAVYYCAR
RVTITADTSASTAYMELSSLRSEDTAVYYCAR
RVTITADTSASTAYMELSSLRSEDTAVYYCAR
RVTITRDTSASTAYMELSSLRSEDTAVYYCAR
RVTITADTSASTAYMELSSLRSEDTAVYYCAR

HVR-H3 FR4
  K  D       G GSALTVSS (SEQ ID NO: 01)
EGKTGDLLLRS WGQGTLVTVSS (SEQ ID NO: 07)
EGKTGDLLLRS WGQGTLVTVSS (SEQ ID NO: 08)
EGKTGDLLLRS WGQGTLVTVSS (SEQ ID NO: 09)
EGKTGDLLLRS WGQGTLVTVSS (SEQ ID NO: 10)
EGKTGDLLLRS WGQGTLVTVSS (SEQ ID NO: 11)
EGKTGDLLLRS WGQGTLVTVSS (SEQ ID NO: 12)
EGKTGDLLLRS WGQGTLVTVSS (SEQ ID NO: 13)
EGKTGDLLLRS WGQGTLVTVSS (SEQ ID NO: 14)
EGKTGDLLLRS WGQGTLVTVSS (SEQ ID NO: 15)
EGKTGDLLLRS WGQGTLVTVSS (SEQ ID NO: 16)
EGKTGDLLLRS WGQGTLVTVSS (SEQ ID NO: 17)
EGKTGDLLLRS WGQGTLVTVSS (SEQ ID NO: 18)
EGKTGDLLLRS WGQGTLVTVSS (SEQ ID NO: 19)
EGKTGDLLLRS WGQGTLVTVSS (SEQ ID NO: 20)
EGKTGDLLLRS WGQGTLVTVSS (SEQ ID NO: 21)

### CD81K04 VL humanization variants (the original murine sequence is shown on top):

FR1
CD81K04 DIVLTQSPASLSVSLGQRATISC
JA-10-hVK_4_1 DIVMTQSPDSLAVSLGERATINC
JA_GG-08-hVK_4_1 DIVMTQSPDSLAVSLGERATINC
JA_GG-09-hVK_4_1 DIVMTQSPDSLAVSLGERATINC

GG-02-hVK_4_1 DIVMTQSPDSLAVSLGERATINC
GG-03-hVK_4_1 DIVMTQSPDSLAVSLGERATINC
GG-04-hVK_4_1 DIVMTQSPDSLAVSLGERATINC
GG-05-hVK_3_11 EIVLTQSPATLSLSPGERATLSC
GG-06-hVK_1_39 DIQMTQSPSSLSASVGDRVTITC
JA_GG-07-hVK_7_3 DIVLTQSPASLAVSPGQRATITC

HVR-L1 FR2 HVR-L2
RASKSVSTSIYSYMH WYQQKPGQPPKLLIK YASYLES
RASKSVSTSIYSYMH WYQQKPGQPPKLLIK YASYLES
KSSKSVSTSIYSYMH WYQQKPGQPPKLLIK YASYLES
KSSKSVSTSIYSYMH WYQQKPGQPPKLLIK YASTRES
KSSKSVSTSIYSYMH WYQQKPGQPPKLLIY YASYRES
KSSKSVSTSIYSYLA WYQQKPGQPPKLLIY YASYRES
KSSKSVSTSIYSYLA WYQQKPGQPPKLLIY YASTRES
RASKSVSTSIYSYLH WYQQKPGQAPRLLIY YASNRET
RASKSVSTSIYSYLN WYQQKPGKAPKLLIY YASYLQS
RASKSVSTSIYSYMH WYQQKPGQPPKLLIK YASNKDT

FR3
GVPARFSGSGSGTDFTLNIHPVEEEDAATYYC
GVPDRFSGSGSGTDFTLTISSLQAEDVAVYYC
GVPDRFSGSGSGTDFTLTISSLQAEDVAVYYC
GVPDRFSGSGSGTDFTLTISSLQAEDVAVYYC
GVPDRFSGSGSGTDFTLTISSLQAEDVAVYYC
GVPDRFSGSGSGTDFTLTISSLQAEDVAVYYC
GVPDRFSGSGSGTDFTLTISSLQAEDVAVYYC
GIPARFSGSGSGTDFTLTISSLEPEDFAVYYC
GVPSRFSGSGSGTDFTLTISSLQPEDFATYYC
GVPARFSGSGSGTDFTLTINPVEANDAANYYC

HVR-L3 FR4
EHSRE----FPFT FGTGTKLEIK (SEQ ID NO: 04)
EHSRE----FPFT FGQGTKLEIK (SEQ ID NO: 22)
EHSRE----FPFT FGQGTKLEIK (SEQ ID NO: 23)
EHSRE----FPFT FGQGTKLEIK (SEQ ID NO: 24)
QHSRE----FPFT FGQGTKLEIK (SEQ ID NO: 25)
QHSRE----FPFT FGQGTKLEIK (SEQ ID NO: 26)
QHSRE----FPYT FGQGTKLEIK (SEQ ID NO: 27)
QHSRE----FPFT FGQGTKLEIK (SEQ ID NO: 28)
QHSRE----FPYT FGQGTKLEIK (SEQ ID NO: 29)
LHSRE----FPYT FGQGTKLEIK (SEQ ID NO: 30)

### CD81K13 VH humanization variants (the original murine sequence is shown on top):

FR1 HVR-H1
CD81K13 EVRLHQSAAQLVQPGASVRLSCTTS GFNFKDSYLH

01_hVH_1_f      EVQLVQSGAEVKKPGATVKISCKVS GYTFTDSYMH
02_hVH_1_3      QVQLVQSGAEVKKPGASVKVSCKAS GYTFTDSYMH
03_hVH_1_69     QVQLVQSGAEVKKPGSSVKVSCKAS GFNFKDSYLH
04_hVH_1_69     EVQLVESGGGLVQPGRSLRLSCTAS GFNFKDSYLH
05_hVH_1_69     QVQLVQSGAEVKKPGSSVKVSCKAS GFNFKDSYLH
5b_hVH_1_69     QVQLVQSGAELVKPGSSVKVSCKAS GFNFKDSYLH
5b_hVH_1_69-GA QVQLVQSGAELVKPGSSVKVSCKAS GFNFKDSYLH
06_hVH_1_3      QVQLVQSGAEVKKPGASVKVSCKAS GYTFKDSYLH
07_hVH_1_3      QVQLVQSGAEVKKPGASVKVSCKAS GYTFKDSYLH

FR2 HVR-H2
WVKQRPAQGLEWIG RIDTGNGNVKFDPKFQD
WVQQAPGKGLEWMG RIDTGNGNVKFDPKFQG
WVRQAPGQRLEWMG RIDTGNGNTKYSQKFQG
WVRQAPGQGLEWMG RIDTGNGNVKYAQKFQG
WVRQAPGQGLEWMG RIDTGNGNVKFDPKFQG
WVRQAPGQGLEWMG RIDTGNGNVKFDPKFQG
WVRQAPGQGLEWMG RIDTGNGNVKFDPKFQG
WVRQAPGQGLEWMG RIDTGNGNVKFDPKFQG
WVRQAPGQRLEWMG RIDTGNGNVKFDPKFQG
WVRQAPGQGLEWMG RIDTGNGNVKFDPKFQG

FR3
KATITTDIPSMTAYLHLSNLTSEDTAVYYCVP
RVTITADTSTDTAYMELSSLRSEDTAVYYCAP
RVTITTDTSASTAYMELSSLRSEDTAVYYCAP
RVTITADESTSTAYMELSSLRSEDTAVYYCAP
RVTITADESTSTAYMELSSLRSEDTAVYYCVP
RVTITTDESTSTAYMELSSLRSEDTAVYYCVP
RVTITTDESTSTAYMELSSLRSEDTAVYYCVP
RVTITTDESTSTAYMELSSLRSEDTAVYYCVP
RVTITTDTSASTAYMELSSLRSEDTAVYYCAP
RVTITTDTSASTAYMELSSLRSEDTAVYYCAP

HVR-H3 FR4
YGYXX-XGFHS WGDGTTLTVSS (SEQ ID NO: 02)
YGYXX XGFHS WGQGTLVTVSS (SEQ ID NO: 31)
YGYXX-XGFHS WGQGTLVTVSS (SEQ ID NO: 32)
YGYXX-XGFHS WGQGTLVTVSS (SEQ ID NO: 33)
YGYXX-XGFHS WGQGTLVTVSS (SEQ ID NO: 34)
YGYXX-XGFHS WGQGTLVTVSS (SEQ ID NO: 35)
YGYXX-XGFHS WGQGTLVTVSS (SEQ ID NO: 36)
YGYXX-XGFHS WGQGTLVTVSS (SEQ ID NO: 37)
YGYXX-XGFHS WGQGTLVTVSS (SEQ ID NO: 38)
YGYXX-XGFHS WGQGTLVTVSS (SEQ ID NO: 39)

### CD81K13 VL humanization variants (the original murine sequence is shown on top):

FR1 HVR-L1
CD81K13 DIQMTQSPASLSVSVGETVTITC RASENIY----RTLA
01_hVK_3_15 EIVMTQSPATLSVSPGERATLSC RASENIY----RTLA
1b_hVK_3_15 EIVMTQSPATLSVSPGERATLSC RASENIY----RTLA
1c_hVK_3_15 EIVMTQSPATLSVSPGERATLSC RASENIY----RTLA
03_hVK_1_9 DIQLTQSPSFLSASVGDRVTITC RASENIY----RTLA
04_hVK_1_9 DIQLTQSPSFLSASVGDRVTITC RASENIY----RTLA
05_hVK_1_39 DIQMTQSPSSLSASVGDRVTITC RASENIY----RTLA
5b_hVK_1_39 DIQMTQSPSSLSASVGDRVTITC RASENIY----RTLA
5b_hVK_1_39-GA DIQMTQSPSSLSASVGDRVTITC RASENIY----RTLA
06_hVK_1_39 DIQMTQSPSSLSASVGDRVTITC RASENIY----RTLA
07_hVK_1_27 DIQMTQSPSSLSASVGDRVTITC RASENIY----RTLA

FR2 HVR-L2
WYLQKQGKSPQLLVY GATTLAD
WYQQKPGQAPRLLIY GATTRAT
WYQQKPGQAPRLLIY GATTLAD
WYQQKPGQAPRLLIY GATTLAD
WYQQKPGKAPKLLIY AATTLAS
WYQQKPGKAPKLLIY AATTLAS
WYQQKPGKAPKLLIY AATTLAS
WYQQKPGKAPKLLIY GATTLAD
WYQQKPGKAPKLLIY GATTLAD
WYQQKPGKAPKLLIY AATTLAS
WYQQKPGKVPKLLIY AATTLAS

FR3
GVPSRFSGSGSGTQYYLKINSLQSEDFGTYHC
GIPARFSGSGSGTEFTLTISSLQSEDFGVYYC
GIPARFSGSGSGTEFTLTISSLQSEDFAVYYC
GIPARFSGSGSGTEFTLTISSLQSEDFGVYYC
GVPSRFSGSGSGTEFTLTISSLQPEDFATYYC
GVPSRFSGSGSGTEFTLTISSLQPEDFGTYYC
GVPSRFSGSGSGTDFTLTISSLQPEDFATYYC
GVPSRFSGSGSGTDFTLTISSLQPEDFATYYC
GVPSRFSGSGSGTDFTLTISSLQPEDFATYYC
GVPSRFSGSGSGTDFTLTISSLQPEDFGTYYC
GVPSRFSGSGSGTDFTLTISSLQPEDVGTYYC

HVR-L3 FR4
QHFWG----TPWT FGGGTKVEIK (SEQ ID NO: 05)
QHFWG----TPWT FGQGTKVEIK (SEQ ID NO: 40)
QHFWG----TPWT FGQGTKVEIK (SEQ ID NO: 41)
QHFWG----TPWT FGQGTKVEIK (SEQ ID NO: 42)
QHFWG----TPWT FGQGTKVEIK (SEQ ID NO: 43)
QHFWG----TPWT FGQGTKVEIK (SEQ ID NO: 44)
QHFWG----TPWT FGQGTKVEIK (SEQ ID NO: 45)

QHFWG----TPWT FGQGTKVEIK (SEQ ID NO: 46)
QHFWG----TPWT FGQGTKVEIK (SEQ ID NO: 47)
QHFWG----TPWT FGQGTKVEIK (SEQ ID NO: 48)
QKFWG----TPWT FGQGTKVEIK (SEQ ID NO: 49)

### Rb86 VH humanization variants (the original rabbit sequence is shown on top):

FR1
Rb86 -QSVEESGGRLVTPGTPLTLTCTVS
001_IGHV3_23_04 -QQLVESGGGLVQPGGSLRLSCAAS
002--IMGT_hVH_3_23 -QSVLESGGGLVQPGGSLRLSCAAS
003--IMGT_hVH_3_23 -QSVLESGGGLVQPGGSLRLSCAVS
004--IMGT_hVH_3_23 -QSVLESGGGLVQPGGSLRLSCAVS
005--IMGT_hVH_3_23 -QSLLESGGGLVQPGGSLRLSCAAS
006--IMGT_hVH_3_30_3 -QSLVESGGGVVQPGRSLRLSCAAS
007--IMGT_hVH_3_30_3 -QSVVESGGGVVQPGRSLRLSCAAS
009--IMGT_hVH_1_18 -QSVVQSGAEVKKPGASVKVSCKAS
010--IMGT_hVH_1_18 -QSVVQSGAEVKKPGASVKVSCKAS
011--IMGT_hVH_3_66 -QQLVESGGGLVQPGGSLRLSCAAS
012--IMGT_hVH_3_66 -QQLVESGGGLVQPGGSLRLSCAAS
013--IMGT_hVH_3_66 -QQLVESGGGLVQPGGSLRLSCAAS
014--IMGT_hVH_3_66 -QQLVESGGGLVQPGGSLRLSCAAS
015--IMGT_hVH_3_66 -QSVVESGGGLVQPGGSLRLSCAAS
016--IMGT_hVH_3_53 -QSVVESGGGLIQPGGSLRLSCAAS

HVR-H1 FR2 HVR-H2
GFSLSSNAIN WVRQAPGKGLEWIG YIAV-SGNTYYASWAKG
GFSLSSNAIN WVRQAPGKGLEWVG YIAV-SGNTYYASWAKG
GFSLSSNAIN WVRQAPGKGLEWVG YIAV-SGNTYYASWAKG
GFSLSSNAIN WVRQAPGKGLEWVG YIAV-SGNTYYASWAKG
GFSLSSNAIN WVRQAPGKGLEWVG YIAV-SGNTYYASWAKG
GFTLSSNAIN WVRQAPGKGLEWVS YIAV-SGNTYYASWAKG
GFTLSSNAIN WVRQAPGKGLEWVA YIAV-SGNTYYASWAKG
GFSLSSNAIN WVRQAPGKGLEWVG YIAV-SGNTYYASWAKG
GYTLSSNAIN WVRQAPGQGLEWMG YIAV-SGNTYYASWAQG
GYTLSSNAIN WVRQAPGQGLEWMG YIAV-SGNTYYASWAQG
GFTVSSNAIN WVRQAPGKGLEWVS YIAV-SGNTYYADSVKG
GFTVSSNAIN WVRQAPGKGLEWVS YIAV-SGNTYYASWAKG
GFTVSSNAIN WVRQAPGKGLEWVS YIAV-SGNTYYASWAKG
GFSLSSNAIN WVRQAPGKGLEWVG YIAV-SGNTYYASWAKG
GFSLSSNAIN WVRQAPGKGLEWVG YIAV-SGNTYYASWAKG
GFSLSSNAIN WVRQAPGKGLEWVG YIAV-SGNTYYASWAKG

FR3
RFTISKAS--TTVDLKMTSPTAEDTG
RFTISRDNSKNTLYLQMNSLRAEDTA
RFTISRDNSKNTLYLQMNSLRAEDTA
RFTISRDNSKNTLYLQMNSLRAEDTA

RFTISRDS--TTLYLQMNSLRAEDTA
RFTISRDNSKNTLYLQMNSLRAEDTA
RFTISRDNSKNTLYLQMNSLRAEDTA
RFTISRDNSKNTLYLQMNSLRAEDTA
RVTMTTDTSTSTAYMELRSLRSDDTA
RVTMTKAS--STAYMELRSLRSDDTA
RFTISRDNSKNTLYLQMNSLRAEDTA
RFTISRDNSKNTLYLQMNSLRAEDTA
RFTISRDNSKNTLYLQMNSLRAEDTA
RFTISRDNSKNTLYLQMNSLRAEDTA
RFTISRDNSKNTLYLQMNSLRAEDTA
RFTISRDNSKNTLYLQMNSLRAEDTA

FR3 HVR-H3 FR4
TYFCGK SX-XX-XXXNI WGPGTLVTVSL (SEQ ID NO: 03)
VYYCGK SX-XX-XXXNI WGQGTLVTVSS (SEQ ID NO: 50)
VYYCGK SX-XX-XXXNI WGPGTLVTVSS (SEQ ID NO: 51)
VYYCGK SX-XX-XXXNI WGPGTLVTVSS (SEQ ID NO: 52)
VYYCGK SX-XX-XXXNI WGPGTLVTVSS (SEQ ID NO: 53)
VYYCAK SX-XX-XXXNI WGQGTLVTVSS (SEQ ID NO: 54)
VYYCGK SX-XX-XXXNI WGQGTLVTVSS (SEQ ID NO: 55)
VYYCGK SX-XX-XXXNI WGPGTLVTVSS (SEQ ID NO: 56)
VYYCGK SX-XX-XXXNI WGPGTLVTVSS (SEQ ID NO: 57)
VYYCGK SX-XX-XXXNI WGPGTLVTVSS (SEQ ID NO: 58)
VYYCGK SX-XX-XXXNI WGQGTLVTVSS (SEQ ID NO: 59)
VYYCGK SX-XX-XXXNI WGQGTLVTVSS (SEQ ID NO: 60)
VYYCGK SX-XX-XXXNI WGPGTLVTVSS (SEQ ID NO: 61)
VYYCGK SX-XX-XXXNI WGPGTLVTVSS (SEQ ID NO: 62)
VYYCGK SX-XX-XXXNI WGPGTLVTVSS (SEQ ID NO: 63)
VYYCGK SX-XX-XXXNI WGPGTLVTVSS (SEQ ID NO: 64)

### Rb86 VL humanization variants (the original rabbit sequence is shown on top):

FR1
Rb86 AQVLTQTTSPVSAAVGSTVTISC
001--IMGT_hVK_1_5 DIQMTQSTSTLSASVGDRVTITC
002--IMGT_hVK_4_1 AQVMTQSPDSLAVSLGERATINC
003--IMGT_hVK_4_1 AQVMTQSPDSLAVSLGERATINC
004--IMGT_hVK_4_1 DIVMTQSPDSLAVSLGERATINC
005--IMGT_hVK_4_1 DIVMTQSPDSLAVSLGERATINC
006--IMGT_hVK_7_3 DIVLTQSPASLAVSPGQRATITC
007--IMGT_hVK_2_24 DIVMTQTPLSSPVTLGQPASISC
008--IMGT_hVK_1_17 DIQMTQSPSSLSASVGDRVTITC
009--IMGT_hVK_1_5 DIQMTQSPSTLSASVGDRVTITC
010--IMGT_hVK_1_17 DIQMTQSPSSLSASVGDRVTITC

011--IMGT_hVK_1_17 DIQMTQSPSSLSASVGDRVTITC
012--IMGT_hVK_1_17 DIQMTQSPSSLSASVGDRVTITC
013--IMGT_hVK_1_17 DIQMTQSTSSLSASVGDRVTITC
014--IMGT_hVK_1_17 DIQMTQSPSSLSASVGDRVTITC
015--IMGT_hVK_1_17 DIQMTQSPSSLSASVGDRVTITC
016--IMGT_hVK_1_17 DIQMTQSPSSLSASVGDRVTITC
017--IMGT_hVK_1_17 DIQMTQSPSSLSASVGDRVTITC

HVR-L1 FR2 HVR-L2
QSSQSVRT---NKLA WFQQKPGQPPKRLIY SASTLDF
QSSQSVRT---NKLA WFQQKPGKAPKRLIY SASTLDF
KSSQSVRT---NKLA WFQQKPGQPPKRLIY SASTLDS
KSSQSVRT---NKLA WFQQKPGQPPKRLIY SASTLDS
KSSQSVRT---NKLA WFQQKPGQPPKRLIY SASTLDS
KSSQSVRT---NKLA WFQQKPGQPPKRLIY SASTLDS
QSSQSVRT---NKLA WFQQKPGQPPKRLIY SASTLDF
RSSQSVRT---NKLA WLQQRPGQPPRRLIY SASTLDF
RASQSVRT---NKLG WYQQKPGKAPKRLIY SASTLDF
RASQSVRT---NKLA WYQQKPGKAPKRLIY SASTLES
RASQGVRT---NKLG WYQQKPGKAPKRLIY SASTLQS
RASQGVRT---NKLA WFQQKPGKAPKRLIY SASTLQS
RASQGVRT---NKLA WFQQKPGQPPKRLIY SASTLQS
RASQGVRT---NKLA WFQQKPGQPPKRLIY SASTLQS
RASQGVRT---NKLA WFQQKPGQPPKRLIY SASTLQS
RASQGVRT---NKLA WFQQKPGQPPKRLIY SASTLQS
RASQGVRT---NKLA WFQQKPGQPPKRLIY SASTLQS
RSSQSVRT---NKLA WFQQKPGQPPKRLIY SASTLDF

FR3
GVPSRFSASGSGTQFTLTISDVQCDDAATYYC
GVPSRFSGSGSGTEFTLTISSLQPDDFATYYC
GVPDRFSGSGSGTDFTLTISSLQAEDVAVYYC
GVPDRFSGSGSGTDFTLTISSLQAEDVAVYYC
GVPDRFSGSGSGTDFTLTISSLQAEDVAVYYC
GVPDRFSGSGSGTDFTLTISSLQAEDVAVYYC
GVPARFSGSGSGTDFTLTINPVEANDTANYYC
GVPDRFSGSGAGTDFTLKISRVEAEDVGVYYC
GVPSRFSGSGSGTEFTLTISSLQPEDFATYYC
GVPSRFSGSGSGTEFTLTISSLQPDDFATYYC
GVPSRFSGSGSGTEFTLTISSLQPEDFATYYC
GVPSRFSGSGSGTEFTLTISSLQPEDFATYYC
GVPSRFSGSGSGTEFTLTISSLQPEDFATYYC
GVPSRFSGSGSGTEFTLTISSLQPEDFATYYC
GVPSRFSGSGSGTEFTLTISSLQSEDFATYYC
GVPSRFSGSGSGTEFTLTISSLQPEDFATYYC
GVPSRFSGSGSGTEFTLTISSLQPEDFATYYC
GVPSRFSGSGSGTEFTLTISSLQPEDFATYYC

HVR-L3 FR4
LGYFDCSIADCVA FGGGTEVVVK (SEQ ID NO: 06)
LGYFDCSIADCVA FGGGTKVEIK (SEQ ID NO: 65)
LGYFDCSIADCVA FGGGTKVEIK (SEQ ID NO: 66)
LGYFDCSIADCVA FGGGTEVVVK (SEQ ID NO: 67)
LGYFDCSIADCVA FGGGTKVEIK (SEQ ID NO: 68)
LGYFDSSIADSVA FGGGTKVEIK (SEQ ID NO: 69)
LGYFDCSIADCVA FGGGTKVEIK (SEQ ID NO: 70)
LGYFDCSIADCVA FGGGTKVEIK (SEQ ID NO: 71)
LGYFDCSIADCVA FGGGTKVEIK (SEQ ID NO: 72)
LGYFDCSIADCVA FGGGTKVEIK (SEQ ID NO: 73)
LGYFDCSIADCVA FGGGTKVEIK (SEQ ID NO: 74)
LGYFDCSIADCVA FGGGTKVEIK (SEQ ID NO: 75)
LGYFDCSIADCVA FGGGTKVEIK (SEQ ID NO: 76)
LGYFDCSIADCVA FGGGTKVEIK (SEQ ID NO: 77)
LGYFDCSIADCVA FGGGTKVEIK (SEQ ID NO: 78)
LGYFDSSIADSVA FGGGTKVEIK (SEQ ID NO: 79)
LGYFDSSIADRVA FGGGTKVEIK (SEQ ID NO: 80)
LGYFDCSIADCVA FGGGTKVEIK (SEQ ID NO: 81)

The sequence variants have been designed using the general grafting principle. Grafting, in general, was developed to produce humanized antibodies. In addition grafting can also be used to obtain antibodies compatible to other species, or simply in order to exchange the framework of one antibody in order to get other biophysical properties for this antibody or antibody fragment.

After cloning of the humanized variable regions on the human constant region counterpart, the antibodies are expressed in a "matrix" by combining all heavy chain plasmids with all light chain plasmids. The first row and the first column are then half-humanized antibodies, whereas the first cell is the original murine or rabbit antibody in its chimeric form, and the rest of the matrix are the fully humanized antibodies.

For the anti-CD81 antibodies CD81K04 and CD81K13, the binding data are biochemical (cellular binding) ELISA data as summarized in Table 14 and Table 15 below, respectively.

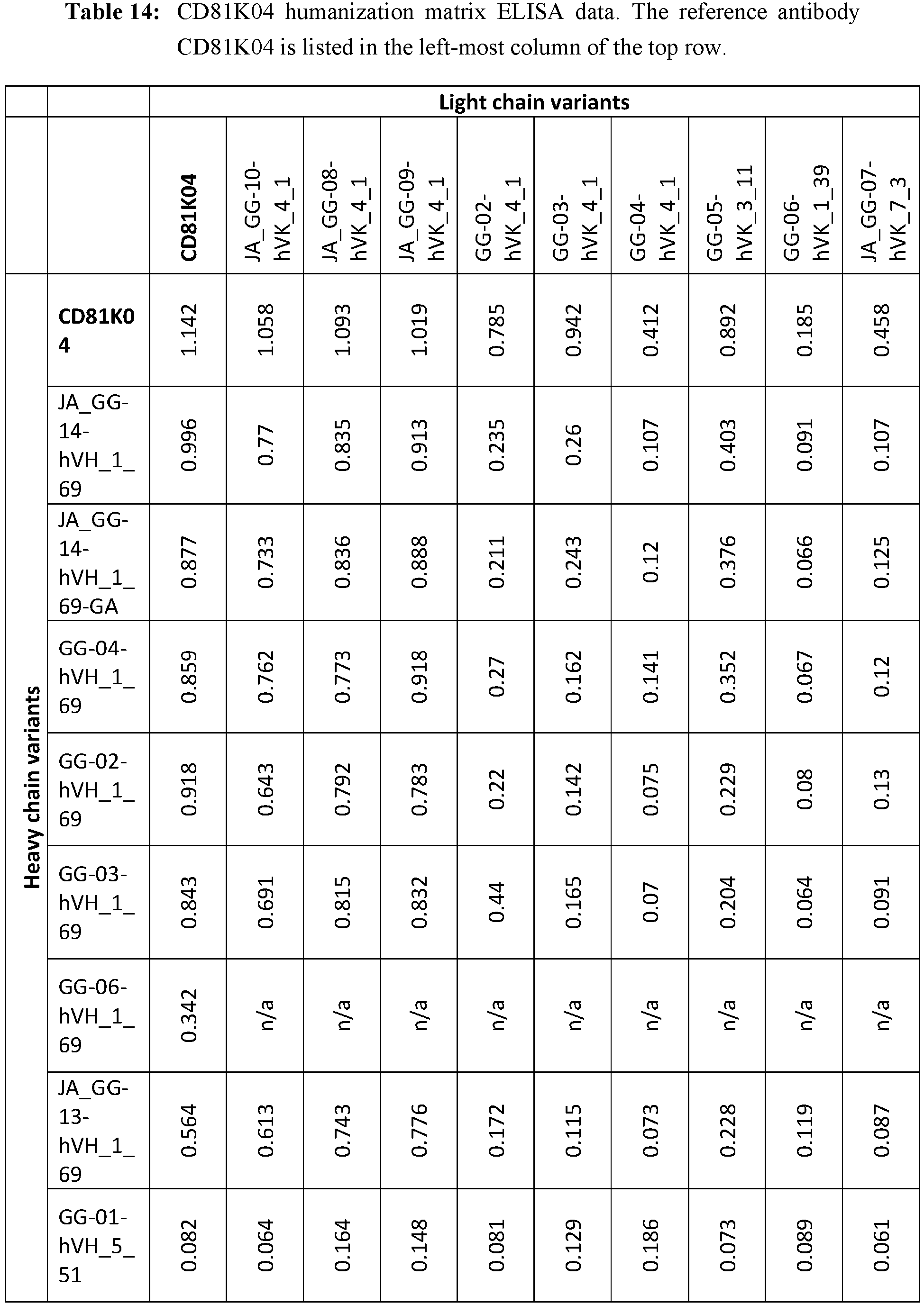

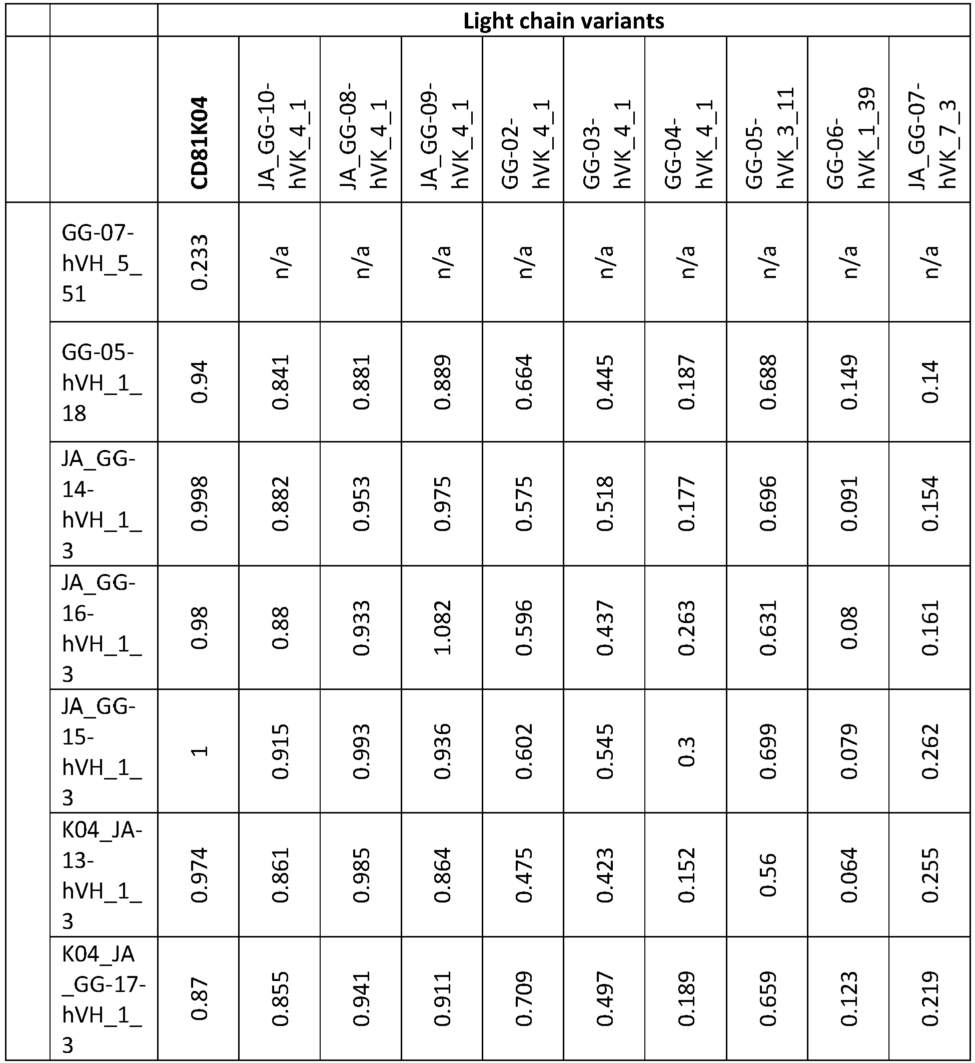

The chimeric form of CD81K04 is close to the value of 1.15, and the humanized variants are slightly less effective binders. For some of the variants, the affinity drops more drastically.

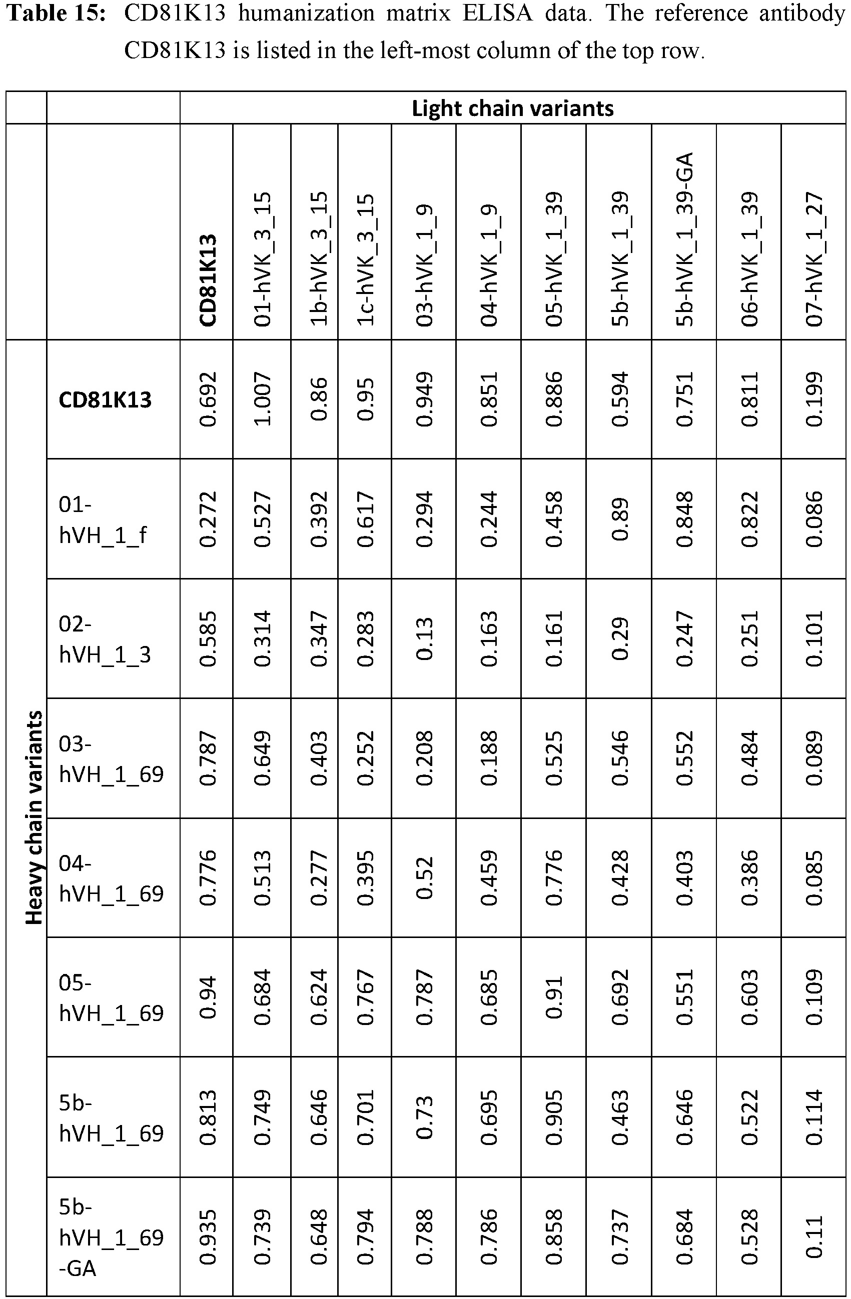

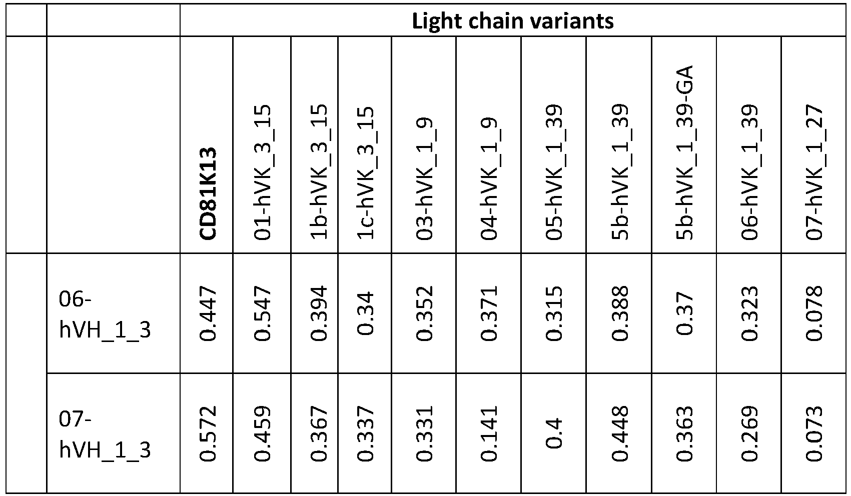

For the rabbit antibody Rb86 micro-purified material from the supernatants were analyzed in the first screening for their ability to have association and dissociation parameters that do not deviate too much from the ones of the original antibody. The binding late (BL) RU (response unit in SPR/BIAcore experiments at the end of the association phase) are compiled for each variant and for the reference rabbit antibody, as well as the dissociation constant kd [1/s] which can be translated in half-life of the antibody on its target (t1/2=ln2/kd), see Table 16 and Table 17, respectively. For some variants that associated very poorly (RU in association phase close to zero or negative), there is also no way to determine a kd value; the half-life value is set to 0.

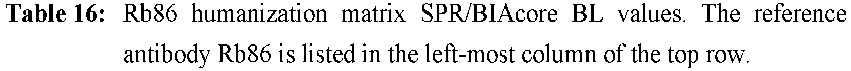

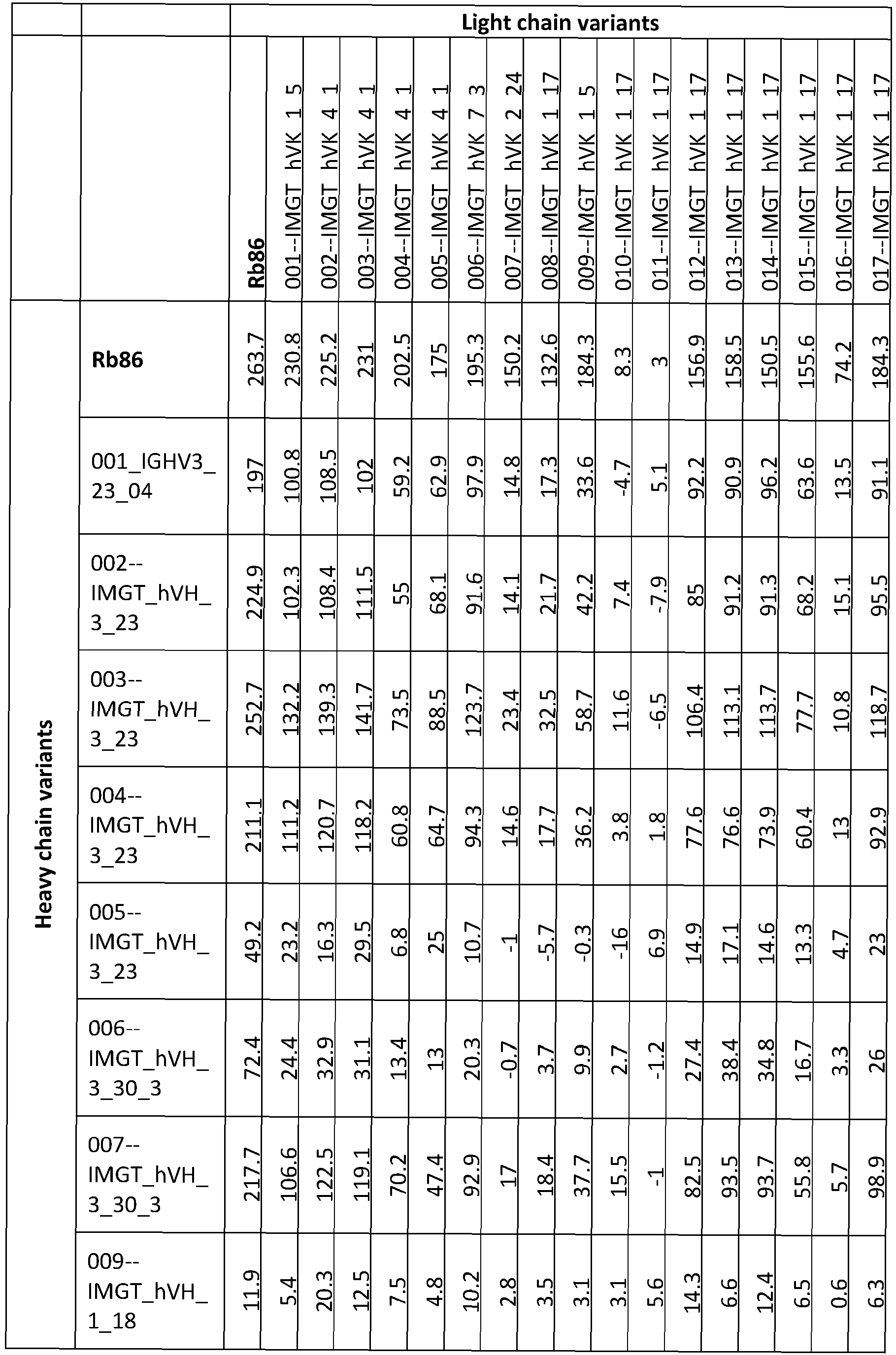

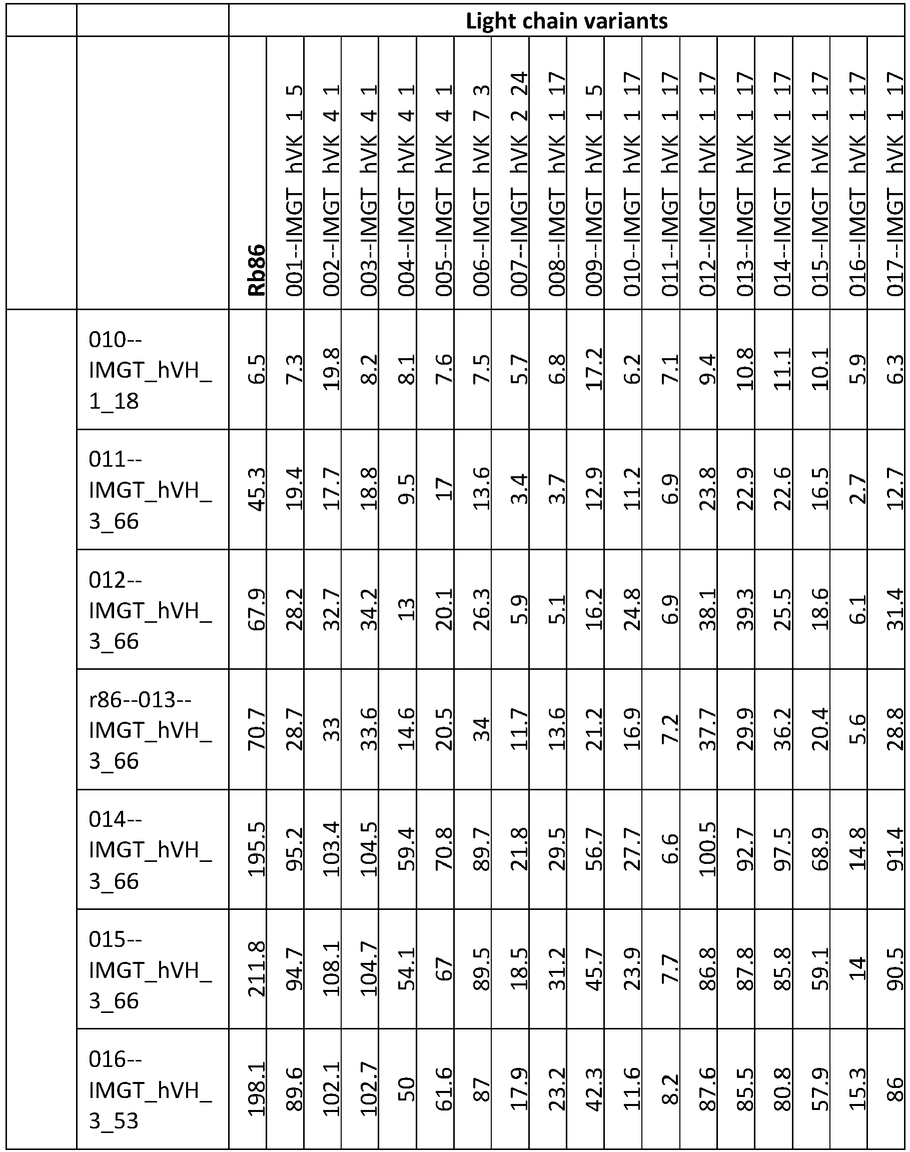

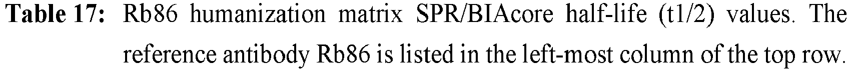

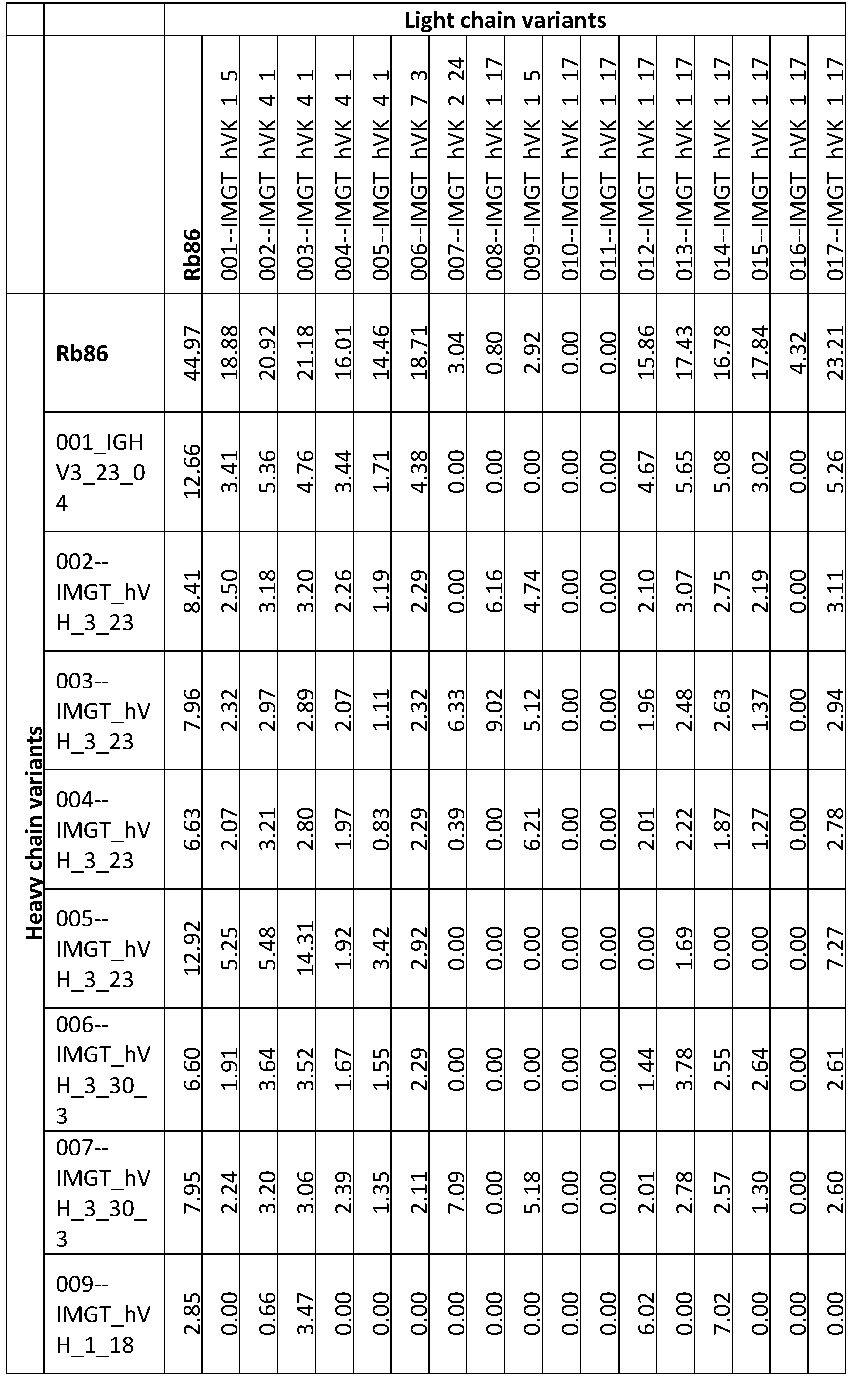

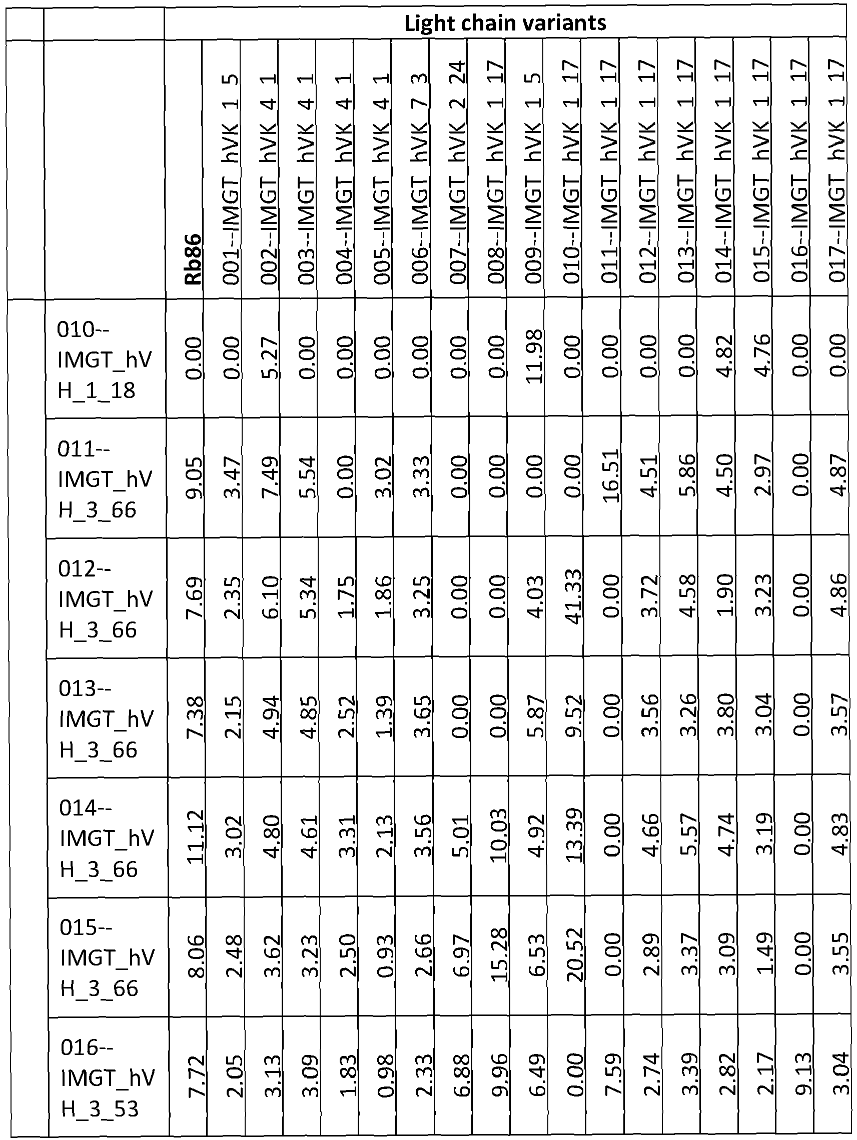

The predicted ABangle distances of the humanization variants with regard to the reference antibody are listed in Table 19 (CD81K04), Table 20 (CD81K13) and Table 21 (Rb86), consistent with the ordering of the ELISA and SPR/BIAcore data stated above (Table 14 to 17).

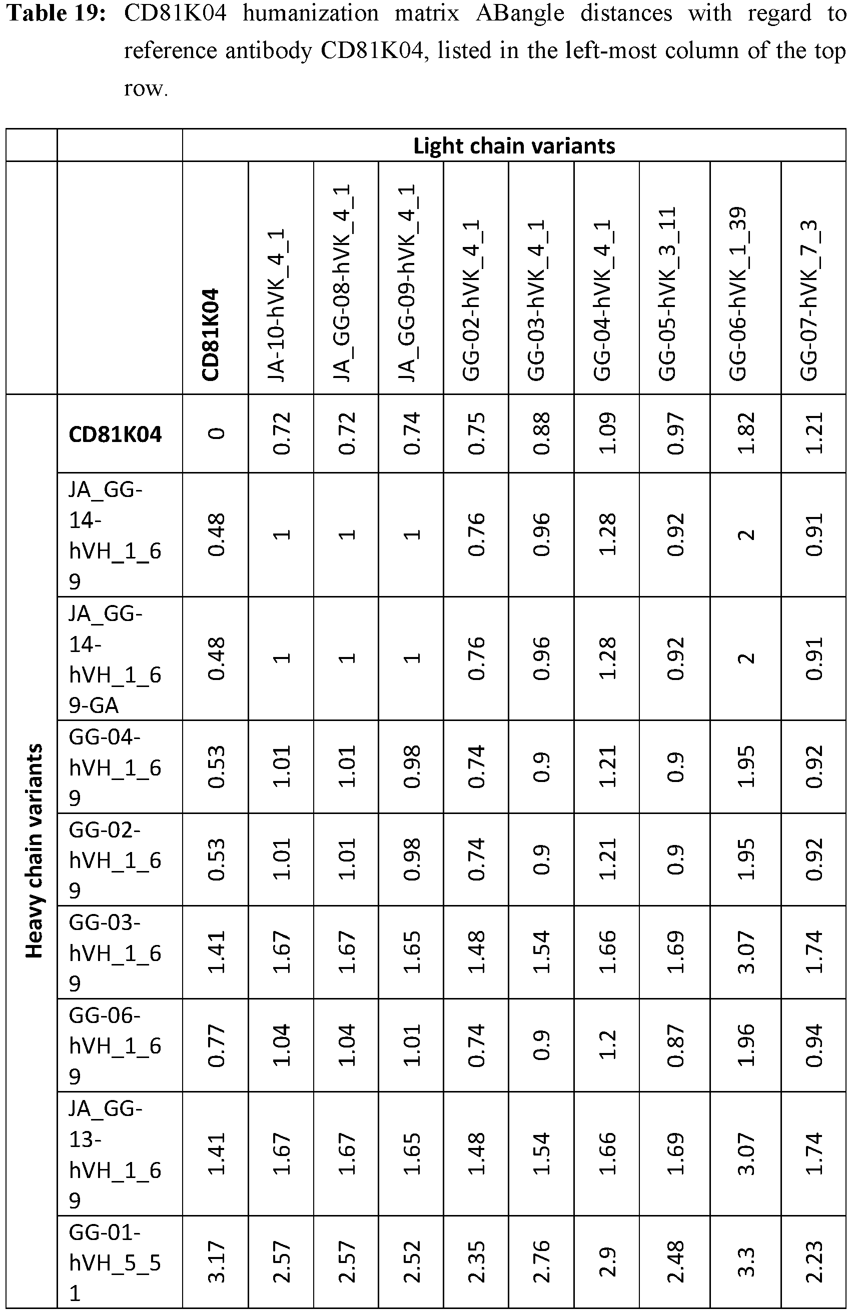

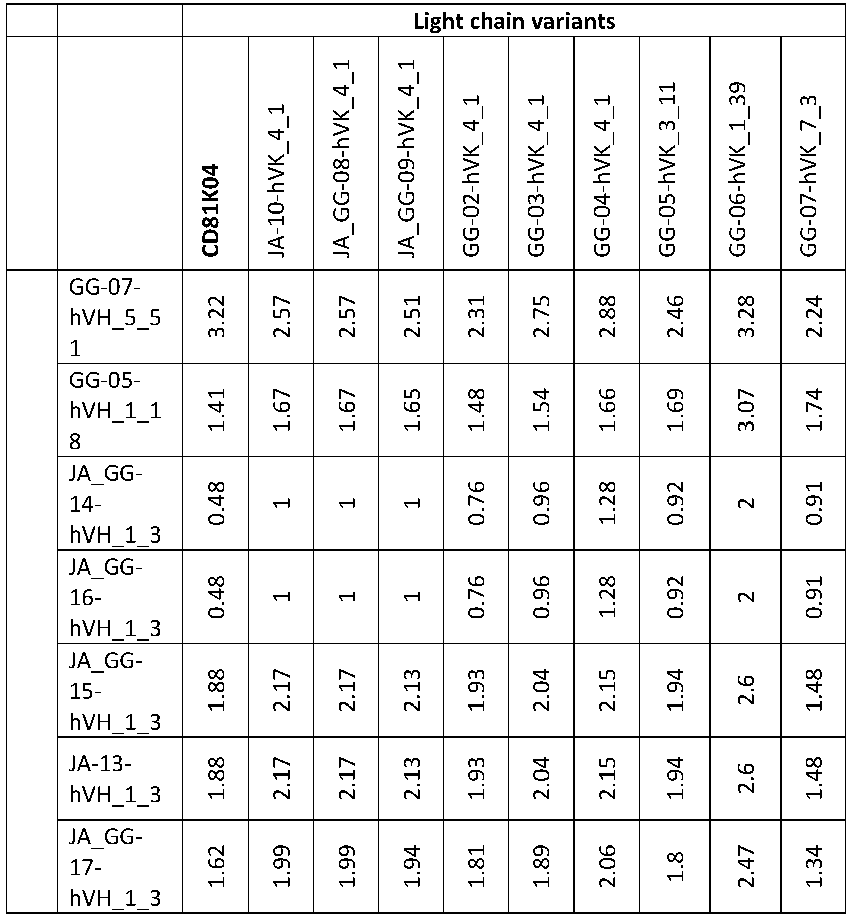

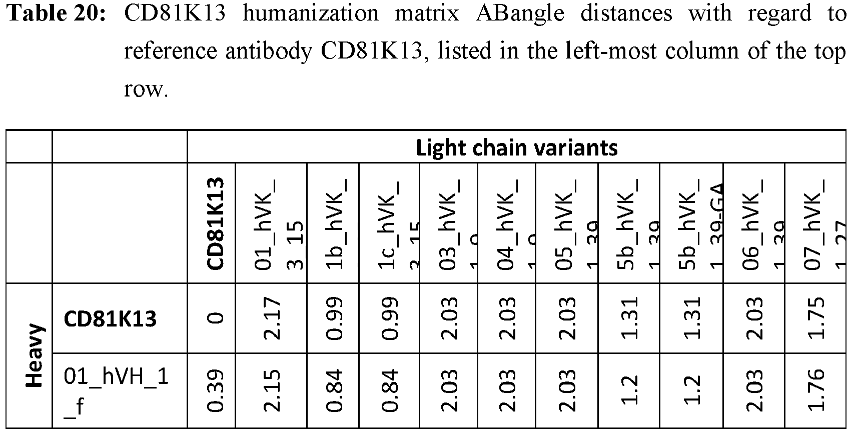

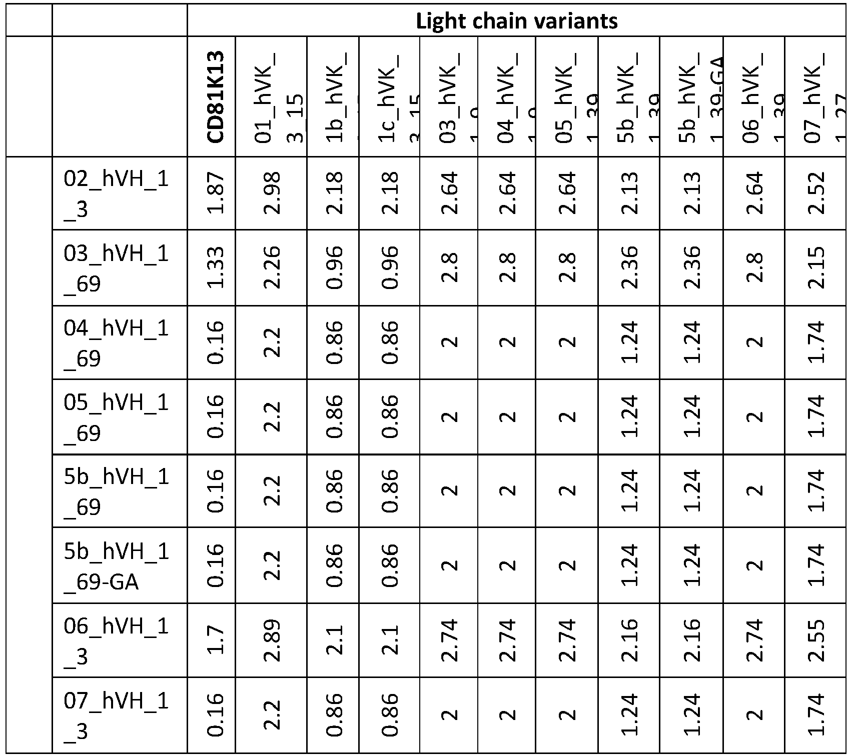

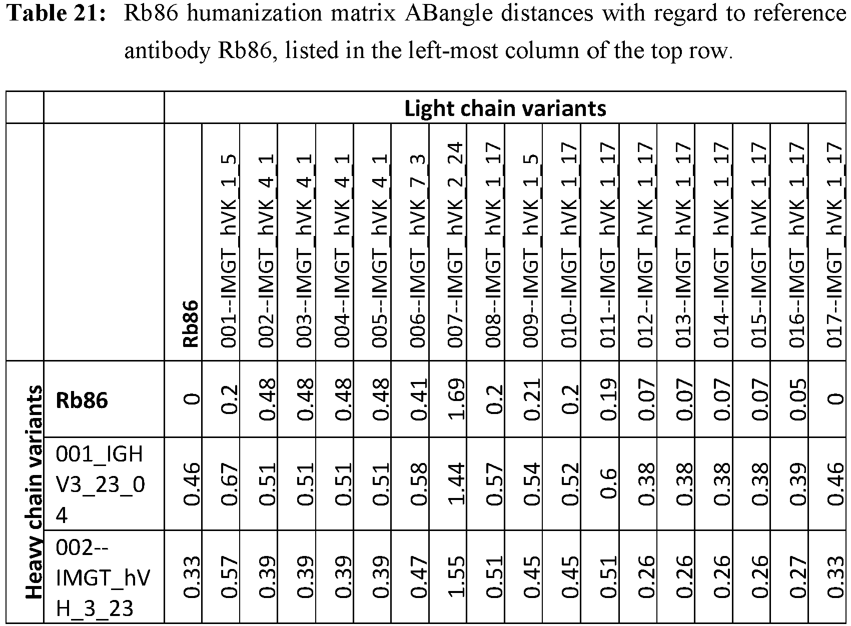

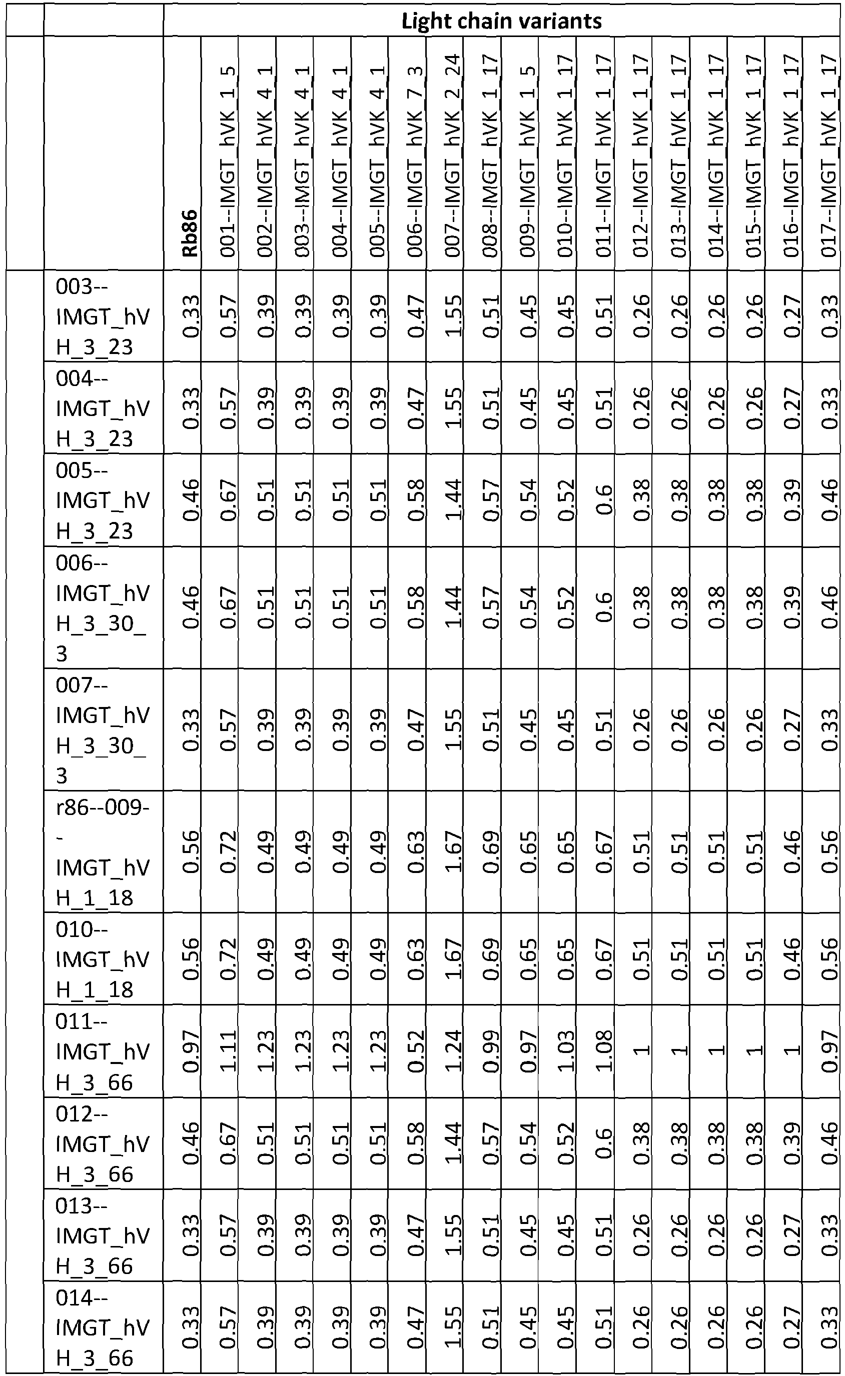

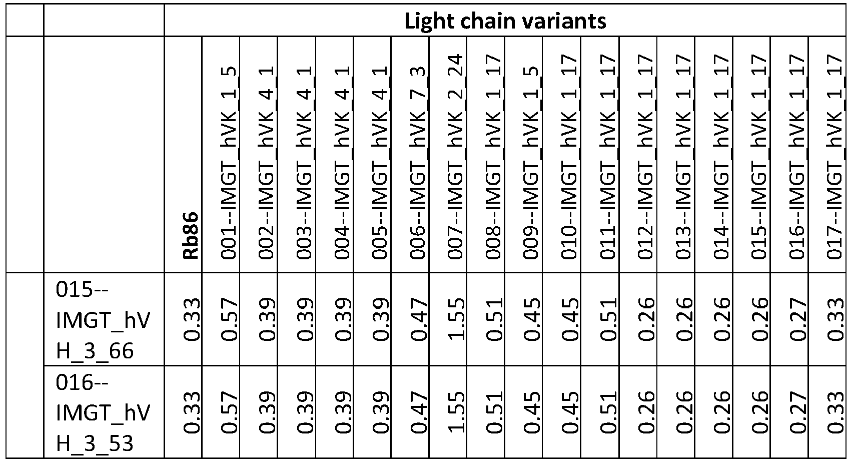

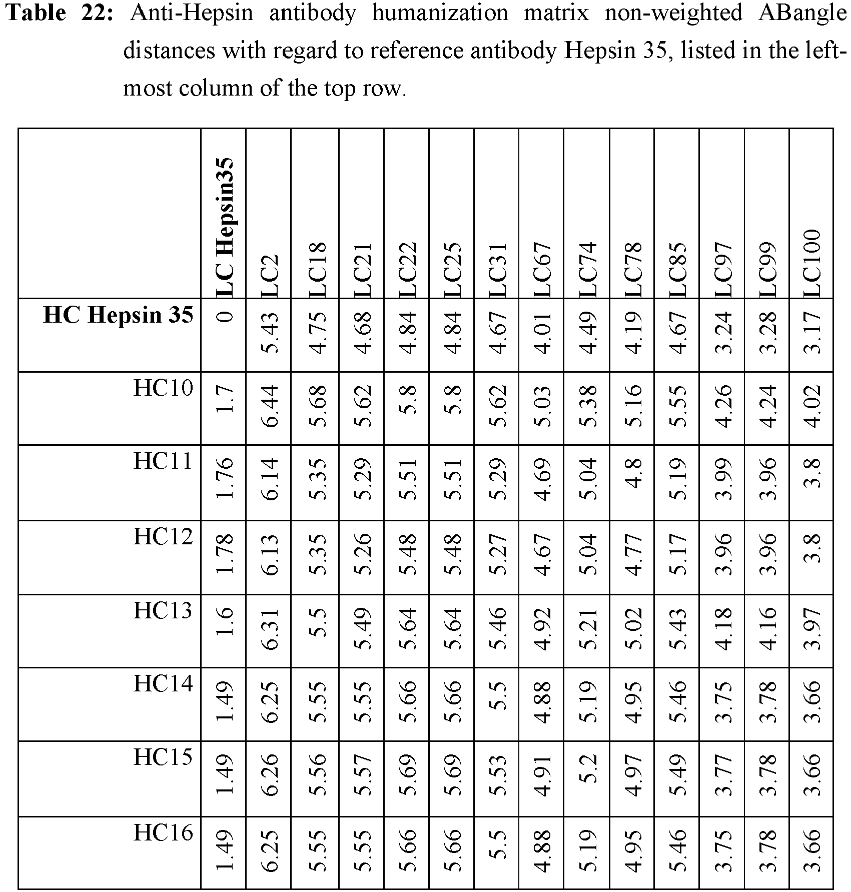

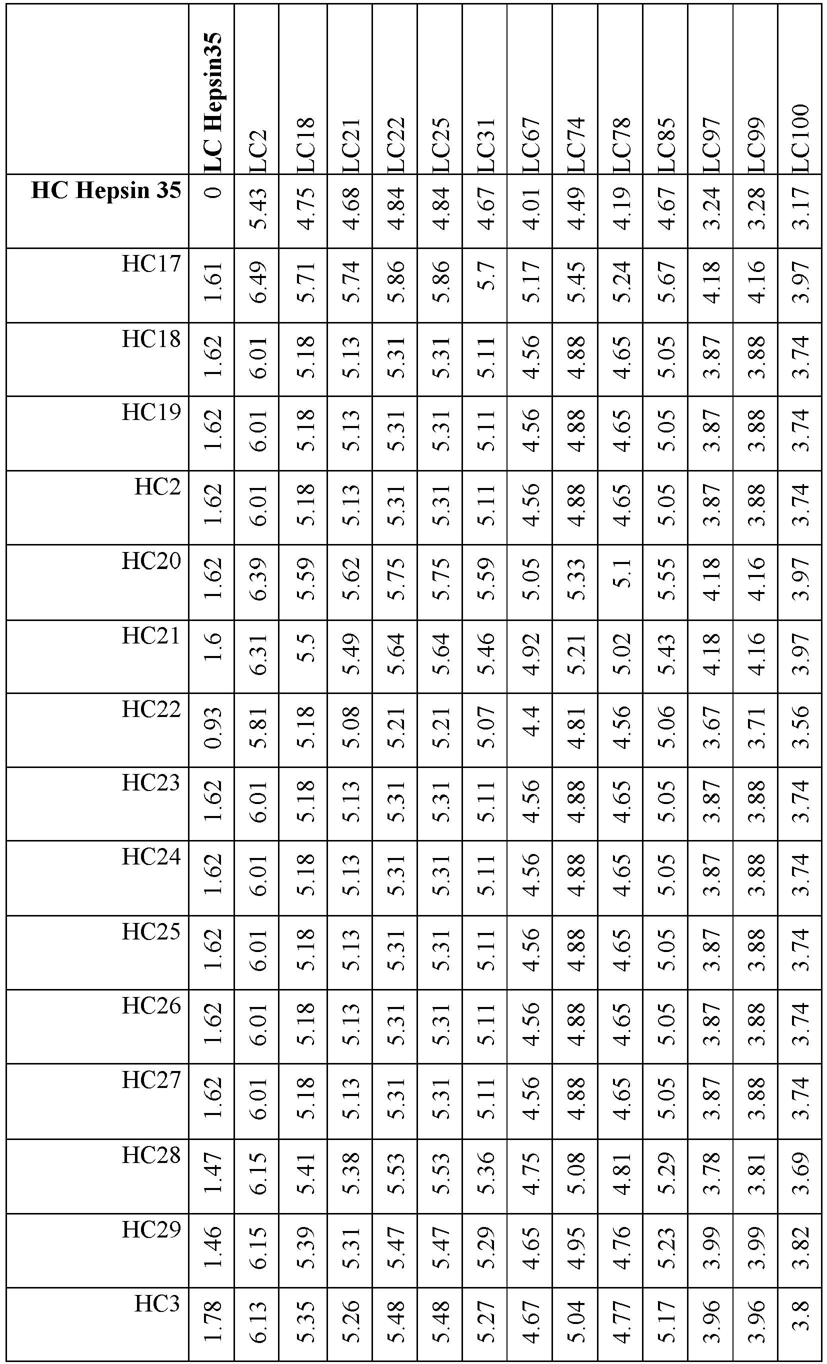

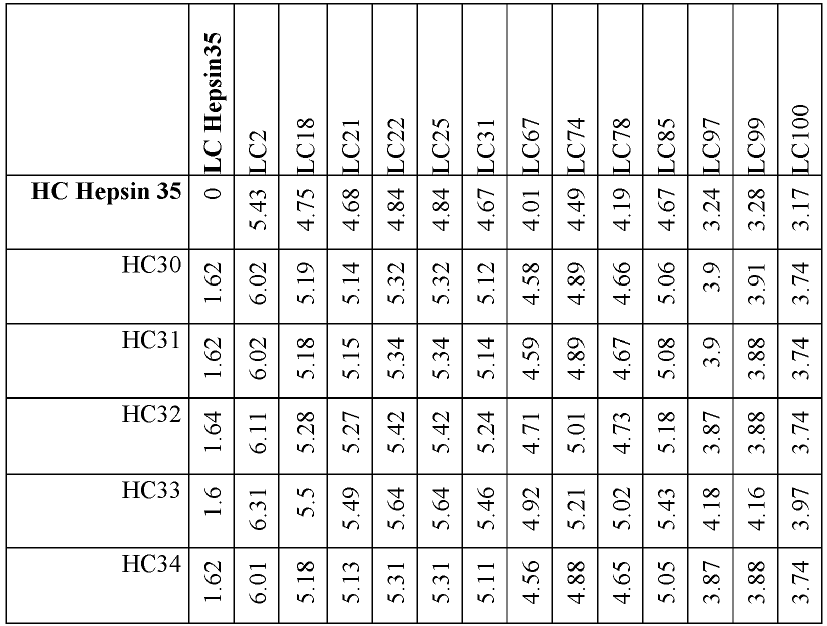

### Correlation between ABangle distance and binding

The matrices can be correlated using the RV coefficient or other coefficients such as the correlation coefficient from the PROTEST method. These methods essentially evaluate the correlation between two data sets, where we have not one but several measurements for each sample and are therefore, to some degree, extensions of the standard univariate correlation coefficient. The RV coefficient is used in the following. In Table 23 the RV coefficient and its p-values for the three different data sets from the perspective of the HCs and the LCs is shown.

**Table 23: RV coefficients and corresponding p-values for all four data sets. The RV coefficient is calculated from the perspective of HCs as samples and hence the LCs as multivariate measurements and vice versa. The p-values are calculated via a permutation test and indicate the probability of reaching a RV coefficient as high as or higher than the one calculated.**

| | **RV HC** | **p-value HC** | **RV LC** | **p-value LC** |
|---|---|---|---|---|
| CD81K04 | 0.2713155 | 0.05665427 | 0.3385992 | 0.03257287 |
| CD81K13 | 0.4597497 | 0.03238687 | 0.1094843 | 0.627880 |
| Rb86human BL | 0.4305386 | 0.01185543 | 0.09112101 | 0.2462529 |
| Rb81human t1/2 | 0.2072768 | 0.1809696 | 0.1127859 | 0.339187 |

A less restricted view on the data set would be to view each mAb as an individual. In that case it makes sense to vectorize both matrices and calculate the Pearson correlation. Table 24 shows the correlation coefficient and the according p-value for the different data sets.

**Table 24: Pearson correlation coefficient for all three data sets and corresponding p-value. The correlation is calculated on vectorized versions of the angle-distance and binding matrices. The p-value indicates the probability to reach the calculated correlation under the null hypothesis of having no correlation.**

| | **Pearson correlation coefficient** | **p-value** |
|---|---|---|
| CD81K04 | -0.3827539 | 3.054e-06 |
| CD81K13 | -0.3351066 | 0.0003455 |
| Rb86human BL | -0.3670692 | 1.292e-10 |
| Rb81human t1/2 | -0.1512745 | 0.01014 |

All data sets show a correlation. Thus, it has been found that methods which reject individual antibodies solely based on the angle-distance can be used to select humanized antibody variants.

### Antibody subset rejection

Three methods for the selection of antibody subsets are analyzed in the following as to their performance. These methods are the selection of the worst 20%, the selection of bad HC/LC combinations and the selection of whole HCs or LCs as described herein.

In order to evaluate the performance of the different selection methods different statistics were calculated. The first one is the ratio of median binding length between the kept and rejected subsets. For this ratio also a p-value was calculated using a permutation test. Furthermore the distribution of binding lengths or for one data set ICso values in the two subsets using stacked histograms was inspected visually.

### CD81K04

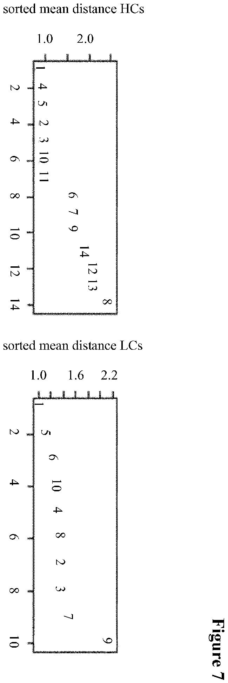

For both methods that use HC/LC information, the HCs/LCs that are expected to perform worse than the rest have to be chosen. Figure 7 depicts the average angle distance for the HCs (rows of the matrix, left) and the LCs (columns of the matrix, right). Antibodies comprising the HCs 6, 7, 8, 9, 12, 13 and 14, and LCs 7 and 9 were deselected.

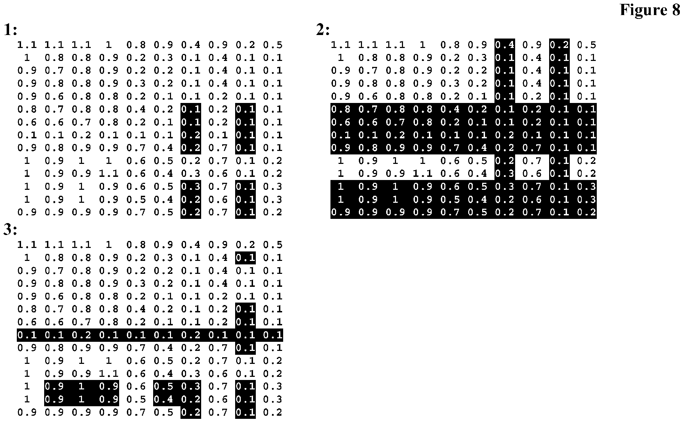

In Figure 8 it is shown which antibodies are removed by the respective selection method (shaded) and which are kept.

Table 25 shows the results of the comparison of the subsets of antibodies as to their binding length. The median binding length in both sets was calculated and the ratio of both was formed. In the table the results for all three methods together with the p-value, which indicates the probability of getting a ratio at least this low, are shown.

**Table 25: For the three different antibody rejection methods, the ratio of the median ELISA measurement between the rejected and kept antibodies was calculated. Additionally a p-value was calculated via a permutation method, which shows that probability of reaching a value as low or lower as the found median ratio.**

| **CD81K04** | **median ratio (deselected/selected)** | **p-value** |
|---|---|---|
| reject worst 20% | 0.2279 | 0.0373 |
| reject bad HC/LC combinations | 0.20661 | 0.0162 |
| reject whole HCs and LCs | 0.3152 | <0.001 |

Independent of the method the subset of antibodies kept has 3-5 times longer binding length than the deselected antibodies. Furthermore, these results are significant (p < 0.05).

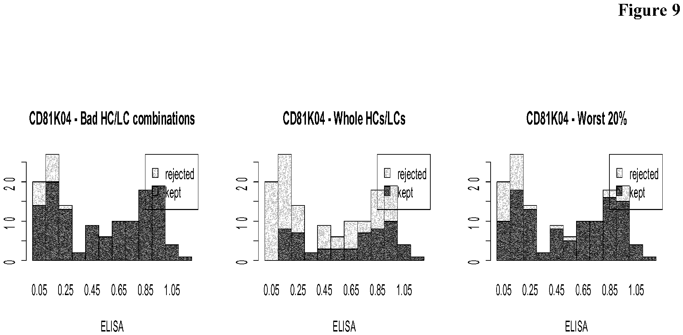

In Figure 9 stacked histograms of the ELISA measurements for the three selection methods are shown. The histograms confirm the median ratio results. All three methods reject low-binding monoclonal antibodies.

### CD81K13

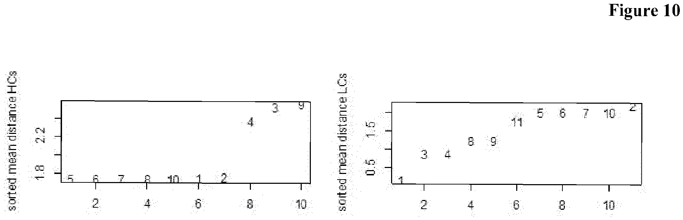

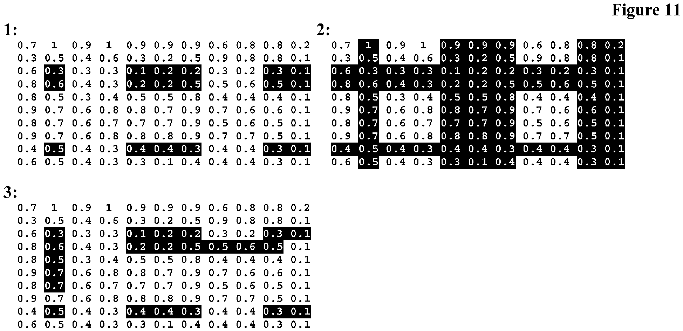

The same approach as outlined above was performed for the humanized variants of antibody CD81K13. The results are shown in Figures 10 to 12 and the following Table 26.

Antibodies comprising the HCs 3, 4 and 9, and LCs 2, 5, 6, 7, 10 and 11 were deselected.

All three methods lead to a subset binding 1.6 - 2 times longer than the antibodies in the deselected subset (see Table 26).

**Table 26: For the three different antibody selection methods the ratio of the median ELISA measurement between the rejected and kept antibodies was calculated. Additionally a p-value was calculated via a permutation method, which shows that probability of reaching a value as low or lower as the found median ratio.**

| **CD81K13** | **median ratio (deselected/selected)** | **p-value** |
|---|---|---|
| reject worst 20% | 0.6255 | 0.0042 |
| reject bad HC/LC combinations | 0.51223 | 0.0062 |
| reject whole HCs and LCs | 0.6238 | 0.0313 |

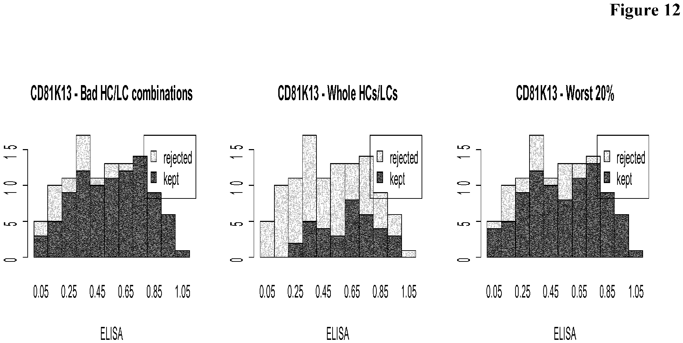

Figure 12 shows that predominantly antibodies with lower binding length are rejected.

### Rb86

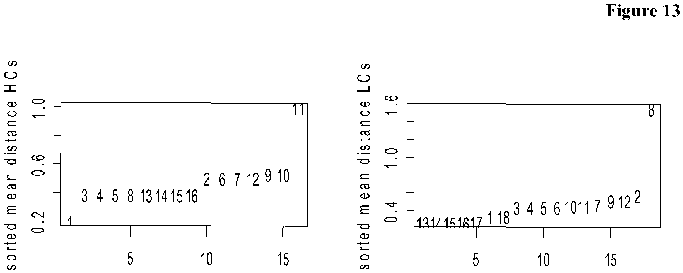

The same approach as outlined above was performed for the humanized variants of antibody Rb86. The results are shown in Figures 13 to 15 and the following Table 27.

Antibodies comprising the HCs 9, 10 and 11, and LCs 2 and 8 were deselected.

For the variants of antibody Rb86 the SPR data is used in the selection/deselection step. Two different measurements regarding the binding behavior of the different antibodies are available.

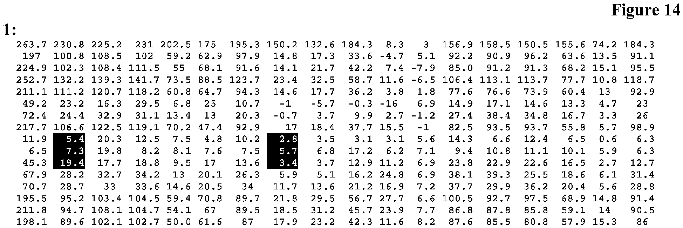

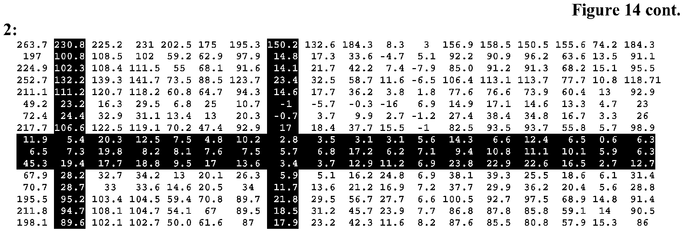

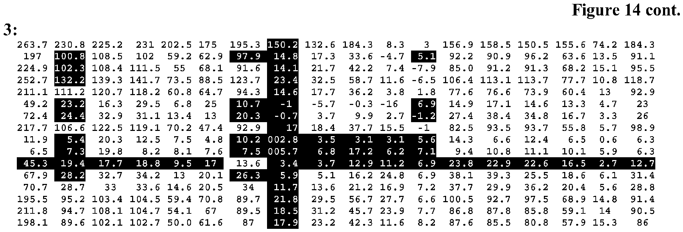

Based on the BL data different antibodies were deselected (see Figure 14).

All three selection methods select antibodies that bind 3-5.5 times better on average (see Table 27) with regard to "BL". The p-value indicates that this result is not by chance, but due to the beneficial way to select antibodies in the different methods.

**Table 27: For the three different antibody rejection methods the ratio of the median BL measurement between the rejected and kept antibodies is calculated. Additionally a p-value is calculated via a permutation method, which shows that probability of reaching a value as low or lower as the found median ratio.**

| **Rb86human BL** | **median ratio (deselected/selected)** | **p-value** |
|---|---|---|
| reject worst 20% | 0.303 | <0.001 |
| reject bad HC/LC combinations | 0.182 | 0.0149 |
| reject whole HCs and LCs | 0.223 | 0.007 |

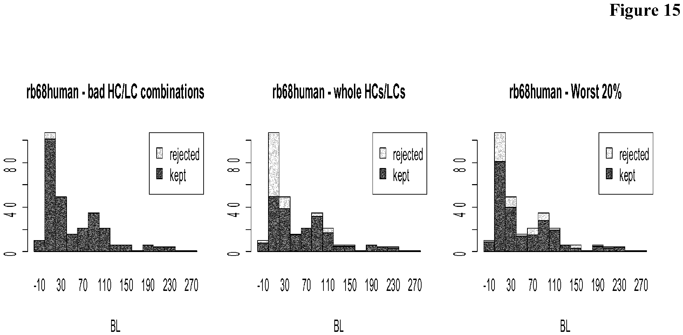

This is underlined in Figure 15 wherein the stacked histograms show that predominantly antibodies with low binding length were deselected.

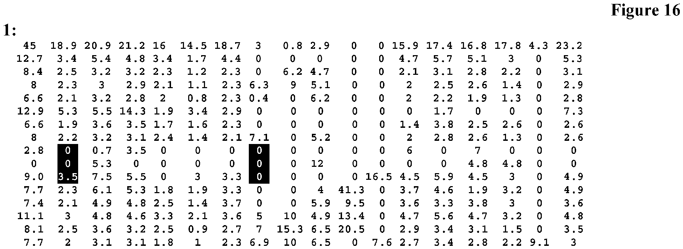

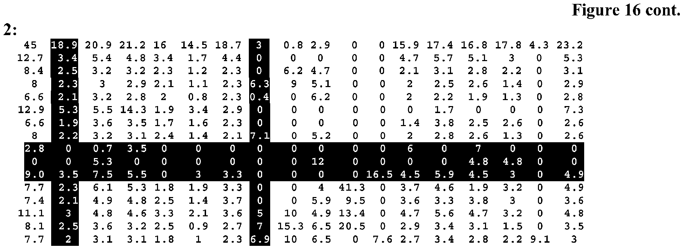

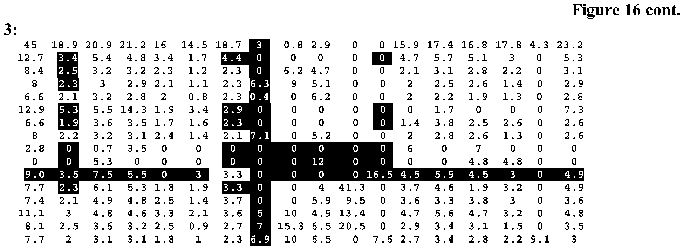

As alternative approach based on the t1/2 data antibodies were deselected (see Figure 16). As can be seen the same antibodies are selected as based on the BL data.

The median ratio for the different selection methods shows that the deselected subset of antibodies is always worse than the kept subset (see Table 28). With the "Bad HC/LC combination"-method only a few antibodies were deselected, but there are a number of antibodies with no half-life in the set. This is the reason why for this method the p-value is not significant. For the other methods the p-values indicate that the deselected subset was chosen well.

**Table 28: For the three different antibody selection methods the ratio of the median t1/2 measurement between the deselected and selected antibodies was calculated. Additionally a p-value was calculated via a permutation method, which shows that probability of reaching a value as low or lower as the found median ratio.**

| **Rb68human t1/2** | **median ratio (deselected/selected)** | **p-value** |
|---|---|---|
| reject worst 20% | 0.0698 | <0.001 |
| reject bad HC/LC combinations | 0 | 0.132 |
| reject whole HCs and LCs | 0.181 | 0.0232 |

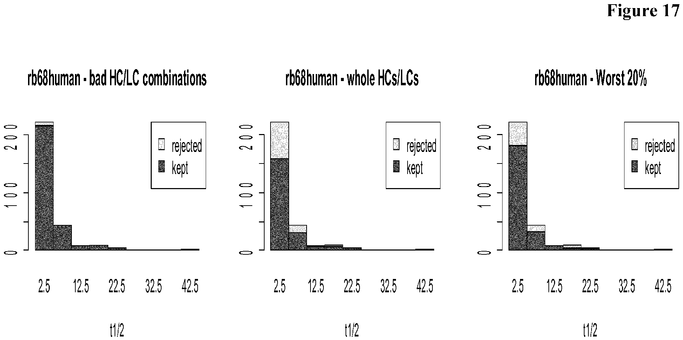

This is also shown in the stacked histograms in Figure 17. All methods deselect a good amount of the many antibodies with very low half-life.

### Summary

It has been found that, when VH-VL-orientation (VH-VL-angle) prediction was employed on humanization variants that are all derived from a common original parent antibody, good humanized variants respect more closely the angle parameters of the parent antibody. The three methods used to reject antibodies with a suboptimal VH-VL-orientation, i.e. reject the "worst 20%", or a set of whole HCs/LCs, or the bad HC/LC combinations, resulted in similar antibody subsets. Stacked histograms and correlation analysis confirmed that angle-distance is a good indicator of the binding behavior. It has been found that by using a selection method as reported herein the quality of such filtered humanization matrix can be increased drastically.

In one embodiment of the invention the confidence matrix is incorporated as an additional step, e.g. first the deselected subset is chosen and then the high confidence subset is chosen.

In one embodiment of the invention the distance information between all antibodies to compute clusters and identify clusters of antibodies that are most far away from the cluster incorporating the reference is used.

The following are examples of methods and compositions of the invention. It is understood that various other embodiments may be practiced, given the general description provided above. The examples are not to be understood to limit the invention. The true scope is set forth in the claims.

### EXAMPLES

### Example 1

### Materials and Methods

### Roche Antibody Database 3D (RAB3D)

The antibody structure database RAB3D contains mostly publicly available Fv structures. The Fv structures are processed and annotated with the in-house "Wolfguy" numbering scheme (see next section). All annotated Fv structures are superimposed on a consensus Fv framework, on a consensus VH framework, and on a consensus VL framework. The consensus structures are calculated using a subset of high-resolution structures from the PDB. The annotated and reoriented Fv structures serve as template repository for homology modeling.

### Wolfguy Numbering Scheme

The Wolfguy numbering defines CDR regions as the set union of the Kabat and Chothia definition. Furthermore, the numbering scheme annotates CDR loop tips based on CDR length (and partly based on sequence) so that the index of a CDR position indicates if a CDR residue is part of the ascending or the descending loop. A comparison with established numbering schemes is shown in the following Table 1.

**Table 1: Numbering of CDR-L3 and CDR-H3 using Chothia/Kabat (Ch-Kb), Honegger and Wolfguy numbering schemes. The latter has increasing numbers from the N-terminal basis to the CDR peak and decreasing ones starting from the C-terminal CDR end. Kabat schemes fix the two last CDR residues and introduce letters to accommodate for the CDR length. In contrast to Kabat nomenclature, the Honegger numbering does not use letters and is common for VH and VL.**

| | | | | |
|---|---|---|---|---|
| 326 | 88 | 102 | 84 | 730 |
| 327 | 89 | 103 | 85 | 731 |
| 328 | 90 | 104 | 86 | 732 |
| 329 | 91 | 105 | 87 | 733 |
| 330 | 92 | C | 88 | 734 |
| 331 | 93 | 107 | 89 | 751 |
| 332 | 94 | 108 | 90 | 752 |
| 351 | 95 | 109 | 91 | 753 |
| 352 | 96 | 110 | 92 | 754 |
| 353 | 97 | 111 | 91 | 755 |
| 354 | 98 | 112 | 94 | 756 |
| 355 | 99 | 113 | 95 | 757 |
| 356 | 100 | 114 | 95a | 758 |
| 357 | 100a | 115 | 95b | 759 |
| 358 | 100b | 116 | 95c | 760 |
| 359 | 100c | 117 | 95d | 761 |
| 360 | 100d | 118 | 95e | 762 |
| 361 | 100e | 119 | 95f | 763 |
| 362 | 100f | 120 | | 764 |
| 363 | 100g | 121 | | 765 |
| 364 | 100h | 122 | | 766 |
| 384 | 100i | 123 | | 784 |
| 385 | 100i | 124 | | 785 |
| 386 | 100k | 125 | | 786 |
| 387 | 1001 | 126 | | 787 |
| 388 | | 127 | | 788 |
| 389 | | 128 | | 789 |
| 390 | | 129 | | 790 |
| 391 | | 130 | | 791 |
| 392 | | 131 | | 792 |
| 393 | | 132 | | 793 |
| 394 | | 133 | | 794 |
| 395 | | 134 | | 795 |
| 396 | | 135 | | 796 |
| 397 | | 136 | | 797 |
| 398 | 101 | 137 | 96 | 798 |
| 399 | 102 | 138 | 97 | 799 |
| 401 | 103 | F W | 98 | 801 |
| 402 | 104 | 140 | 99 | 802 |
| 403 | 105 | 141 | 100 | 803 |
| 404 | 106 | 142 | 101 | 804 |
| **Wolfguv VH** | **Ch-Kb** | **Honegger** | **Ch-Kb** | **Wolfguy VL** |

Wolfguy is designed such that structurally equivalent residues (i.e. residues that are very similar in terms of conserved spatial localization in the Fv structure) are numbered with equivalent indices as far as possible. This is illustrated in Figure 1.

An example for a Wolfguy-numbered full-length VH and VL sequence can be found in the following Table 2.

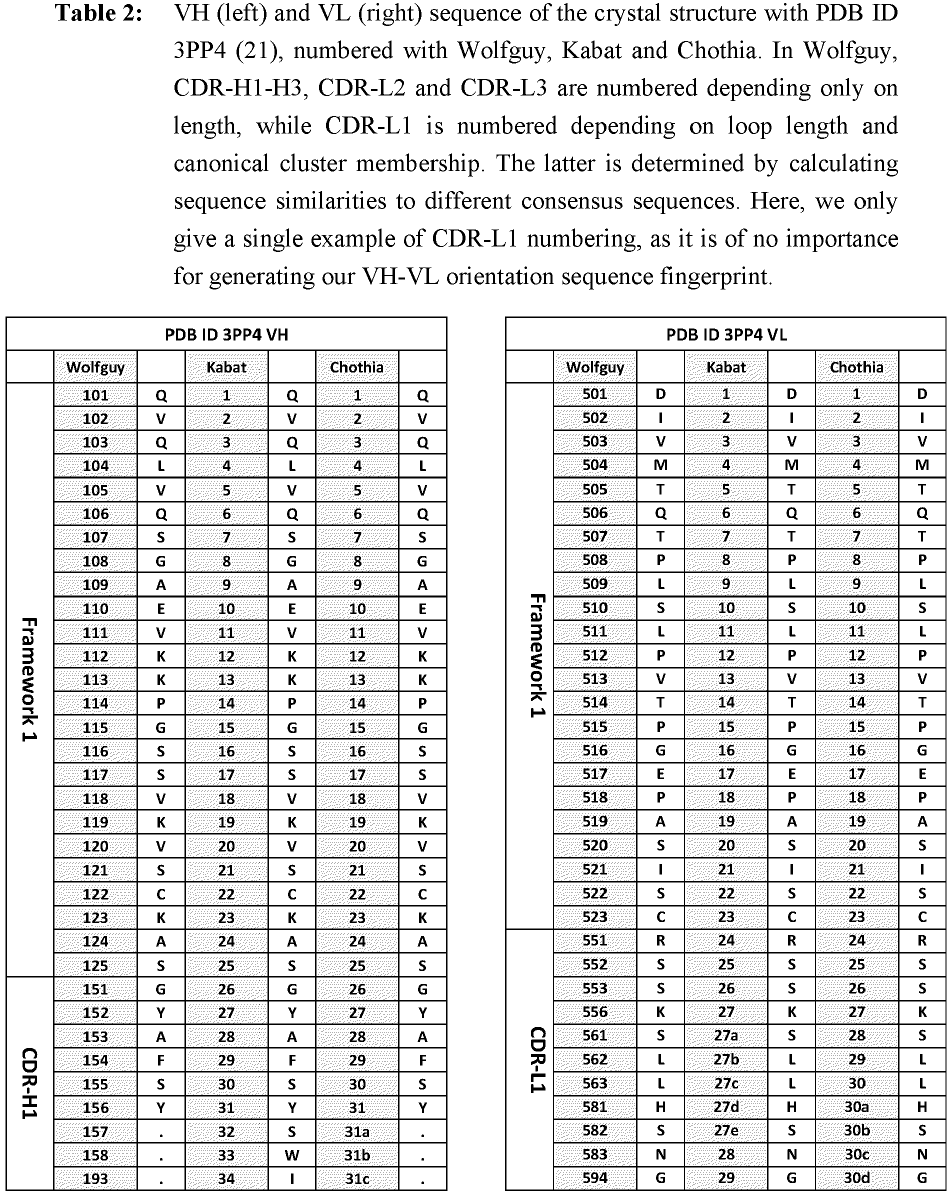

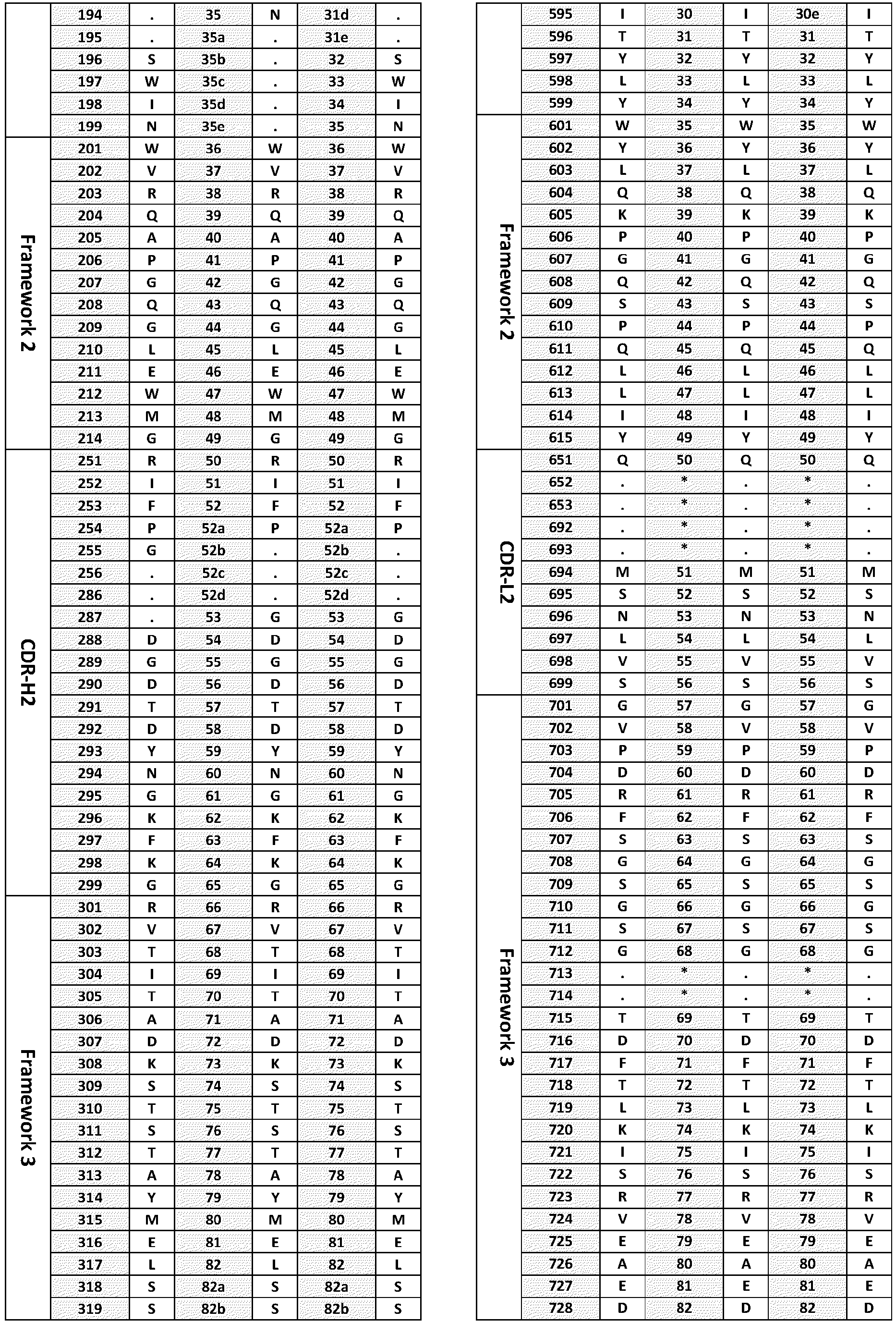

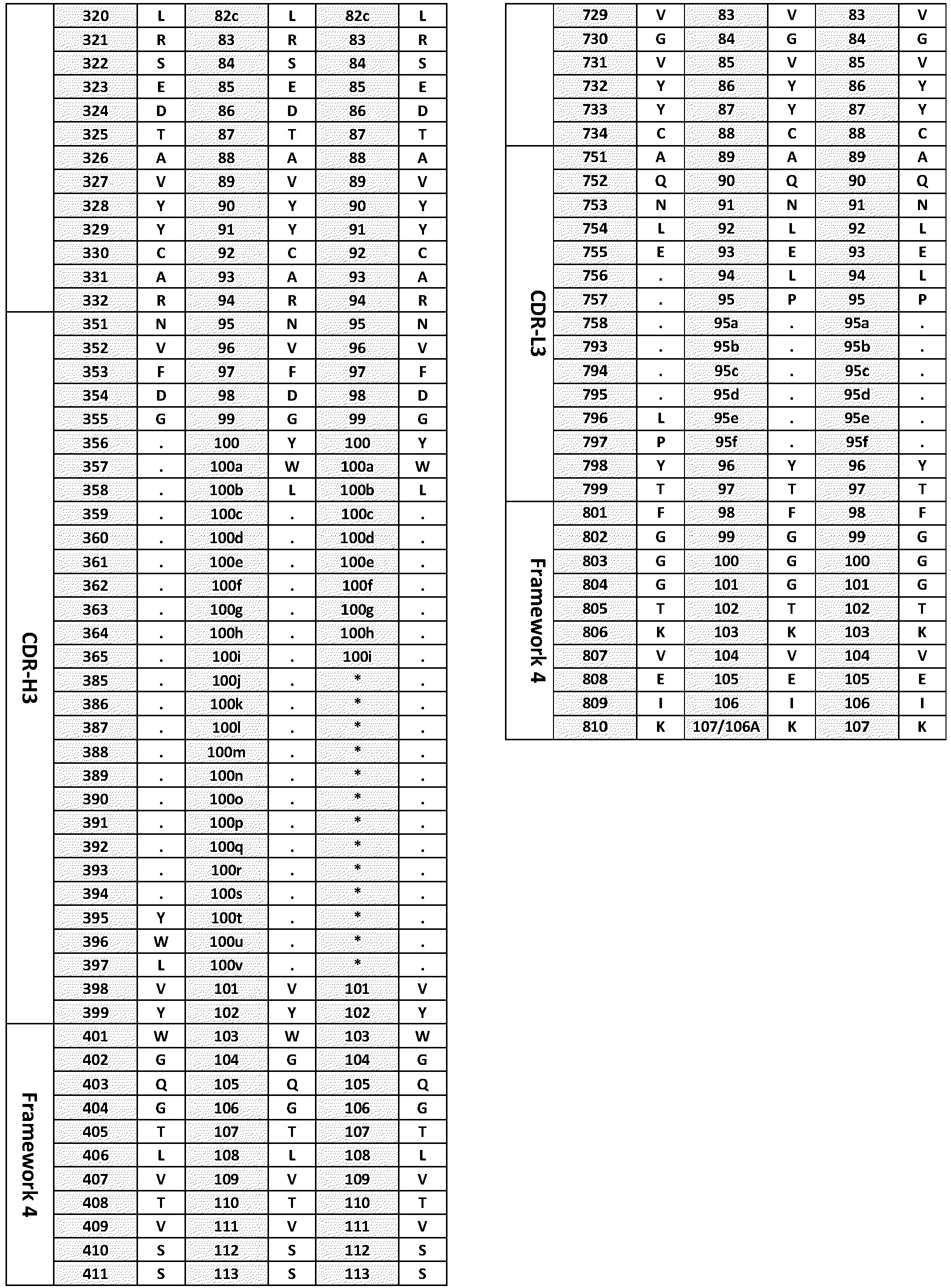

### Example 1

### VH-VL Orientation Fingerprint Selection

The VH-VL orientation is herein predicted from a (meaningful) subset of Fv sequence positions (a "sequence fingerprint") rather than from complete Fv sequences. Based on the assumption that VH-VL orientation is governed by residues on or near the VH-VL interface, a set of interface residues has been identified wherein a residue is defined to be part of the VH-VL interface if its side chain atoms are neighboring atoms of the opposite chain with a distance of less or equal than 4 Å in at least 90 % of all superimposed Fv structures in the database, e.g. in RAB3D. The results are summarized in Table 29, which also states if a sequence position has previously been connected to being a determinant of VH-VL orientation based on statistical analyses (4, 5, 7).

**Table 29: VH-VL interface residues where a residue is part of the interface if its side chain atoms are neighboring atoms of the opposite chain with a distance of less or equal than 4 Å in at least 90 % of all superpositioned Fv structures in RAB3D.**

| **Wolfguy Index** | **Chothia (14) Index** | **Wolfguy Region** | **Dunbar et al. (7)** | **Abhinandan, Martin (4)** | **Chailyan et al. (5)** |
|---|---|---|---|---|---|
| 199 | H35⁺ | CDR-H1 | X | | |
| 202 | H37⁺ | VH-FW2 | | | |
| 204 | H39⁺ | VH-FW2 | | | |
| 210 | H45⁺ | VH-FW2 | | X | |
| 212 | H47⁺ | VH-FW2 | | | |
| 251 | H50 | CDR-H2 | X | | |
| 292* | H58 | CDR-H2 | X | | |
| 294* | H60 | CDR-H2 | | X | |
| 295* | H61 | CDR-H2 | X | | |
| 329 | H91⁺ | VH-FW3 | | X | |
| 351 | H95 | CDR-H3 | | | |
| 352* | H96 | CDR-H3 | | | |
| 354* | H98 | CDR-H3 | | | |
| 395* | H100x-2* | CDR-H3 | | | |
| 396* | H100x-1* | CDR-H3 | | | |
| 397* | H100x* | CDR-H3 | | | |
| 398* | H101 | CDR-H3 | | | |
| 399 | H102 | CDR-H3 | | | |
| 401 | H103⁺ | VH-FW4 | | | |
| 403 | H105⁺ | VH-FW4 | | X | |
| 597* | L32 | CDR-L1 | | | |
| 599 | L34⁺ | CDR-L1 | X | | |
| 602 | L36⁺ | VL-FW2 | X | | X |
| 604 | L38⁺ | VL-FW2 | X | X | |
| 609 | L43⁺ | VL-FW2 | X | | X |
| 610 | L44⁺ | VL-FW2 | X | X | X |
| 612 | L46⁺ | VL-FW2 | X | X | |
| 615 | L49 | VL-FW2 | X | | |
| 651 | L50 | CDR-L2 | X | | |
| 698* | L55⁺ | CDR-L2 | X | | |
| 733 | L87⁺ | VL-FW3 | X | X | |
| 751 | L89 | CDR-L3 | X | | |
| 753* | L91 | CDR-L3 | X | | |
| 796* | L95x-1* | CDR-L3 | X | | |
| 797* | L95x* | CDR-L3 | X | | |
| 798* | L96 | CDR-L3 | X | | |
| 801 | L98⁺ | VL-FW4 | | | |

| | | | | | |
|---|---|---|---|---|---|
| *Numbering depending on loop length +Part of the VH-VL interface as defined by Chothia et al. (13) | | | | | |

The above set of interface residues is missing some of the sequence positions that had been listed among the "top 10 important input variables" for VH-VL orientation by Dunbar et al. (7). Those sequence positions are listed in the following Table 30.

**Table 30: Additional sequence positions listed among the "top 10 important input variables" for VH-VL orientation by Dunbar et al. (7).**

| **Wolfguy Index** | **Chothia (14) Index** | **Wolfguy Region** | **Abhinandan, Martin (4)** | **Chailyan et al. (5)** |
|---|---|---|---|---|
| 197* | H33 | CDR-H1 | X | |
| 208 | H43 | VH-FW2 | | |
| 209 | H44⁺ | VH-FW2 | | |
| 211 | H46 | VH-FW2 | | |
| 296* | H62 | CDR-H2 | X | |
| 327 | H89 | VH-FW3 | | |
| 355* | H99 | CDR-H3 | | |
| 607 | L41 | VL-FW2 | X | X |
| 608 | L42 | VL-FW2 | | X |
| 611 | L45 | VL-FW2 | | |
| 696* | L53 | CDR-L2 | | |
| 699 | L56 | CDR-L2 | | |
| 731 | L85 | VL-FW3 | | |
| 755* | L93 | CDR-L3 | | |
| 796* | L94 | CDR-L3 | | |
| 799 | L97 | CDR-L3 | | |
| 803 | L100⁺ | VL-FW 4 | | |

| | | | | |
|---|---|---|---|---|
| *Numbering depending on loop length +Part of the VH-VL interface as defined by Chothia et al. (13) | | | | |

From this collection of potentially VH-VL orientation determinant sequence positions, three sequence fingerprints were assembled for statistical evaluation:
Fingerprint 1 contains all sequence positions that have been found to be part of the VH-VL interface as stated in Table 29, with position 801 (L98) being discarded given their high degree of sequence conservation.
Fingerprint 2 contains all sequence positions listed among the ABangle "top 10 important input variables" (7), i.e. 197, 199, 208, 209, 211, 251, 292, 295, 296, 327, 355, 599, 602, 604, 607, 608, 609, 610, 611, 612, 615, 651, 696, 698, 699, 731, 733, 751, 753, 755, 796, 797, 798, 799, 803 (H33, H35, H43, H44, H46, H50, H58, H61, H62, H89, H99, L34, L36, L38, L41, L42, L43, L44, L45, L46, L49, L50, L53, L55, L56, L85, L87, L89, L91, L93, L94/L95x-1, L95x, L96, L97, L100), and, positions 289 and 290 (H55 and H56).
Fingerprint 3 is the set union of Fingerprint 1 and Fingerprint 2.

In order to evaluate in how far it is possible to predict VH-VL orientation based only on framework sequence, we generated two reduced variants for each of the three fingerprints, namely
a: with only the outmost CDR residues at the edge of the framework, and
b: 5 without any CDR residues.

### Example 2

### VH-VL Orientation Predictor Training

Antibody Fv crystal structures publicly available as of October 2013 were accumulated from the RCSB PDB (www.rcsb.org) (24) and, for each structure, calculated the ABangle VH-VL-orientation parameters as described herein (see example 4). Furthermore, for each structure, the VH-VL-orientation sequence fingerprint was generated as illustrated above (black highlighting in the sequences). The sequence fingerprint consists of 54 amino acids, 29 in the VH region, and 25 in the VL region. The sequence fingerprint also contains residues belonging to the hypervariable regions, that, depending on loop length, may not be present in a given antibody sequence. In this case, the unoccupied sequence fingerprint position is denoted with an 'X', instead of the regular amino acid description in one-letter code. After both ABangle parameters as well as sequence fingerprint had been calculated, the dataset (n = 2249) was categorized into complex (n_complex = 1468) and apo (n_apo = 781) structures.

The "random forest" method turned out to be the statistically significant best predictor for each of the ABangle orientation parameters, followed by "neural net" and "decision tree". The method "boosted tree" performed the least good on our dataset (data not shown).

For each ABangle parameter, 50 runs each were performed (each run consisting of a training and a test phase) while varying the number of decision trees in the random forest from 10 to 100 in order to determine the optimal number of trees with regard to the Q² value of the test set. For each individual run, the input dataset was randomly split into 70 % training and 30 % test set. The random forest model was implemented using Accelrys Pipeline Pilot 8.5 (19) with the component "Learn RP (random partitioning) Forest Model" in "Regression" mode. A list of the forest model parameter settings is listed in Table 31.

**Table 31: Parameter settings for the regression using the "Learn RP (random partitioning) Forest Model" component in Accelrys Pipeline Pilot 8.5.**

| **Tree Options** | |
|---|---|
| Minimum Samples Per Node | 10 |
| Maximum Tree Depth | 20 |
| Split Method | Gini |
| Weighting Method | By Class |

| **Forest Options** | |
|---|---|
| Number of Trees | Depending on ABangle parameter, see Table 2 |
| Ensemble Method | Bagging |
| Voting Method | Mean Score |
| Equalize Class Sizes | False |
| Minimum Samples Per Class | 5 |
| Number of Descriptors | All |

| **Advanced Tree Options** | |
|---|---|
| Maximum Knots Per Property | 20 |
| Minimum Alpha | 0.0 |
| Maximum Pruned Trees | 20 |
| Disregard Uncorrelated Questions | False |
| Minimum Correlation Squared | 0.00001 |
| Maximum Lookahead Depth | 0 |
| Number of Lookahead Alternatives | 3 |
| Maximum Generic Depth | 0 |
| Generic Node Weighting | 1.5 |

| **Learn Options** | |
|---|---|
| Numeric Distance Function | Euclidean |
| Numeric Scaling | Mean-Center and Scale, Scale by Number of Dimensions |
| Fingerprint Distance Function | Tanimoto |
| Model Domain Fingerprint | FCFP 2 |
| Number Records Before Caching | 100000 |
| Node Pool Size | 50000 |

Table 32 shows the Q² and root-mean-square error (RMSE) values for the prediction of the six ABangle parameters averaged over 50 runs with randomly chosen training and test set.

**Table 32: Q² and RMSE values for the prediction of the six ABangle parameters averaged over 50 runs. The number of trees per random forest model was tuned manually so as to maximize Q². The values in brackets specify the standard deviation.**

| | | **Apo and complex structures (n = 2249)** | | **Complex structures only (n complex = 1468)** | |
|---|---|---|---|---|---|
| **Parameter** | **N trees** | **Q² test set** | **RMSE test set** | **Q² test set** | **RMSE test set** |
| HL | 33 | 0.68 (0.02) | 2.28 (0.08) | 0.67 (0.02) | 2.26 (0.10) |
| HC1 | 50 | 0.77 (0.02) | 1.04 (0.05) | 0.80 (0.02) | 0.97 (0.04) |
| LC1 | 50 | 0.73 (0.02) | 1.26 (0.05) | 0.75 (0.02) | 1.25 (0.06) |
| HC2 | 50 | 0.78 (0.01) | 1.48 (0.04) | 0.79 (0.02) | 1.40 (0.07) |
| LC2 | 75 | 0.65 (0.02) | 1.40 (0.07) | 0.69 (0.03) | 1.30 (0.06) |
| dc | 100 | 0.56 (0.08) | 0.21 (0.05) | 0.67 (0.02) | 0.18 (0.01) |

The random forest model has been trained once on the complete dataset of apo and complex structures (Table 32, central column) and once on the complex structures only (Table 32 above, right column). Despite the fact that the training set is reduced by almost 550 structures, the Q² and RMSE values improve when only complex structures are considered. For HL, LC2 and dc, Q² values are about 0.68, while HC1, LC1 and LC2 have Q² values of 0.75 and above (when considering complex structures). Figure 2 shows exemplary regression plots for predicted versus actual ABangle parameters on the complex structures only dataset.

Further it has been evaluated if the size of the validation set (either 1/3 or 1/2 of the dataset) has an impact on the random forest predictions. For all ABangle parameters, it has been found a difference in R², as to be expected favoring the smaller validation and larger training set (data not shown).

Finally, the prediction performance of the three fingerprints and their reduced variants for the six different ABangle parameters over three repetitions has been evaluated (see Table 33).

**Table 33: Mean R² values for the prediction of the six ABangle parameters HL, HC1, LC1, HC2, LC2 and dc over three repetitions using the three sequence fingerprints and their variants with only the outmost CDR residues at the edge of the framework (a) and without any CDR residues (b).**

| | **Fingerprint** | **HL** | **HC1** | **LC1** | **HC2** | **LC2** | **dc** | **Mean** |
|---|---|---|---|---|---|---|---|---|
| Interface | 1 | 0.616 | 0.755 | 0.687 | 0.750 | 0.602 | 0.569 | 0.663 |
| | 1a | 0.577 | 0.693 | 0.640 | 0.680 | 0.496 | 0.537 | 0.604 |
| | 1b | 0.290 | 0.564 | 0.410 | 0.450 | 0.359 | 0.354 | 0.405 |
| ABangle | 2 | 0.601 | 0.752 | 0.679 | 0.761 | 0.612 | 0.567 | 0.662 |
| | 2a | 0.566 | 0.705 | 0.669 | 0.703 | 0.549 | 0.520 | 0.619 |
| | 2b | 0.481 | 0.633 | 0.602 | 0.653 | 0.486 | 0.465 | 0.553 |
| Interface + ABangle | 3 | 0.598 | 0.758 | 0.684 | 0.751 | 0.616 | 0.554 | 0.660 |
| | 3a | 0.566 | 0.708 | 0.615 | 0.714 | 0.543 | 0.538 | 0.614 |
| | 3b | 0.478 | 0.632 | 0.638 | 0.630 | 0.485 | 0.502 | 0.561 |

Fingerprint 1, based on the set of interface residues, and Fingerprint 2, based on the ABangle top input variable positions, were equally well, while combining the two (Fingerprint 3) seems neither to confer additional predictive power nor to impair the results. In all three cases, the two reduced fingerprint variants do worse, which confirms that framework sequence information alone is insufficient for determining VH-VL domain orientation.

Fingerprint 3 has been chosen for further evaluation and a random forest model for learning. In order to incorporate the predicting component into the homology modeling solution, the random forest model was implemented and trained using Accelrys Pipeline Pilot 8.5 (19) with the component "Learn RP (random partitioning) Forest Model" in "Regression" mode. For all Fv structures available in RAB3D (n = 2249), the ABangle VH-VL orientation parameters were calculated as well as Fingerprint 3, and the members of the dataset were categorized into complex (n = 1468) and apo (n = 781) structures. For each ABangle parameter, 50 runs were performed each while varying the number of decision trees in the random forest from 10 to 100 in order to determine the optimal number of trees with regard to the Q² value of the test set. For each individual run, the input dataset was randomly split into 70 % test and 30 % training set.

### Example 3

### Antibody Homology Modeling Algorithm with VH-VL Orientation

### Adjustment

The modeling software for modeling the Fv region of Antibodies (MoFvAb) uses the annotated and reoriented structures, e.g. from the RAB3D database, as template repository. A given pair of heavy and light chain input sequence was annotated with Wolfguy and reduced to VH and VL, respectively. Both VH and VL were then divided into seven functional segments, i.e. Framework 1, CDR 1, Framework 2, CDR 2, Framework 3, CDR 3, and Framework 4 (see Table 10). In contrast to other homology modeling approaches, no common framework template was picked per Fv or per chain, but every fragment was looked up/aligned/determined independently based on sequence homology. For example a single MoFvAb model might be assembled from fourteen different template structures, and possibly even more, as it is feasible to reconstruct the ascending and descending section of CDR loops from different template structures, too. Fragment template hits were ranked in the following order by
1) sequence similarity (BLOSUM62 matrix score),
2) number of incomplete side chains,
3) resolution of the template structure, and
4) alignment RMSD of the template structure versus the RAB3D consensus framework.

Optionally, it is possible to augment (or even replace in whole) the available template selection for a given fragment by a de novo segment.

All template structures have been aligned onto a common consensus framework and therefore share the same coordinate system. Thus, the template coordinates were transferred to a raw model file without further adjustments. The raw model was then processed: Non-homologous side chains were exchanged, incomplete template side chains were remodeled, and steric clashes were removed by rotamer optimization. Due to the fact that each fragment was picked independently, the number of side chain exchanges necessary per model is manageable. The processed model was parameterized for the CHARMm force field and minimized using the Generalized Born with a simple Switching (GBSW) implicit water model, first by the Steepest Descent and then by the Conjugate Gradient method. In order to preserve a maximum of conformational information from the template structures, all residues that have not been remodeled and that were not situated at fragment edges (with adjacent residues originating from different template structures) were restrained during the minimization. MoFvAb is available as a web service based on a protocol implemented in Accelrys Pipeline Pilot 8.5 (19) using the Accelrys Discovery Studio 3.5 (20) interface.

Three variants of MoFvAb model building were compared in order to assess the impact of VH-VL domain adjustment:
Variant 1: models were built from template structures aligned per chain, i.e. on a consensus VH framework and on a consensus VL framework, respectively, and, prior to model processing and energy minimization, the VH-VL orientation of the model was adjusted by chain-wise alignment onto a consensus Fv structure. This variant produced models that have a generic, average VH-VL orientation unrelated with sequence.
Variant 2: the VH-VL orientation of the model was predicted based on a sequence fingerprint as described above, and the most similar Fv template in the database in terms of its ABangle parameters was looked up. The VH-VL orientation of the model was then adjusted by chain-wise alignment onto the so-called orientation template.
   Both in Variant 1 and 2, the chain-wise alignment onto either consensus Fv or orientation template was realized by Cα superposition of the 35 ABangle core-set residues defined by Dunbar et al. (7).
Variant 3: the models were built from template structures aligned onto a common consensus Fv framework instead of a per-chain consensus structure and VH-VL orientation was not adjusted.

In order to create a representative test set, the MoFvAb was used to build the 11 antibody Fv structures from AMAII. The AMAII structures were diverse with regard to species (rabbit, mouse, human) and consist mainly of protein-binding antibodies, with anti-DNA Fab A52 (PDB ID 4M61) being the exception. All AMAII reference structures were crystallized in the unbound form. At the time of model building, access to more template structures than the original AMAII "contestants" was possible, including a number of rabbit antibody structures. Therefore, the modeling results in terms of RMSD presented herein cannot be directly compared to the results presented by the original blind modeling studies. In order to at least simulate a blind modeling scenario, no template fragments from structures with larger or equal to 95 % CDR sequence identity per chain were used, which obviously included the original crystal structures of AMAII, as well as sequence variants thereof. The identification of sequence-identical template structures to exclude from model building was performed using the software CD-HIT (15, 16).

### Example 4

### ABangle Distance Calculation

In order to compare similarity in ABangle space, a set of ABangle parameters as the tuple $θ : = \left(HL,HC1,LC1,HC2,LC2,dc\right) : = \left({ϑ}_{1}, {ϑ}_{2}, {ϑ}_{3}, {ϑ}_{4}, {ϑ}_{5}, {ϑ}_{6}\right)$ was defined. The Euclidean distance between two sets of ABangle parameters is then ${dist}_{ABangle} \left({θ}_{a}, {θ}_{b}\right) = \sqrt{{\sum }_{i = 1}^{6} {\left({ϑ}_{{i}_{a}}-{ϑ}_{{i}_{b}}\right)}^{2}} .$

A predicted set of ABangle parameters *θ̃* comes with set of associated standard deviations $\begin{matrix}{{θ}^{˜}}_{stddev} : = \left(σ\left(HL\right),σ\left(HC1\right),σ\left(LC1\right),σ\left(HC2\right),σ\left(LC2\right),σ\left(dc\right)\right) \\ : = \left({σ}_{1}, {σ}_{2}, {σ}_{3}, {σ}_{4}, {σ}_{5}, {σ}_{6}\right) .\end{matrix}$

When calculating the distance between a predicted set of ABangle parameters *θ̃* with standard deviations *θ̃_{stddev}* and a measured set of ABangle parameters *θ*, the uncertainty of the prediction was factored by using a weighted distance function ${\overline{dιst}}_{ABangle} \left({θ}^{˜}, θ\right) : = \sqrt{{\sum }_{i = 1}^{6} {\left(\left({{ϑ}^{˜}}_{i}-{ϑ}_{i}\right)/{σ}_{i}\right)}^{2}} .$

The weighted distance function was used for finding orientation templates in the database that best match a predicted set of ABangle parameters. As *dist_{ABangle}* and *dlst_{ABangle}* mingle angular (HL, HC1, LC1, HC2, LC2) with linear (dc) distance measures, they cannot be interpreted as factual distance in angular space but serve only as an abstract distance measure.

For calculating ABangle orientation parameters, the program code published by Dunbar et al. (7) available at http://www.stats.ox.ac.uk/∼dunbar/abangle/ was used in a slightly modified version that works on Wolfguy-numbered structures.

### Example 5

### Correlations

### Pearson correlation coefficient

The Pearson correlation coefficient measures the linear correlation between two variables X and Y. It is calculated as $r = \frac{cov \left(X, Y\right)}{{σ}_{X} {σ}_{Y}} ,$

With cov(X,Y) being the covariance between X and Y and σ the standard deviation. The standard cor.test method in R(25) was used to calculate the correlation coefficient and the p-value to evaluate if it differs significantly from zero.

### RV coefficient

The RV coefficient was introduced by Escoufier to measure the similarity between square symmetric matrices (26). The definition can be easily extended to rectangular matrices (27). For two matrices X and Y the RV coefficient can be calculated as $RV = \frac{trace \left\{{S}^{T}T\right\}}{\sqrt{\left(trace\left\{{S}^{T}S\right\}\right) \times \left(trace\left\{{T}^{T}T\right\}\right)}} ,$ with S = XX^{T} and T = YY^{T}.

In order to calculate an associated p-value for the RV coefficient, that is whether it is as high as it is just by chance, the coeffRV-method from the FactoMine^{R} package was used (28) in R, which implements a permutation test as described in Josse et al.(29).

### Methods for rejecting antibodies based on angles distances to a reference antibody

Under the assumption that bigger angle distances hint to a worse binding behavior of antibodies compared to a reference, many different methods to reject antibodies are conceivable.

### i) Reject the worst 20%

Herein a certain percentage of antibodies is rejected directly based on their angle distance to the reference. As an example we here chose to reject 20%. So the steps in this algorithm are
1. sort the angle-distance matrix and remember the indices
2. use the indices of the 20% highest angle-distances to reject the worst antibodies

### ii) Reject whole HCs/LCs

Herein whole HCs or LCs is/are rejected and just produce the other HC/LC combinations. In order to do this we propose the following algorithm
1. calculate the average angle-distance for each HC/LC
2. visualize these distances and select a subset of HCs/LCs for rejection

### iii) Reject bad HC/LC combinations

A variant of the subsequent method is to just reject antibodies which have "bad" HC/LC combinations. This method might perform well if the correlation of angle-distance to antibody is not so strong for individual antibodies but is better preserved over whole HCs and LCs. The algorithm is:
1. calculate the average angle-distance for each HC/LC
2. visualize these distances and select a subset of HCs/LCs in order to reject only all possible combinations between these.

### Example 6

### Carbonyl RMSD

For the sake of consistency with AMAII, the carbonyl RMSD and the definition of β-sheet core and CDR loops according to Teplyakov et al. (2) were used In order to determine the carbonyl RMSD for a given fragment, first the model was superimposed onto the crystal structure using the Cα atoms of the β-sheet core with the superposition method provided in Accelrys Discovery Studio 3.5 (20). The carbonyl RMSD was then calculated as the deviation of the backbone carbonyl group atoms of the given segment with regard to the crystal structure. Compared to the commonly used Cα or whole backbone RMSD, the carbonyl RMSD is more sensitive with regard to deviations in backbone conformation. While in AMAII all carbonyl RMSD values were calculated based on a superposition of the β-sheet core of either VH or VL only, herein additionally the carbonyl RMSD based on a superposition of the β-sheet core of VH and VL simultaneously was calculated. Superpositioning on the whole Fv lead to worse RMSD values as it factors in flaws in VH-VL orientation.

When recalculating RMSD carbonyl values of the original AMAII models downloaded from http://www.3dabmod.com, not all values from the original reference could be reproduced exactly, which was attributed to either minor differences in the superpositioning algorithm, or numerical inaccuracies.

### Example 7

### Binding Cell ELISA with humanized anti-CD81 antibody variants

For the binding cell ELISA assay, HuH7-Rluc-H3 (positive cell line expressing CD81) and HuH7-Rluc-L1 (negative control cell line) were propagated in F-12 DMEM medium with 10 % FCS at 37 °C and 5 % CO₂. On day 1, the cells were trypsinized at approximately 90 % confluence and resuspended at 4 x 10⁵ cells/mL. 2 x10⁴ cells/well HuH7-Rluc-H3 and HuH7-Rluc-L1 (negative control cell line) were plated in 50 µL DMEM medium and allowed to adhere to the 96 well poly-D-Lysine plate (Greiner, Cat-Nr. 655940) for 24 hours at 37 °C and 5 % CO₂. On day 2, the antibody samples to be tested were prepared in a separate polypropylene round bottom plate with a twofold desired concentration with a final volume of 120 µl. All of the assay samples were diluted in cell culture medium. 50 µL of each antibody sample (duplicate wells) were added to cells to give final volume of 100 µL/well and incubated for 2 hours at 4 °C. Following the primary incubation the samples were removed by aspiration and the cells were fixed with 0.05 % glutaraldehyde in PBS solution (Roche Diagnostics GmbH, Mannheim, Germany, Cat-Nr. 1666789) for 10 minutes at room temperature. After fixation, each well was washed 3 times with 200 µL PBS/0.05 % Tween. The secondary incubation step for detection of bound anti-CD81 antibodies was performed for 2 h at room temperature on a reciprocal shaker. For humanized CD81K antibodies, detection was performed using peroxidase conjugate sheep anti-human-IgG-gamma chain specific antibody (The Binding Site, Cat.-Nr. AP004) and a goat anti-mouse IgG, (H+L)-HRP conjugate (BIORAD, Cat-Nr. 170-6516) was used for the JS81 mouse positive control antibody (BD Biosciences, Cat. Nr. 555675) both diluted 1:1000 in PBS 10 % blocking buffer. Each well was washed three times with 200 µL PBS/0.05 % Tween to remove unbound antibodies. The HRP activity was detected using 50 µL ready-to-use TMB solution (Roche Diagnostics GmbH, Mannheim, Germany, Cat-Nr. 1432559) and reaction was stopped after approximately 7-10 minutes with 50 µL per well 1 M H₂SO₄. The absorbance was read using the ELISA Tecan reader at 450 nm with 620 nm reference wavelength.

### Example 8

### Kinetic screening

The kinetic screening was performed according to Schraeml et al. (Schraeml, M. and M. Biehl, Methods Mol. Biol. 901 (2012) 171-181) on a BIAcore 4000 instrument, mounted with a BIAcore CM5 sensor. The BIAcore 4000 instrument was under the control of the software version V1.1. A BIAcore CM5 series S chip was mounted into the instrument and was hydrodynamically addressed and preconditioned according to the manufacturer's instructions. The instrument buffer was HBS-EP buffer (10 mM HEPES (pH 7.4), 150 mM NaCl, 1 mM EDTA, 0.05 % (w/v) P20). An antibody capture system was prepared on the sensor surface. A polyclonal goat anti-human antibody with human IgG-Fc specificity (Jackson Lab.) was immobilized at 30 µg/ml in 10 mM sodium acetate buffer (pH 5) to spots 1, 2, 4 and 5 in the instrument's flow cells 1, 2, 3 and 4 at 10,000 RU using NHS/EDC chemistry. In each flow cell the antibodies were captured on spot 1 and spot 5. Spot 2 and spot 4 were used as reference spots. The sensor was deactivated with a 1 M ethanolamine solution. Humanized antibody derivatives were applied at concentrations between 44 nM and 70 nM in instrument buffer supplemented with 1mg/ml CMD (carboxymethyldextrane). The antibodies were injected at a flow rate of 30 µl/min for 2 min. The capture level (CL) of the surface-presented antibodies was measured in rel. response units (RU). The analytes in solution, phosphorylated human tau protein, non-phosphorylated human tau protein and the phosphorylated human tau mutant protein T422S, were injected at 300 nM for 3 min. at a flow rate of 30 µl/min. The dissociation was monitored for 5 min. The capture system was regenerated by a 1 min. injection of 10 mM glycine buffer pH 1.7 at 30 µL/min. over all flow cells. Two report points, the recorded signal shortly before the end of the analyte injection, denoted as binding late (BL) and the recorded signal shortly before the end of the dissociation time, stability late (SL), were used to characterize the kinetic screening performance. Furthermore, the dissociation rate constant kd (1/s) was calculated according to a Langmuir model and the antibody/antigen complex half-life was calculated in minutes according to the formula ln(2)/(60^{∗}kd). The molar ratio (MR) was calculated according to the formula MR = (Binding Late (RU)) / (Capture level (RU)) ^{∗} (MW(antibody) /(MW(antigen)). In case the sensor was configured with a suitable amount of antibody ligand capture level, each antibody should be able to functionally bind at least to one analyte in solution, which is represented by a molar ratio of MR = 1.0. Then, the molar ratio is also an indicator for the valence mode of analyte binding. The maximum valence can be MR = 2 for an antibody binding two analytes, one with each Fab valence.

In another embodiment, kinetic rates were determined at 25 °C and 37 °C using the same experimental setup, but using multiple concentration series of each analyte in solution at 0 nM (buffer), 1.2 nM, 3.7 nM, 11.1 nM, 33.3 nM, 100 nM and 300 nM. From the concentration-dependent binding behavior the kinetic data was calculated using the BIAcore evaluation software according to the manufacturer's instructions and a Langmuir 1.1 model with RMAX global.

### REFERENCES

1. Kuroda, D., et al., Protein Eng. Des. Sel., 25 (2012) 507-521.
2. Teplyakov, A., et al. Proteins, 2014; DOI: 10.1002/prot.24554
3. Dunbar, J., et al., Nuc. Acids Res., 42 (2014) D1140-D1146.
4. Abhinandan, K.R. and Martin, A.C.R., Protein Eng. Des. Sel., 23 (2010) 689-697.
5. Chailyan, A., et al., FEBS J., 278 (2011) 2858-2866
6. Narayanan, A., et al., J. Mol. Biol. 388 (2009) 941-953.
7. Dunbar, J., et al., Protein Eng. Des. Sel., 26 (2013) 611-620.
8. Almagro, J.C., et al., Proteins 79 (2011) 3050-3066.
9. Jayaram, N., et al., Protein Eng. Des. Sel. 25 (2012) 523-529.
10. Banfield, M.J., et al., Proteins, 29 (1997) 161-171.
11. Li, Y., et al., Biochem., 39 (2000) 6296-6309.
12. Teplyakov, A., et al., Acta Cryst., F67 (2011) 1165-1167.
13. Chothia, C., et al., J. Mol. Biol., 278 (1998) 457-479.
14. Chothia, C. and Lesk, A.M., J. Mol. Biol. 196 (1987) 901-917.
15. Li, W. and Godzik, A., Bioinformatics, 22 (2006) 1658-1659.
16. Fu, L., et al., Bioinformatics, 28 (2012) 3150-3152.
17. Pan, R., et al., J. Virol., 87 (2013) 10221-10231.
18. Kaas, Q., et al., Brief Funct. Genom. Prot., 6 (2007) 253-264.
19. Accelrys Software Inc., Pipeline Pilot, Release 8.5.0.200, San Diego: Accelrys Software Inc., 2011
20. Accelrys Software Inc., Discovery Studio Modeling Environment, Release 3.5.0.12158, San Diego: Accelrys Software Inc., 2012.
21. Niederfellner, G., et al., Blood, 118 (2011) 358-367.
22. Kaas, Q., et al., Brief Funct. Genom. Prot., 6 (2007) 253-264.
23. Accelrys Software Inc., Pipeline Pilot, Release 8.5.0.200, San Diego: Accelrys Software Inc., 2011.
24. Berman, H.M., et al., Nucl. Acids Res., 28 (2000) 235-242.
25. R Core Team (2013). R: A language and environment for statistical computing. R Foundation for Statistical Computing, Vienna, Austria. URL http://www.R-project.org/.
26. Robert, P. and Escoufier, Y., Appl. Stat. 25 (1967) 257-265
27. Abdi, H., (2007). RV coefficient and congruence coefficient, In N.J. Salkind (Ed): Encyclopedia of measurement and statistics. Thousand Oaks: Sage.
28. Husson, F., et al., (2014). FactoMineR: Multivariate Exploratory Data Analysis and Data Mining with R. R package version 1.26. http://CRAN.R-project.org/package=FactoMineR
29. Josse, J., et al., Comp. Stat. Data Anal., 53 (2008) 82-91.

## Claims

1. A method for selecting one or more variant antibody Fv fragments derived from a non-human antibody Fv fragment comprising the following steps:
- generating a multitude of variant antibody Fv fragments by grafting/transferring the antigen binding specificity determining residues from the non-human antibody Fv fragment on a human antibody Fv fragment germline amino acid sequence , whereby each variant antibody Fv fragment of the multitude of variant antibody Fv fragments differs from the other variant antibody Fv fragments by at least one amino acid residue,
- determining the VH-VL-angle for the non-human Fv fragment and for each of the variant antibody Fv fragments of the multitude of variant antibody Fv fragments based on a set of VH-VL-interface residues comprising residues L44, L46, L87, H45, H62 (numbering according to Chothia index) of the antibody Fv fragment,
- selecting those variant antibody Fv fragments that have the smallest difference in the VH-VL-angle compared to the non-human antibody's VH-VL-angle and thereby selecting one or more variant antibody Fv fragments derived from a non-human antibody Fv fragment,
whereby the one or more variant antibody Fv fragments bind to the same antigen as the non-human antibody Fv fragment,
wherein the VH-VL-angle is determined by calculating the six parameters
- the length of C, dc,
- the torsion angle, HL, from H1 to L1 measured about C,
- the bend angle, HC1, between H1 and C,
- the bend angle, HC2, between H2 and C,
- the bend angle, LC1 between L1 and C, and
- the bend angle, LC2, between L2 and C,
wherein reference frame planes are registered by i) aligning the Cα coordinates corresponding to the eight positions H36, H37, H38, H39, H89, H90, H91 and H92 of VH and fitting a plane through them and ii) aligning the Cα coordinates corresponding to the eight positions L35, L36, L37, L38, L85, L86, L87 and L88 of VL and fitting a plane through them, iii) placing a placed on each plane, whereby each structure has equivalent mesh points and equivalent VH-VL mesh point pairs, and iv) measuring the Euclidean distance for each pair of mesh points in each structure, whereby the vector C joins the pair of points with the minimum variance in their separation distance,
wherein H1 is the vector running parallel to the first principal component of the VH plane, H2 is the vector running parallel to the second principal component of the VH plane, L1 is the vector running parallel to the first principal component of the VL plane, L2 is the vector running parallel to the second principal component of the VL plane, the HL angle is the torsion angle between the two domains, the HC1 and LC1 are the bend angles equivalent to tilting-like variations of one domain with respect to the other, and the HC2 and LC2 bend angles are equivalent to the twisting-like variations of one domain to the other.

2. The method according to claim 1 comprising the following step:
- selecting those variant antibody Fv fragments that have the highest similarity in the VH-VL-interdomain angle compared to the parent antibody's VH-VL-interdomain angle and thereby selecting one or more variant antibody Fv fragments derived from a parent antibody Fv fragment.

3. The method according to any one of claims 1 to 2, wherein the set of VH-VL-interface residues comprises residues H33, H35, H37, H39, H43, H44, H45, H46, H47, H50, H55, H56, H58, H60, H61, H62, H89, H91, H95, H96, H98, H99, H100x-2, H100x-1, H100x, H101, H102, H103, H105, L32, L34, L36, L38, L41, L42, L43, L44, L45, L46, L49, L50, L53, L55, L56, L85, L87, L89, L91, L93, L94/L95x-1, L95x, L96, L97, LI00 (numbering according to Chothia index).

4. The method according to any one of claims 1 to 3, wherein the VH-VL-angle is determined by calculating the VH-VL-angle parameters using one random forest method for each parameter.

5. The method according to any one of claims 1 to 4, wherein the VH-VL-angle is determined by calculating the torsion angle, the four bend angles (two per variable domain), and the length of the pivot axis of VH and VL (HL, HC1, LC1, HC2, LC2, dc) using a random forest model.

6. The method according to any one of claims 4 to 5, wherein the random forest model is trained only with complex antibody structure data.

7. The method according to any one of claims 1 to 6, wherein the smallest difference is the highest Q² value.

8. The method according to any one of claims 1 to 7, wherein the highest similarity is the lowest average root-mean-square deviation (RMSD).

9. The method according to any one of claims 1 to 8, wherein a model assembled from template structures aligned on either consensus VH or VL framework, followed by VH-VL reorientation on a VH-VL orientation template structure chosen based on similarity is used to determine the VH-VL-angle.

## Patentansprüche

1. Verfahren zum Auswählen von einer oder mehreren Antikörper-Fv-Fragmentvarianten, die von einem nicht humanen Antikörper-Fv-Fragment abgeleitet sind, umfassend die folgenden Schritte:
- Erzeugen einer Vielzahl von Antikörper-Fv-Fragmentvarianten durch Pfropfen/Übertragen der Antigenbindungsspezifität-bestimmenden Reste aus dem nicht humanen Antikörper-Fv-Fragment auf eine Keimbahn-Aminosäuresequenz eines humanen Antikörper-Fv-Fragments, wobei sich jede Antikörper-Fv-Fragmentvariante der Vielzahl von Antikörper-Fv-Fragmentvarianten von anderen Antikörper-Fv-Fragmentvarianten um mindestens einen Aminosäurerest unterscheidet,
- Bestimmen des VH-VL-Winkels für das nicht humane Fv-Fragment und für jede der Antikörper-Fv-Fragmentvarianten der Vielzahl von Antikörper-Fv-Fragmentvarianten basierend auf einem Satz von VH-VL-Grenzflächenresten, umfassend Reste L44, L46, L87, H45, H62 (Nummerierung gemäß Chothia-Index) des Antikörper-Fv-Fragments,
- Auswählen derjenigen Antikörper-Fv-Fragmentvarianten, die den geringsten Unterschied des VH-VL-Winkels im Vergleich zu dem VH-VL-Winkel des nicht humanen Antikörpers haben, und dabei Auswählen von einer oder mehreren Antikörper-Fv-Fragmentvarianten, die von einem nicht humanen Antikörper-Fv-Fragment abgeleitet sind,
wobei die eine oder mehreren Antikörper-Fv-Fragmentvarianten an dasselbe Antigen wie das nicht humane Antikörper-Fv-Fragment binden,
wobei der VH-VL-Winkel durch Berechnen der folgenden sechs Parameter bestimmt wird
- der Länge von C, dc,
- des Drehwinkels, HL, von H1 zu L1, gemessen um C,
- des Biegewinkels, HC1, zwischen H1 und C,
- des Biegewinkels, HC2, zwischen H2 und C,
- des Biegewinkels, LC1, zwischen L1 und C und
- des Biegewinkels, LC2, zwischen L2 und C,
wobei Referenzrahmenebenen erfasst werden durch i) Zuordnen der Cα-Koordinaten entsprechend den acht Positionen H36, H37, H38, H39, H89, H90, H91 und H92 von VH und Einpassen einer Ebene durch diese und ii) Zuordnen der Cα-Koordinaten entsprechend den acht Positionen L35, L36, L37, L38, L85, L86, L87 und L88 von VL und Einpassen einer Ebene durch diese, iii) Platzieren eines plaziert auf jeder Ebene, wodurch jede Struktur äquivalente Gitterpunkte und äquivalente VH-VL-Gitterpunktpaare aufweist, und iv) Messen des euklidischen Abstands jedes Paars von Gitterpunkten in jeder Struktur, wodurch der Vektor C das Paar von Punkten mit der kleinsten Varianz ihres Trennungsabstands verbindet,
wobei H1 der Vektor ist, der parallel zu der ersten Hauptkomponente der VH-Ebene verläuft, H2 der Vektor ist, der parallel zu der zweiten Hauptkomponente der VH-Ebene verläuft, L1 der Vektor ist, der parallel zu der ersten Hauptkomponente der VL-Ebene verläuft, L2 der Vektor ist, der parallel zu der zweiten Hauptkomponente der VL-Ebene verläuft, der HL-Winkel der Drehwinkel zwischen den beiden Domänen ist, der HC1 und LC1 die Biegewinkel äquivalent zu neigungsähnlichen Variationen einer Domäne bezogen auf die andere sind und die HC2- und LC2-Biegewinkel äquivalent zu den verdrehungsähnlichen Variationen einer Domäne gegenüber der anderen sind.

2. Verfahren nach Anspruch 1, umfassend den folgenden Schritt:
- Auswählen derjenigen Antikörper-Fv-Fragmentvarianten, die die stärkste Ähnlichkeit bezüglich des VH-VL-Interdomänenwinkels im Vergleich zu dem parentalen VH-VL-Interdomänenwinkel des Antikörpers haben, und dabei Auswählen von einer oder mehreren Antikörper-Fv-Fragmentvarianten, die von einem parentalen Antikörper-Fv-Fragment abgeleitet sind.

3. Verfahren nach einem der Ansprüche 1 bis 2, wobei der Satz von VH-VL-Grenzflächenresten Reste H33, H35, H37, H39, H43, H44, H45, H46, H47, H50, H55, H56, H58, H60, H61, H62, H89, H91, H95, H96, H98, H99, H100x-2, H100x-1, H100x, H101, H102, H103, H105, L32, L34, L36, L38, L41, L42, L43, L44, L45, L46, L49, L50, L53, L55, L56, L85, L87, L89, L91, L93, L94/L95x-1, L95x, L96, L97, L100 (Nummerierung gemäß Chothia-Index) umfasst.

4. Verfahren nach einem der Ansprüche 1 bis 3, wobei der VH-VL-Winkel durch Berechnen der VH-VL-Winkelparameter unter Anwendung eines Random-Forest-Verfahrens für jeden Parameter bestimmt wird.

5. Verfahren nach einem der Ansprüche 1 bis 4, wobei der VH-VL-Winkel durch Berechnen des Drehwinkels, der vier Biegewinkel (zwei pro variable Domäne) und der Länge der Schwenkachse von VH und VL (HL, HC1, LC1, HC2, LC2, dc) unter Anwendung eines Random-Forest-Modells bestimmt wird.

6. Verfahren nach einem der Ansprüche 4 bis 5, wobei das Random-Forest-Modell nur mit komplexen Antikörperstrukturdaten trainiert wird.

7. Verfahren nach einem der Ansprüche 1 bis 6, wobei der geringste Unterschied der höchste Q²-Wert ist.

8. Verfahren nach einem der Ansprüche 1 bis 7, wobei die stärkste Ähnlichkeit die geringste durchschnittliche Standardabweichung (RMSD) ist.

9. Verfahren nach einem der Ansprüche 1 bis 8, wobei ein Modell, zusammengesetzt aus Matrixstrukturen, die auf Consensus-VH- oder -VL-Gerüst ausgerichtet sind, gefolgt von VH-VL-Umorientierung auf einer VH-VL-Orientierungsmatrixstruktur, die basierend auf Ähnlichkeit ausgewählt wurde, zum Bestimmen des VH-VL-Winkels verwendet wird.

## Revendications

1. Procédé pour sélectionner un ou plusieurs fragments Fv d'anticorps variants dérivés d'un fragment Fv d'anticorps non humain comprenant les étapes suivantes :
- génération d'une multitude de fragments Fv d'anticorps variants par greffage/transfert des résidus déterminant la spécificité de liaison à l'antigène du fragment Fv d'anticorps non humain sur une séquence d'acides aminés de lignée germinale de fragment Fv d'anticorps humain, moyennant quoi chaque fragment Fv d'anticorps variant de la multitude de fragments Fv d'anticorps variants diffère des autres fragments Fv d'anticorps variants par au moins un résidu d'acide aminé,
- détermination de l'angle VH-VL pour le fragment Fv non humain et pour chacun des fragments Fv d'anticorps variants de la multitude de fragments Fv d'anticorps variants sur la base d'un ensemble de résidus d'interface VH-VL comprenant les résidus L44, L46, L87, H45, H62 (numérotation selon l'indice de Chothia) du fragment Fv d'anticorps,
- sélection de ces fragments Fv d'anticorps variants qui présentent la plus petite différence d'angle VH-VL par comparaison avec l'angle VH-VL de l'anticorps non humain et sélection ainsi d'un ou plusieurs fragments Fv d'anticorps variants dérivés d'un fragment Fv d'anticorps non humain,
moyennant quoi le ou les fragments Fv d'anticorps variants se lient au même antigène que le fragment Fv d'anticorps non humain,
dans lequel l'angle VH-VL est déterminé en calculant les six paramètres suivants
- la longueur de C, dc,
- l'angle de torsion, HL, de H1 à L1 mesuré autour de C,
- l'angle de pliage, HC1, entre H1 et C,
- l'angle de pliage, HC2, entre H2 et C,
- l'angle de pliage, LC1, entre L1 et C, et
- l'angle de pliage, LC2, entre L2 et C,
dans lequel les plans de charpente de référence sont enregistrés par i) alignement des coordonnées Cα correspondant aux huit positions H36, H37, H38, H39, H89, H90, H91 et H92 de VH et ajustement d'un plan à travers celles-ci et ii) alignement des coordonnées Cα correspondant aux huit positions L35, L36, L37, L38, L85, L86, L87 et L88 de VL et ajustement d'un plan à travers celles-ci, iii) placement d'une placée sur chaque plan, moyennant quoi chaque structure présente des points de maille équivalents et des paires de points de maille VH-VL équivalents, et iv) mesure de la distance euclidienne pour chaque paire de points de maille dans chaque structure, moyennant quoi le vecteur C relie la paire de points avec la variance minimale dans leur distance de séparation,
dans lequel H1 est le vecteur qui s'étend parallèlement à la première composante principale du plan de VH, H2 est le vecteur qui s'étend parallèlement à la seconde composante principale du plan de VH, L1 est le vecteur qui s'étend parallèlement à la première composante principale du plan de VL, L2 est le vecteur qui s'étend parallèlement à la seconde composante principale du plan de VL, l'angle HL est l'angle de torsion entre les deux domaines, HC1 et LC1 sont les angles de pliage équivalents aux variations de type inclinaison d'un domaine par rapport à l'autre et les angles de pliage HC2 et LC2 sont équivalents aux variations de type torsion d'un domaine par rapport à l'autre.

2. Procédé selon la revendication 1 comprenant l'étape suivante :
- sélection de ces fragments Fv d'anticorps variants qui présentent la similarité la plus élevée d'angle VH-VL-interdomaine par comparaison avec l'angle VH-VL-interdomaine de l'anticorps parent et sélection ainsi d'un ou plusieurs fragments Fv d'anticorps variants dérivés d'un fragment Fv d'anticorps parent.

3. Procédé selon l'une quelconque des revendications 1 à 2, dans lequel l'ensemble de résidus d'interface VH-VL comprend les résidus H33, H35, H37, H39, H43, H44, H45, H46, H47, H50, H55, H56, H58, H60, H61, H62, H89, H91, H95, H96, H98, H99, H100x-2, H100x-1, H100x, H101, H102, H103, H105, L32, L34, L36, L38, L41, L42, L43, L44, L45, L46, L49, L50, L53, L55, L56, L85, L87, L89, L91, L93, L94/L95x-1, L95x, L96, L97, L100 (numérotation selon l'indice de Chothia).

4. Procédé selon l'une quelconque des revendications 1 à 3, dans lequel l'angle VH-VL est déterminé en calculant les paramètres d'angle VH-VL en utilisant une méthode de forêt aléatoire pour chaque paramètre.

5. Procédé selon l'une quelconque des revendications 1 à 4, dans lequel l'angle VH-VL est déterminé en calculant l'angle de torsion, les quatre angles de pliage (deux par domaine variable) et la longueur de l'axe pivot de VH et VL (HL, HC1, LC1, HC2, LC2, dc) en utilisant un modèle de forêt aléatoire.

6. Procédé selon l'une quelconque des revendications 4 à 5, dans lequel le modèle de forêt aléatoire est formé uniquement avec des données de structures d'anticorps complexes.

7. Procédé selon l'une quelconque des revendications 1 à 6, dans lequel la différence la plus faible est la valeur de Q² la plus élevée.

8. Procédé selon l'une quelconque des revendications 1 à 7, dans lequel la similarité la plus élevée est l'écart quadratique moyen (EQM) le plus faible.

9. Procédé selon l'une quelconque des revendications 1 à 8, dans lequel un modèle assemblé à partir de structures de matrices alignées sur l'une ou l'autre charpente consensus de VH ou de VL, suivi par réorientation de VH-VL sur une structure de matrice d'orientation de VH-VL choisie sur la base de la similarité est utilisé pour déterminer l'angle VH-VL.
